# EUROPEAN PATENT SPECIFICATION

(11) **EP 0 560 235 B1**
(45) Date of publication and mention of the grant of the patent: **04.06.1997**
(21) Application number: 93103614.9
(22) Date of filing: 06.03.1993
(51) Int. Cl.: C07D 401/06, A61K 31/445, C07D 209/08, C07D 215/14, C07D 223/16, C07D 225/06, C07D 403/06, C07D 413/06

(54) **Condensed heterocyclic ketone derivatives, their production and use**
Kondensierte heterocyclische Ketonderivate, ihre Herstellung und ihre Verwendung
Dérivés cétoniques hétérocycliques condensées, leur préparation et leur utilisation

(30) Priority: 09.03.1992 JP 50960/92; 17.04.1992 JP 97948/92; 05.06.1992 JP 145852/92; 06.08.1992 JP 210225/92; 29.09.1992 JP 259606/92
(43) Date of publication of application: 15.09.1993
(73) Proprietor: Takeda Chemical Industries, Ltd., Osaka-shi, Osaka 541 (JP)
(72) Inventor: Goto, Giichi, Toyono-gun, Osaka 563-01 (JP); Miyamoto, Masaomi, Takarazuka, Hyogo 665 (JP); Ishihara, Yuji, Itami, Hyogo 664 (JP)
(74) Representative: von Kreisler, Alek, Dipl.-Chem.

(56) References cited:
- EP-A- 0 035 228
- EP-A- 0 229 391
- EP-A- 0 294 647
- EP-A- 0 326 106
- EP-A- 0 351 282
- EP-A- 0 487 071
- US-A- 4 521 606

## Description

This invention relates to novel condensed heterocyclic ketone derivatives and salts thereof. These compounds are useful as medicines, more specifically, cholinesterase inhibitors, especially as therapeutic or/and prophylactic agents for senile dementia, Alzheimer's disease and so on.

With an increasing number of elderly people, there have been proposed various compounds having therapeutic or/and prophylactic actions on senile dementia. Among them, in physostigmine, a naturally-occurring cholinesterase inhibitor, there has been found therapeutic or/and prophylactic actions on senile dementia (International Journal of Clinical Pharmacology, Therapy and Toxicology, Vol. 29, No. 1, p. 23-37(1991) etc.). Physostigmine is, however, possessed of such drawbacks as relatively short duration of the action and high toxicity.

On the other hand, various heterocyclic compounds have been proposed as synthetic medicines (for example, in EP-A-0,378,207, USP 4,849,431 and USP 4,895,841, cholinesterase inhibitors having N-containing heterocyclic ring being described, and, in JPA S52(1977)-72829 and JPA S55(1980)-9070, antidepressant or antianxiety drugs having chemical structures analogous to the above-mentioned cholinesterase inhibitor being described).

More specifically, in EP-A-0,378,207, there are disclosed cyclic amine compounds represented by the formula: 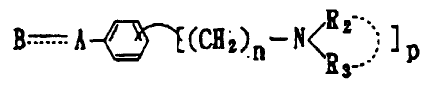 wherein B stands for an optionally substituted saturated or unsaturated 5- to 7-membered azaheterocyclic group; A stands for a bond or an alkylene group or alkenylene group optionally substituted with a hydrocarbon residue,oxo group or hydroxyl group; 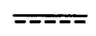 means single bond or double bond (provided that, when A stands for a bond, 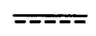 means single bond); R₂ and R₃ independently stand for H or an optionally substituted hydrocarbon residue (provided that they are not H at the same time) or may form a cyclic amino group taken together with the adjacent nitrogen atom; n denotes 0, 1 or 2; p denotes 1 or 2, or salts thereof, practically the following compound, among others.

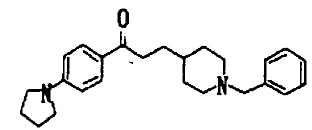

In USP 4,849,431, there are disclosed piperidine derivatives represented by the formula: 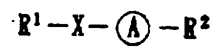 wherein R¹ stands for a monovalent group derived from a member selected from optionally substituted benzene, pyridine, pyrazine, indole, anthraquinone, quinoline, optionally substituted phthalimide, homophthalimide, pyridine carboxylimide, pyridine-N-oxide, pyrazine carboxylimide, naphthalenedicarboxylimide, optionally substituted quinazolinedione, 1,8-naphthalimide, bicyclo[2,2,2]oct-5-ene-2,3-dicarboxylimide and pyromellylimide;
X stands for a group shown by the formula: -(CH₂)ₘ-(wherein m denotes a whole number of 0 to 7), a group shown by the formula: -O(CH₂)ₙ-, a group shown by the formula: -S(CH₂)ₙ, a group shown by the formula: - NH(CH₂)ₙ-, a group shown by the formula: -SO₂NH(CH₂)ₙ-, a group shown by the formula: -NH-CO-(CH₂)ₙ-, a group shown by the formula: -NH(CH₂)ₙ-CO-, a group shown by the formula: -CO-NR³-(CH₂)ₙ- (in the definition of X, n in the above formulae denotes a whole number of 1 to 7, and R³ stands for a lower alkyl or benzyl group), a group shown by the formula: -O-CH₂CH₂CH(CH₃)-, a group shown by the formula: -O-CH₂CH₂CH=, or a group shown by the formula: -O-CH₂CH(OH)CH₂-; Ring A stands for a group shown by the formula: 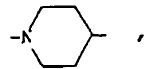 a group shown by the formula: 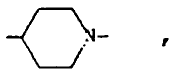 , a group shown by the formula: 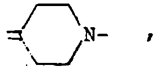 a group shown by the formula: 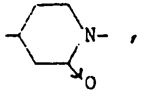 R² stands for H, a lower alkyl group, optionally substituted benzyl group, optionally substituted benzoyl group, pyridyl group, 2-hydroxyethyl group, pyridylmethyl group, or a group shown by the formula: 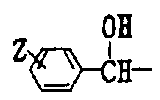 (wherein Z stands for a halogen atom), or pharmaceutically acceptable salts thereof, practically the following compound .

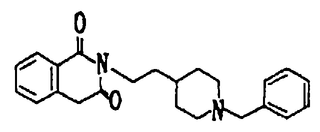

In USP 4,895,841, there are disclosed cyclic amine derivatives represented by the general formula: 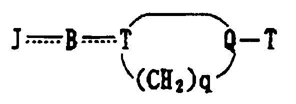 wherein J stands for (a) optionally substituted groups shown as follows; (1) phenyl group, (2) pyridyl group, (3) pyrazyl group, (4) quinolyl group, (5) cyclohexyl group, (6) quinoxalyl group or (7) furyl group,
(b) a mono- or di-valent group selected from the following groups optionally substituted with phenyl group; (1) indanyl, (2) indanonyl, (3) indenyl, (4) indenonyl, (5) indanedionyl, (6) tetralonyl, (7) benzsperonyl, (8) indanolyl, (9) group shown by the formula: 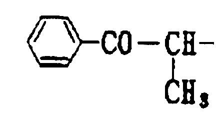
(c) a monovalent group derived from a cyclic amido compound,
(d) a lower alkyl group, or
(e) a group shown by the formula: R¹-CH=CH- (wherein R¹ stands for H or a lower alkoxycarbonyl group);
B stands for a group shown by the formula: -(C(R²)H)ₙ-, a group shown by the formula: -CO-(C(R²)H)ₙ-, a group shown by the formula: -NR²-(C(R²)H)ₙ- (wherein R² stands for H, a lower alkyl group, acyl group, a lower alkylsulfonyl group, optionally substituted phenyl group or benzyl group), a group shown by the formula: - CO-NR⁴-(C(R²)H)ₙ- (wherein R⁴ stands for H, a lower alkyl group or phenyl group), a group shown by the formula: -CH=CH-(C(R²)H)ₙ-, a group shown by the formula: -O-COO-(C(R²)H)ₙ-, a group shown by the formula: -O-CO-NH-(C(R²)H)ₙ, a group shown by the formula: -NH-CO-(C(R²)H)ₙ-, a group shown by the formula: -CH₂-CO-NH-(C(R²)H)ₙ-, a group shown by the formula: -CO-NH-(C(R²)H)ₙ-, a group shown by the formula: -C(OH)H-(C(R²)Hₙ- (in the above formulae, n denotes 0 or a whole number of 1 to 10, R² stands for H or methyl group in the form of alkylene group shown by the formula: -(C(R²)H)ₙ- which is unsubstituted or having one or more than one methyl group), a group shown by the formula: =(CH-CH=CH)_{b}- (wherein b denotes a whole number of 1 to 3), a group shown by the formula: =CH-(CH₂)_{c}- (wherein c denotes a whole number of 1 to 9), a group shown by the formula: =(CH-CH)_{d}= (wherein d denotes 0 or a whole number of 1 to 5), a group shown by the formula: -CO-CH=CH-CH₂-, a group shown by the formula: -C(CH₃)H-CO-NH-CH₂-, a group shown by the formula: -CH=CH-CO-NH-(CH₂)₂-, the group shown by the formula: -NH-, the group shown by the formula: -O-, the group shown by the formula: -S-, dialkylaminoalkylcarbonyl group or a lower alkoxycarbonyl group,
T stands for N or C,
Q stands for N, C or a group shown by the formula: >N→ O,
K stands for H, an optionally substituted phenyl group, an arylalkyl group optionally substituted with phenyl group, a cinnamyl group optionally substituted with phenyl group, a lower alkyl group, pyridylmethyl group, cycloalkylalkyl group, adamantanemethyl group, furylmethyl group, cycloalkyl group, a lower alkoxycarbonyl group or acyl group,
q denotes a whole number of 1 to 3, and 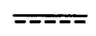 denotes a single bond or a double bond
or their pharmaceutically acceptable salts, a practical embodiment being the following compound.

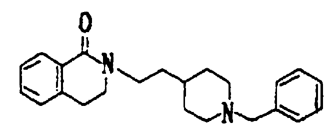

In USP4,064,255, there are disclosed pharmaceutical compositions containing a compound represented by the general formula: 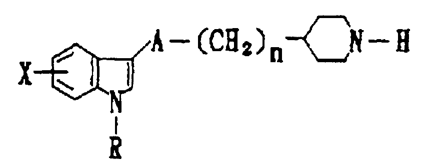 wherein R stands for H, a C₁₋₄ alkyl group or an aralkyl group whose alkyl moiety has one or two carbon atoms; X stands for H or a halogen atom, alkyl, alkoxy or alkylthio group, each optionally having one to four carbon atoms, trifluoromethyl, nitro, hydroxyl or unsubstituted amino group or amino group substituted with one or two alkyl groups or acyl or alkylsulfonyl group; A stands for the group -CO- or the group -CH₂-; and n denotes 0, 1 or 2, or a pharmaceutically acceptable salt thereof, which are useful in treatment of pathological conditions caused by disturbances in serotonin systems, and, in USP 4,208,417, there are disclosed indole derivatives represented by the general formula: 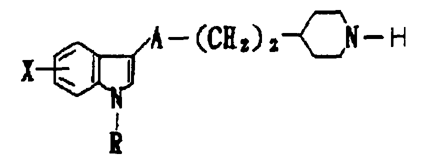 wherein R stands for H, a C₁₋₄ alkyl group or an aralkyl group whose alkyl moiety has one or two carbon atoms; X stands for H or a halogen atom, alkyl group, alkoxy group or an alkylthio group whose alkyl moiety has one to four carbon atoms; A stands for -CO- or -CH₂-; and n denotes 1 or 2, which are medicinally active compounds having affinity for the ³H-diazepam binding site.

Further, in WO91/03243, there are disclosed compounds represented by the general formula: 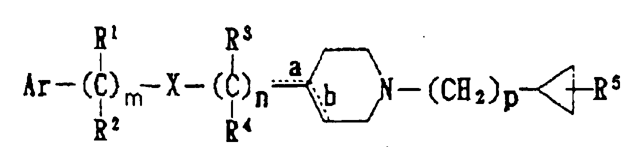 wherein m denotes 0 to 3; n denotes 0 to 3; m and n are not 0 simultaneously; p denotes 0 to 3; X stands for O, S, SO, SO₂, NR⁶, CR⁷R⁸, CO or CHOH; R¹, R³ and R⁷ each stand for H, C₁₋₅ alkyl, halogen, NR¹⁰R¹¹, OH, COOH, C₂₋₆ carboalkoxy, CN, Ar, C₁₋₅ alkoxy or C₁₋₅ alkylthio; R², R⁴ and R⁸ each stand for H, C₁₋₅ alkyl, C₂₋₆ carboalkoxy, CN, C₁₋₅ alkoxy or Ar¹; when X is O, S, SO, SO₂ or NR⁶, R¹, R², R³ and R⁴ are not C₁₋₅ alkoxy, C₁₋₅ alkylthio, NR¹⁰R¹¹ or OH; R⁵ stands for H, alkyl, halogen, OH or alkenyl; R⁶ stands for H, C₁₋₅ alkyl or Ar¹; Ar and Ar¹ respectively stand for naphthyl, pyridyl, pyrimidyl, indolyl, quinolinyl or phenyl, these groups being optionally substituted with C₁₋₃ alkyl, C₁₋₃ alkoxy, C₁₋₃ haloalkyl having 1 to 7 halogen atoms, SH, S(O)ₜ-C₁₋₃ alkyl (t denotes 1, 2 or 3), C₂₋₆ dialkylamino, halogen, C₁₋₃ alkylamino, NH₂, CN, NO₂, SO₃H, tetrazole, COOH, C₂₋₆ carboalcoxy, CONH₂, SO₂NO₂, COR⁹, CONR¹²R¹³, SO₂NR¹²R¹³, Ar², OAr² or SAr²; Ar² stands for naphthyl group or phenyl group, these groups being optionally substituted with C₁₋₃ alkyl, C₁₋₃ haloalkyl having one to seven halogen atoms, C₁₋₃ alkoxy, halogen or C₁₋₃ alkylthio; R⁹, R¹⁰, R¹¹, R¹² and R¹³ respectively stand for H, C₁₋₅ alkyl or phenyl, R¹⁰ and R¹¹ may, taken together, form C₃₋₆ alkylene chain, R¹² and R¹³ may, taken together, form C₃₋₆ alkylene chain; a or b shows double bond or single bond, and both are not double bond, or pharmaceutically acceptable salts thereof, which are useful in the treatment of physiological or drug-induced psychosis or dyskinesia.

On the other hand, a variety of O-containing or S-containing condensed ketone derivatives have been produced, and their biological activity and pharmacological actions are disclosed. However, nothing was disclosed on the actions as cholinesterase inhibitor and therapeutic or/and prophylactic agents of senile dementia.

More specifically, in Chem. Abstr., 107, 190332h (1987), there is disclosed that the compound represented by the general formula: 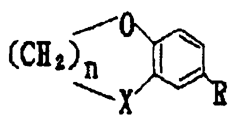 (wherein R=Ac, COEt, COPr, COCHMe₂, CO(CH₂)₂Cl, CO(CH₂)₃Cl, COCH₂NMe₂, CO(CH₂)₂NMe₂, CO(CH₂)₃NMe₂, and salts of them or R=COCH=CHPh, X=CH₂ or O, n=1, 2 or 3) has an antiinflammatory action. In Chem. Abstr., 89, 36594y (1978), there is disclosed that the compound represented by the general formula: 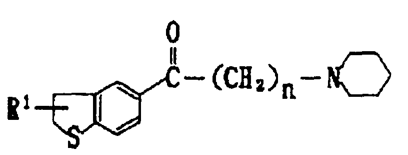 (wherein R¹=H, Me; n=2, 3) has a convulsive action, an arterial blood pressure lowering action and a local anesthetic action.

In Chem. Abstr., 87, 152125d (1977), there is disclosed that the compound represented by the general formula: 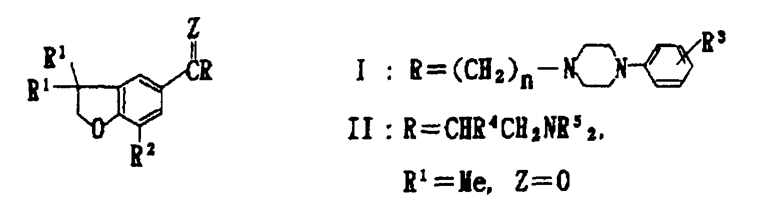 wherein R¹=H, Me; R²=H, Cl, Me; R³=H, F, Me, OMe, Cl; n=1, 2, 3; Z=O, OH, H (for Compound I) or R²=H, Cl; R⁴=H, Me; NR⁵=NMe₂, morpholino, piperidino (for Compound II) has an antidepressant action.

In EP-163,537, there is disclosed the compound represented by the general formula: 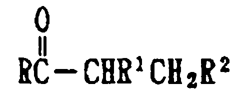 (wherein R=4-cycloalkylphenyl, 3,4-methylenedioxyphenyl, 2,3-dihydro-5-benzofuranyl; R¹=alkyl, cycloalkyl, cyclopentylmethyl; R²=optionally substituted pyrrolidino, piperidino, hexahydro-1H-azepin-1-yl, octahydro-1-azocinyl) has a muscle-relaxing action.

In Chem.Abstr., 91, 211631y (1979), there is disclosed that the compound represented by the formula: 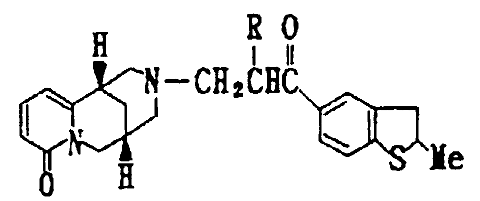 (wherein R=H, Me) is synthesized as a derivative of cytisine, an alkaloid, having an anticholinergic action.

And, as N-containing condensed heterocyclic ketone derivative, in Helvetica Chimica Acta, 51, 1616 (1968), the compounds represented by the formula (A) and the formula (B): 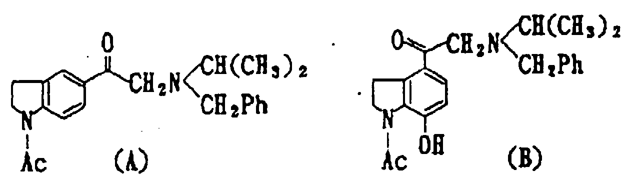 are disclosed as intermediates for synthesizing alkanolamine, an agent of acting on sympathetic nervous system, represented by the formula (C):

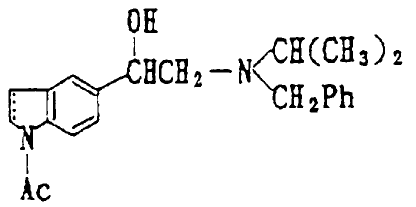

US-A-4 521 606 discloses 5-indolyl substituted aminoethanols for treating hypertension.

From EP-A-0 294 647 thiadiazinones are known which can be used for the treatment of cardiovascular disorders.

In these days when senile dementia is increasing, however, there is needed development of excellent therapeutic or/and prophylactic agents having a stronger action and longer action and less toxicity than the compounds already known to have therapeutic or/and prophylactic efficacy on senile dementia.

The present invention was accomplished by succeeding in creation of novel compounds having a condensed heterocyclic group of specific chemical structure and by finding that these novel compounds have unexpectedly excellent cholinesterase inhibitory activity and monoamine reuptake inhibitory activity, thus being useful as therapeutic or/and prophylactic agents for senile dementia. More specifically, the present invention relates to;
(1) condensed heterocyclic ketone derivatives of the formula: 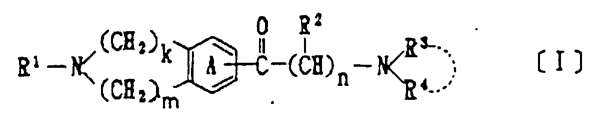 wherein R¹ stands for H, an optionally substituted hydrocarbon group or an optionally substituted acyl group; ring A stands for an optionally further substituted benzene ring; n denotes a whole number of 1 to 10; R², R³ and R⁴ independently stand for H or an optionally substituted hydrocarbon group; R³ and R⁴ may form an optionally substituted heterocyclic group, taken together with the adjacent nitrogen atom; R²'s may be different from one another in the repetition of n; k denotes a whole number of 0 to 3; and m denotes a whole number of 1 to 8; provided that when k=0 and m=2, n denotes a whole number of not less than 2, or salts thereof,
(2) a method of producing the compound [I] or a salt thereof, which comprises reacting a compound represented by the formula: 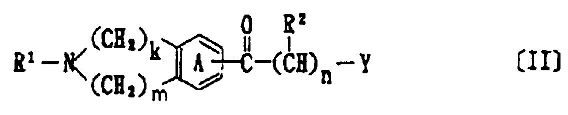 wherein Y stands for a leaving group; R¹, ring A, R², n, k and m are of the same meanings as defined above, or a salt thereof with a compound represented by the formula: 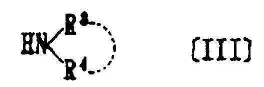 wherein R³ and R⁴ are of the same meanings as defined above, or a salt thereof,
(3) a method of producing a compound represented by the formula: 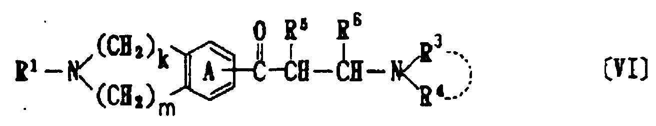 wherein R¹, R³, R⁴, ring A, k and m are of the same meanings as defined above, R⁵ stands for H or an optionally substituted hydrocarbon group, and R⁶ stands for H or an optionally substituted hydrocarbon group, or a salt thereof, which comprises reacting a compound represented by the formula: 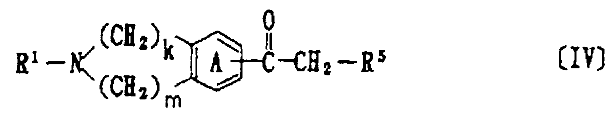 wherein R¹, R⁵, ring A, k and m are of the same meanings as defined above, or a salt thereof and a compound represented by the formula:

   R⁶-CHO [V]

   wherein R⁶ is of the same meaning as defined above with a compound represented by the formula 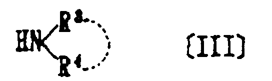 wherein R³ and R⁴ are of the same meanings as defined above, or a salt thereof,
(4) compounds represented by the formula: 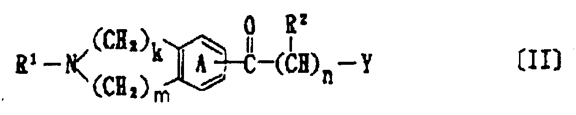 wherein Y, R¹, ring A, R², n, k and m are of the same meanings as defined above, or salts thereof,
(5) a cholinesterase inhibitor, which contains a condensed heterocyclic ketone derivative represented by the formula: 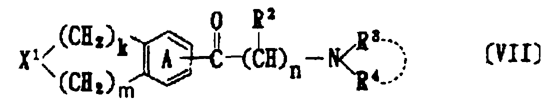 wherein X¹ stands for R¹-N (wherein R¹ stands for H, an optionally substituted hydrocarbon group or an optionally substituted acyl group), O or S; ring A stands for an optionally further substituted benzene ring; n denotes a whole number of 1 to 10; R², R³ and R⁴ independently stand for H or an optionally substituted hydrocarbon group; R³ and R⁴ may form an optionally substituted heterocyclic group, taken together with the adjacent nitrogen atom; R²'s may be different from one another in the repetition of n; k denotes a whole number of 0 to 3; and m denotes a whole number of 1 to 8, or a salt thereof, and
(6) a therapeutic or/and prophylactic agent for senile dementia which contains the compound [VII] or a salt thereof.

The compound [I] or salts thereof of this invention are novel compounds having structural characteristics in that the heterocyclic ring containing a hetero atom (O, S or N) condensed on the benzene ring is saturated one and a substituent 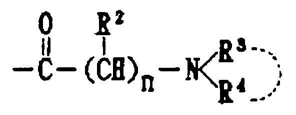 is bonded to the carbon atom on the benzene ring, and, based on these characteristics, these compounds show excellent therapeutic or/and prophylactic actions for senile dementia.

In the foregoing formulae, R¹ stands for H, an optionally substituted hydrocarbon group or an optionally substituted acyl group.

R² stands for H or an optionally substituted hydrocarbon group, and R²'s may be different from one another in the repetition of n.

R³ and R⁴ stand for H or an optionally substituted hydrocarbon group, and, they may form, taken together with the adjacent nitrogen atom, an optionally substituted heterocyclic group.

R⁵ and R⁶ stand for H or an optionally substituted hydrocarbon group.

Examples of "hydrocarbon group" of "optionally substituted hydrocarbon group" shown by the above-mentioned mentioned R¹, R², R³, R⁴, R⁵ and R⁶ include chain-like or cyclic or their combined type C₁₋₁₈ hydrocarbon groups. Examples of the chain-like hydrocarbon groups include straight-chain or branched C₁₋₁₁ alkyl groups (e.g. methyl, ethyl, n-propyl, i-propyl, n-butyl, i-butyl, tart-butyl, n-pentyl, n-hexyl, etc.), straight-chain or branched C₂₋₄ alkenyl groups (e.g. vinyl, allyl, 2-butenyl, etc.) and straight-chain or branched C₂₋₄ alkynyl groups (e.g. propargyl, 2-butynyl, etc.). Examples of the cyclic hydrocarbon groups include C₃₋₇ monocyclic cycloalkyl groups (e.g. cyclobutyl, cyclopentyl, cyclohexyl etc.), C₈₋₁₄ bridge ring saturated hydrocarbon groups (e.g. bicyclo[3.2.1]oct-2-yl, bicyclo[3.3.1]non-2-yl, adamantan-1-yl, etc.), C₆₋₁₄ aryl groups (e.g. phenyl group, naphthyl group, etc.), among others.

Examples of hydrocarbon groups composed of chain-like and cyclic ones include C₇₋₁₈ aralkyl (phenyl-C₁₋₁₂ alkyl or naphthyl-C₁₋₈ alkyl such as phenylmethyl, phenylethyl, phenylpropyl, phenylbutyl, phenylpentyl, phenylhexyl or α-naphthylmethyl; diphenyl-C₁₋₃ alkyl such as diphenylmethyl or diphenylethyl), C₆₋₁₄ aryl-C₂₋₁₂ alkenyl (phenyl-C₂₋₁₂ alkenyl such as styryl, cinnamyl, 4-phenyl-2-butenyl or 4-phenyl-3-butenyl), C₆₋₁₄ aryl-C₂-₁₂ alkynyl (phenyl-C₂₋₁₂ alkynyl such as phenylethynyl, 3-phenyl-2-propynyl or 3-phenyl-1-propynyl), C₃₋₇ cycloalkyl-C₁₋₆ alkyl (e.g. cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl, cyclohexylhexyl, cycloheptylhexyl, etc.).

Preferable examples of the "hydrocarbon group" of the "optionally substituted hydrocarbon group" shown by R¹, R², R³, R⁴, R⁵ and R⁶ include straight-chain or branched C₁₋₁₁ alkyl groups, especially straight-chain or branched C₁₋₇ alkyl groups (e.g. methyl, ethyl, n-propyl, propyl, i-propyl, n-butyl, i-butyl, tert-butyl, n-pentyl, pentyl, n-hexyl, etc.) or C₇₋₁₈ aralkyl groups, especially C₇₋₁₀ aralkyl groups (e.g. phenyl-C₁₋₄ alkyl such as phenylmethyl, phenylethyl and phenylpropyl).

The hydrocarbon groups shown by R¹, R², R³, R⁴, R⁵ and R⁶ may optionally have substituents, and, as such substituents, use is properly made of those generally used as substituents of hydrocarbon groups. More specifically, as substituents which the above-mentioned C₁₋₁₁ alkyl, C₂₋₄ alkenyl, C₂₋₄ alkynyl, C₃₋₇ monocyclic cycloalkyl and C₈₋₁₄ bridge ring saturated hydrocarbon groups may have, use is made of one to five of those selected from halogen atoms (e.g. fluorine, chlorine, bromine, iodine, etc.), nitro group, cyano group, hydroxyl group, C₁₋₄ alkoxy groups (e.g. methoxy, ethoxy, propyloxy, butyloxy, isopropyloxy, etc.), C₁₋₄ alkylthio groups (e.g. methylthio, ethylthio, propylthio, etc.), amino group, mono- or di- C₁₋₄ alkylamino group (e.g. methylamino, ethylamino, propylamino, dimethylamino, diethylamino, etc.), 5- to 7-membered cyclic amino groups (e.g. pyrrolidino, piperidino, morpholino, etc.), C₁₋₄ alkyl-carbonylamino groups (e.g. acetylamino, propionylamino, butyrylamino, etc.), C₁₋₄ alkylsulfonylamino groups (e.g. methylsulfonylamino, ethylsulfonylamino, etc.), C₁₋₄ alkoxy-carbonyl groups (e.g. methoxycarbonyl, ethoxycarbonyl, propoxycarbonyl, etc.), carboxyl group, C₁₋₆ alkyl-carbonyl groups (e.g. methylcarbonyl, ethylcarbonyl, propylcarbonyl, etc.), carbamoyl group, mono- or di-C₁₋₄ alkylcarbamoyl groups (e.g. methylcarbamoyl, ethylcarbamoyl, etc.), C₁₋₆ alkylsulfonyl groups (e.g. methylsulfonyl, ethylsulfonyl, propylsulfonyl, etc.), C₁₋₄ alkylenedioxy (e.g. methylenedioxy, etc.), 5- or 6- membered heterocyclic groups or its condensed ring containing 1 to 3 hetero atoms selected from N,S and 0 other than carbon atoms which may be substituted with a C₁₋₄ alkyl (e.g. pyridyl, pyrazinyl, pyrimidinyl, quinolinyl, isoquinolinyl, naphthylidinyl, thiazolyl, benzothiazolyl, benzoxazolyl, furyl, furanyl, thiophenyl etc.).

Examples of the substituents, which the C₆₋₁₄ aryl groups shown by R¹, R², R³, R⁴, R⁵ and R⁶ may have, include C₁₋₄ alkyl groups (e.g. methyl, ethyl, propyl, butyl, etc.), halogen atoms (e.g. fluorine, chlorine, bromine, iodine, etc.), nitro group, cyano group, hydroxyl group, C₁₋₄ alkoxy groups (e.g. methoxy, ethoxy, propyloxy, butyloxy, isopropyloxy, etc.), C₁₋₄ alkylthio groups (e.g. methylthio, ethylthio, propylthio, isopropylthio, butylthio, etc.), amino group, mono- or di-C₁₋₄ alkylamino groups (e.g. methylamino, ethylamino, propylamino, dimethylamino, diethylamino, etc.), 5- to 7-membered cyclic amino groups (e.g. pyrrolidino, piperidino, morpholino, etc.), C₁₋₄ alkyl-carbonylamino groups (e.g. acetylamino, propionylamino, butyrylamino, etc.), C₇₋₁₈ aralkyloxy (e.g. phenylmethoxy, phenylethoxy, etc.), aminocarbonyloxy group, mono- or di-C₁₋₄ alkylaminocarbonyloxy groups (e.g. methylaminocarbonyloxy, ethylaminocarbonyloxy, dimethylaminocarbonyloxy, diethylaminocarbonyloxy, etc.), C₁₋₄, alkylsulfonylamino groups (e.g. methylsulfonylamino, ethylsulfonylamino, propylsulfonylamino, etc.), C₁₋₄ alkoxy-carbonyl groups (e.g. methoxycarbonyl, ethoxycarbonyl, propoxycarbonyl, isobutoxycarbonyl, etc.), carboxyl group, C₁₋₆ alkylcarbonyl groups (e.g. methylcarbonyl, ethylcarbonyl, butylcarbonyl, etc.), C₃₋₇ cycloalkyl-carbonyl (e.g. cyclohexylcarbonyl, etc.), carbamoyl group, mono- or di-C₁₋₄ alkyl-carbamoyl groups (e.g. methylcarbamoyl, ethylcarbamoyl, propylcarbamoyl, butylcarbamoyl, diethylcarbamoyl, dibutylcarbamoyl, etc.), C₁₋₆ alkylsulfonyl groups (e.g. methylsulfonyl, ethylsulfonyl, propylsulfonyl, etc.), C₃₋₇ cycloalkylsulfonyl (e.g. cyclopentylsulfonyl, cyclohexylsulfonyl, etc.), C₁₋₄ alkylenedioxy (e.g. methylenedioxy, etc.) as well as phenyl, naphthyl, mono- or di-phenyl-C₁₋₃ alkyl (e.g. benzyl, diphenylmethyl, etc.), phenoxy, benzoyl, phenoxycarbonyl, benzylcarbonyl, phenyl-C₁₋₄ alkylcarbamoyl, phenylcarbamoyl, phenyl-C₁₋₄ alkylcarbonylamino, benzoylamino, phenyl-C₁₋₄ alkylsulfonyl, phenylsulfonyl, phenyl-C₁₋₄ alkylsulfinyl, phenyl-C₁₋₄ alkylsulfonylamino or phenylsulfonylamino which may have 1 to 4 substituents selected from the group consisting of C₁₋₄ alkyl groups such as methyl, ethyl, propyl, butyl, isopropyl, etc., C₁₋₄ alkoxy groups such as methoxy, ethoxy, n-propyloxy, i-propyloxy, n-butyloxy, etc., halogen atoms such as chlorine, bromine, iodine, etc., hydroxyl group, benzyloxy group, amino group, mono- or di-C₁₋₄ alkylamino groups as described above, nitro group, C₁₋₆ alkyl-carbonyl groups as described above, benzoyl group, etc. The number of substituents which may optionally be substituted on these C₆₋₁₄ aryl groups is suitably about 1 to 5.

As the substituents, which C₇₋₁₈ aralkyl, C₆₋₁₄ aryl-C₂₋₁₂ alkenyl, C₆₋₁₄ aryl-C₂₋₁₂ alkynyl, C₃₋₇ cycloalkyl-C₁₋₆ alkyl groups may optionally have, use is made of, for example, those similar to the substituents which the above-mentioned C₆₋₁₄ aryl groups may optionally have. Suitable number of the substituents, which these C₇₋₁₈ aralkyl, C₆₋₁₄ aryl-C₂₋₁₂ alkenyl, C₆₋₁₄ aryl-C₂₋₁₂ alkynyl, C₃₋₇ cycloalkyl-C₁₋₆ alkyl may optionally have, ranges from 1 to 5.

And, R³ and R⁴ may form an optionally substituted heterocyclic group, taken together, with the adjacent nitrogen atom. As the "heterocyclic group" of this "optionally substituted heterocyclic group", use is made of, for example, such heterocyclic groups as containing, other than carbon atoms and one nitrogen atom, optionally 1 to 3 hetero atoms e.g. nitrogen, oxygen or sulfur atom, especially 3- to 13-membered heterocyclic groups. Practically, saturated monocyclic, non-conjugated unsaturated monocyclic, unsaturated monocyclic, polycyclic and bridged heterocyclic groups, for example, are employed.

Examples of the saturated monocyclic heterocyclic group include 5- to 9-membered saturated monocyclic heterocyclic groups such as pyrrolidinyl, piperidinyl, hexamethyleniminyl, heptamethyleniminyl, oxazolidinyl, morpholinyl, thiazolidinyl, thiomorpholinyl, imidazolidinyl, piperazinyl and homopiperazinyl.

Examples of the non-conjugated unsaturated monocyclic or unsaturated monocyclic heterocyclic groups include 5- to 9-membered ones such as pyrrolyl, 1,2-dihydropyridinyl, 1,4-dihydropyridinyl, 1,2,3,6-tetrahydropyridinyl, 2-oxazolidonyl, 2-thiazolidonyl, imidazolyl, pyrazolyl and 1,4,5,6-tetrahydropyrimidinyl.

Examples of the polycyclic heterocyclic group include 2,3-dihydro-1H-indolyl, 1,2,3,4-tetrahydroquinolinyl, 2,3,4,5-tetrahydro-1H-1-benzazepinyl, 2,3-dihydro-1H-isoindolyl, 1,2,3,4-tetrahydroisoquinolinyl, 2,3,4,5-tetrahydro-1H-2-benzazepinyl, 2,3,4,5-tetrahydro-1H-3-benzazepinyl, 1,2,3,4,5,6-hexahydro-1-benzazocinyl, 1,2,3,4,5,6-hexahydro-2-benzazocinyl, 1,2,3,4,5,6-hexahydro-3-benzazocinyl, 2,3,4,5,6,7-hexahydro-1H-1-benzazonyl, 2,3,4,5,6,7-hexahydro-1H-2-benzazonyl, hexahydro-1H-3-benzazonyl, 2,3,4,5,6,7-hexahydro-1H-4-benzazonyl, β-carbolinyl, phenoxazinyl, phenothiazinyl, indolyl, 3H-3-benzazepinyl, 3,4-dihydroquinolinyl, benzimidalyl, 1,4-benzodiazepinyl, etc.

Examples of the bridged heterocyclic group include 1,8-diazaspiro[4.5]decanyl, 1,3,8-triazaspiro[4.5]decanyl, 1,3,8-triazaspiro[4.5]decanyl, diazaspiro[5.5]undecanyl, 1-oxa-3,9-diazaspiro[5.5]undecanyl, 7-azabicyclo[2.2.1]heptanyl, 8-azabicyclo[3.2.1]octanyl, 9-azabicyclo[3.3.1]nonanyl, etc.

Preferable examples of "heterocyclic group" of "optionally substituted heterocyclic group" optionally formed by R³ and R⁴, taken together, with the adjacent nitrogen atom include the afore-described saturated monocyclic heterocyclic groups, polycyclic heterocyclic groups or bridged heterocyclic groups. Specifically, pyrrolidinyl, piperidinyl, piperazinyl, morpholinyl, 1,2,3,4-tetrahydroquinolinyl, 1,2,3,4-tetrahydroisoquinolinyl, 2,3,4,5-tetrahydro-1H-3-benzazepinyl 2,3,4,5-tetrahydro-1H-2-benzazepinyl, 2,3,4,5-tetrahydro-1H-3-benzazepinyl and 1,3,8-triazaspiro[4,5]decanyl are preferable, especially, pyrrolidinyl, piperidinyl, piperazinyl, morpholinyl and 1,2,3,4-tetrahydroquinolinyl are often employed.

As "substituent" of "optionally substituted heterocyclic group", which may optionally be formed by the above-mentioned R³ and R⁴, taken together, with the adjacent nitrogen atom, use is made of 1 to 5 groups, preferably 1 or 2 groups, selected from, for example, optionally substituted hydrocarbon groups referring to the above-mentioned R¹, R², R³, R⁴, R⁵ and R⁶, halogen atoms (e.g. fluorine, chlorine, bromine, iodine, etc.), nitro group, cyano group, hydroxyl group, C₁₋₄ alkoxy groups (e.g. methoxy, ethoxy, propyloxy, butyloxy, isopropyloxy, etc.), C₁₋₄ alkylthio groups (e.g. methylthio, ethylthio, propylthio, isopropylthio, etc.), aminogroup, mono- or di-C₁₋₄ alkylamino groups (e.g. methyl amino, ethylamino, propylamino, dimethylamino, diethylamino, etc.), C₁₋₄ alkylcarbonylamino groups (e.g. acetylamino, propionylamino, butyrylamino, etc.), C₁₋₄ alkyl-sulfonylamino groups (e.g. methylsulfonylamino, ethylsulfonylamino, etc.), C₁₋₄ alkoxy-carbonyl groups (e.g. methoxycarbonyl, ethoxycarbonyl, propoxycarbonyl, etc.), carboxyl group, formyl group, C₁₋₆ alkyl-carbonyl groups (e.g. methylcarbonyl, ethylcarbonyl, propylcarbonyl, etc.), C₁₋₄ alkyl-carbonyloxy groups (e.g. acetyloxy, ethylcarbonyloxy, etc.), ω-oxo-ω-(tetrahydrobenzazepinyl)C₁₋₆ alkyl groups (e.g. 1-oxo-1-(tetrahydrobenzazepinyl)methyl, 2-oxo-2-(tetrahydrobenzazepinyl)ethyl, 3-oxo-3-(tetrahydrobenzazepinyl)propyl, etc.), optionally substituted benzoyl groups (herein, as substituents, use is made of 1 to 3 substituents selected from C₁₋₄ alkyl, for example, methyl, ethyl, etc., halogen, for example, fluorine, chlorine, bromine, etc., C₁₋₄ alkoxy, for example, methoxy, ethoxy, etc., mono- or di-C₁₋₄ alkylamino, for example, methylamino, dimethylamino, etc., 5- to 7-membered cyclic amino groups, for example, piperidino, morpholino, etc., nitro, hydroxy, etc.; practical examples of them being benzoyl, 4-fluorobenzoyl, 3,4-dimethoxybenzoyl, etc.), carbamoyl group, mono- or di-C₁₋₄ alkylcarbamoyl groups (e.g. methylcarbamoyl, ethylcarbamoyl, etc.), C₁₋₆ alkylsulfonyl groups (e.g. methylsulfonyl, ethylsulfonyl, propylsulfonyl, etc.), oxo group, the above-mentioned "heterocyclic group" and saturated heterocyclic groups (e.g. pyridyl, pyrazinyl, pyrimidinyl, quinolinyl, isoquinolinyl, napthylidinyl, benzothiazolyl, benzoxazolyl, furanyl, thienyl etc.). Among them, formyl, C₁₋₄ alkyl-carbonyloxy groups (e.g. acetyloxy, etc.), hydroxyl group., oxo group, pyridinyl group, optionally substituted benzoyl groups (e.g. benzoyl groups optionally substituted with halogen such as fluorine, chlorine, bromine, etc.), ω-oxo-ω-(tetrahydrobenzazepinyl) C₁₋₆ alkyl groups (e.g. 1-oxo-1-(tetrahydrobenzazepinyl)methyl, 2-oxo-2-(tetrahydrobenzazepinyl)ethyl, 3-oxo-3-(tetrahydrobenzazepinyl)propyl, etc.), and optionally substituted hydrocarbon groups referring to the above-mentioned R¹, R², R³, R⁴, R⁵, and R⁶ are preferable. Herein, as optionally substituted hydrocarbon groups referred to in R¹, R², R³, R⁴, R⁵ and R⁶, use is often made of, for example, or straight-chain or branched C₁₋₁₁ alkyl groups, especially straight-chain or branched C₁₋₇ alkyl groups (e.g. methyl, ethyl, n-pentyl, n-hexyl, etc.) or C₇₋₁₈ aralkyl groups (e.g. phenyl-C₁₋₁₂ alkyl such as phenylmethyl, phenylethyl, phenylpropyl, phenylhexyl, etc., naphtyl-C₁₋₈ alkyl such as α-naphthylmethyl, etc., diphenyl-C₁₋ₛ alkyl such as diphenylmethyl, etc.), especially C₇₋₁₀ aralkyl groups (e.g. phenylmethyl, phenylethyl, phenylpropyl, etc.), these alkyl and aralkyl groups having optionally halogen (e.g. fluoro, chloro, bromo, etc.), hydroxyl group, C₁₋₄ alkylenadioxy (e.g. methylenedioxy, etc.).

As "acyl group" of "optionally substituted acyl group" shown by R¹, use is made of, for example, carboxylic acid acyl groups (e.g. formyl, C₁₋₈ alkyl-carbonyl or C₆₋₁₄ aryl-carbonyl such as acetyl, propionyl, butyryl, benzoyl, etc.), sulfonic acid acyl groups (e.g. C₁₋₇ alkylsulfonyl or C₆₋₁₄ arylsulfonyl such as methanesulfonyl, ethanesulfonyl, propanesulfonyl, benzenesulfonyl, p-toluenesulfonyl, etc.), phosphonic acid acyl groups (e.g. C₁₋₇ alkylphosphonyl or C₆₋₁₄ arylphosphonyl such as methanephosphonyl, ethanephosphonyl, propanephosphonyl, benzenephosphonyl, etc,), substituted oxycarbonyl groups (e.g. C₁₋₈ alkoxy-carbonyl or C₇₋₁₈ aralkyloxy-carbonyl such as methoxycarbonyl, ethoxycarbonyl, tert-butoxycarbonyl, benzyloxycarbonyl, etc.), heterocyclic-carbonyl groups wherein the heterocyclic group is a 5-or 6- membered one containing 1 to 3 hetero atoms selected from N,S and 0 other than carbon atoms (e.g. pyridylcarbonyl, pyrrolylcarbonyl, quinolylcarbonyl, etc.), carbamoyl group, mono- or di-C₁₋₄ alkylcarbamoyl groups (e.g. methylcarbamoyl, ethylcarbamoyl, etc.), etc. Among them, C₁₋₈ alkyl-carbonyl or C₁₋₈ alkoxycarbonyl mentioned above is preferable.

As the substituent which these acyl groups may have, use is made of 1 to 3, preferably 1 to 2 substituents selected from halogen atoms (e.g. fluorine, chlorine, bromine, iodine, etc.), amino group, mono- or di-C₁₋₆ alkylamino groups (e.g. methylamino, ethylamino, propylamino, hexylamino, dimethylamino, diethylamino, etc.) and C₁₋₄ alkoxy groups (e.g. methoxy, ethoxy, propoxy, etc.).

The benzene ring shown by ring A may have, besides the groups represented by the formulae 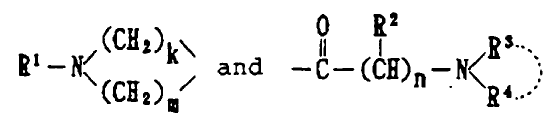 further substituents, and as such substituents, use is made of, for example, those mentioned in reference to C₆₋₁₄ aryl groups of the above-mentioned R¹, R², R³, R⁴, R⁵ and R⁶, the number of them being 1 to 3. Preferable examples of the substituents, which such benzene ring may optionally have, include, among others, halogen such as fluoro, chloro, etc., halogeno-C₁₋₃ alkyl such as trifluoromethyl, etc., C₁₋₃ alkyl such as methyl, etc. and C₁₋₃ alkoxy such as methoxy, etc. Especially, fluoro, for example, is preferable.

The symbol n denotes a whole number of 1 to 10, provided that, in the case of k=0 and m=2, n is more than 1. Preferably, n ranges from 2 to 10, especially from 2 to 8. The symbol k denotes a whole number of 0 to 3, and m denotes a whole number of 1 to 8. In other words, the ring 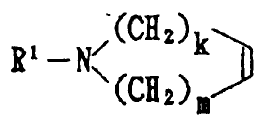 can form a 4- to 14-membered ring.

X¹ stands for R¹-N< (R¹ is of the same meaning as defined above), oxygen atom or sulfur atom.

Preferable examples of R¹ include H, straight-chain or branched C₁₋₁₁ hydrocarbon groups which may be substituted with the above-mentioned substituents (e.g. straight-chain or branched C₁₋₁₁ alkyl such as methyl, ethyl, n-propyl, i-propyl, i-butyl, n-butyl etc., straight-chain or branched C₂₋₄ alkenyl such as vinyl, allyl, 2-butenyl etc., straight-chain or branched C₂₋₄ alkynyl such as propargyl, 2-butynyl etc.), C₇₋₁₈ aralkyl groups which may be substituted with the above-mentioned substituents (e.g. phenylmethyl, (4-methoxyphenyl) methyl, phenylethyl, phenylpropyl, α-naphthylmethyl etc.), formyl group, C₁₋₈ alkyl-carbonyl (e.g. acetyl, propionyl, butyryl etc.), C₆₋₁₄ arylcarbonyl (e.g. benzoyl etc.), C₁₋₈ alkoxy-carbonyl (e.g. methoxycarbonyl, ethoxycarbonyl etc.), heterocyclic-carbonyl (e.g. pyridylcarbonyl etc.), carbamoyl, mono- or di-C₁₋₄ alkyl-carbamoyl (e.g. methylcarbamoyl, ethylcarbamoyl etc.), etc.; especially H, straight-chain or branched C₁₋₄ alkyl groups (e.g. methyl, ethyl, n-propyl, i-propyl, i-butyl, n-butyl etc.), phenyl-C₁₋₃ alkyl groups which may be substituted with 1 to 3 substituents selected from C₁₋₄ alkyl, halogen, nitro, cyano, hydroxy, C₁₋₄ alkoxy and C₇₋₁₈ aralkyloxy (e.g. phenylmethyloxy, (4-methoxyphenyl)methyloxy, phenylethyloxy, phenylpropyloxy etc.), naphthyl-C₁₋₃ alkyl (e.g. α-naphtylmethyl, etc.), C₁₋₄ alkyl-carbonyl (e.g. acetyl, propionyl, butyryl, etc.), phenyloxycarbonyl, C₁₋₄ alkoxy-carbonyl (e.g. methoxycarbonyl, ethoxycarbonyl etc.), etc. More preferable examples of R¹ include H, C₁₋₄ alkyl (e.g. methyl, ethyl, propyl, i-propyl, i-butyl, etc.), C₁₋₄ alkyl-carbonyl (e.g. acetyl, etc.), phenyl-C₁₋₃ alkyl which may be substituted with a C₁₋₄ alkoxy (e.g. phenylmethyl, (4-methoxyphenyl)methyl, phenylethyl, etc.); more especially, C₁₋₄ alkyl group such as i-butyl, etc. or phenyl-C₁₋₃ alkyl which may be substituted by a C₁₋₄ alkoxy such as methoxy, etc (e.g. phenylmethyl, (4-methoxyphenyl)methyl, phenylethyl).

As R², R⁵ and R⁶, are preferable H, straight-chain or branched C₁₋₃ alkyl groups (e.g. methyl, ethyl, n-propyl, i-propyl), phenyl, etc.; especially H is often the case.

Referring to R³ and R⁴, it is preferable that one of them stands for H or straight-chain or branched C₁₋₄ alkyl groups (e.g. methyl, ethyl, n-propyl, i-propyl, n-butyl) and the other stands for straight-chain or branched C₁₋₄ alkyl groups, phenyl-C₁₋₃ alkyl groups (e.g. phenylmethyl, phenylethyl, phenylpropyl) or naphthyl-C₁₋₃ alkyl groups (e.g. α-naphthylmethyl,
And, as each of R³ and R⁴, are also preferable straight-chain or branched C₁₋₄ alkyl group which may be substituted with a hydroxy (e.g. methyl, ethyl, 2-hydroxyethyl, etc.) or phenyl-C₁₋₃ alkyl group which may be substituted with 1 to 3 halogen atoms such as fluorine, chlorine, bromine, etc. (e.g. phenylmethyl, phenylethyl, 2-chloro-phenylmethyl, etc.).

And, such cases as forming heterocyclic rings by R³ and R⁴, taken together, with the adjacent nitrogen atom, are also preferable. Preferable examples include optionally substituted pyrrolidinyl, piperidinyl, piperazinyl, morpholinyl, 1,2,3,4-tetrahydroquinolinyl, 1,2,3,4-tetrahydroisoquinolinyl, 2,3,4,5-tetrahydro-1H-1-benzazepinyl, 2,3,4,5-tetrahydro-1H-2-benzazepinyl, 2,3,4,5-tetrahydro-1H-3-benzazepinyl, 1,3,8-triazaspiro[4.5]decanyl, etc. especially, pyrrolidinyl, piperidinyl, piperazinyl, morpholinyl, 1,2,3,4-tetrahydroisoquinolinyl, etc. Especially preferable examples of the substituents on the heterocyclic groups include formyl group, C₁₋₄ alkyl-carbonyl groups (e.g. acetyl, etc.), hydroxyl group, oxo group, pyridyl group, an optionally substituted benzoyl group (e.g. benzoyl group which may be substituted with 1 to 3 halogen atoms such as fluorine, chlorine, bromine, etc.), optionally substituted straight-chain or branched C₁₋₇ alkyl groups (e.g. methyl, ethyl, n-propyl, i-propyl, n-butyl, tert-butyl, n-pentyl, n-hexyl, which may be substituted with 1 to 3 substituents such as hydroxy group, 5- or 6-membered heterocyclic group containing 1 to 3 hetero atoms of N,S or/and 0 other than carbon atoms such as pyridyl, furyl, thiazol-4-yl, 2-methyl-thiazol-4-yl, etc.), an optionally substituted phenyl group (e.g. phenyl group which may be substituted with 1 to 3 substituents such as halogen (e.g. fluorine, chlorine, bromine, etc.), hydroxy group, C₁₋₄ alkylene-dioxy (e.g. methylenedioxy, etc.), etc.), optionally substituted C₇₋₁₈ aralkyl groups (e.g. phenyl-C₁₋₁₂ alkyl such as phenylmethyl, phenylethyl, phenylpropyl, phenylhexyl, etc., α-naphthylmethyl, diphenyl-C₁₋₈ alkyl such as diphenylmethyl, etc., which may be substituted with 1 to 3 substituents such as halogen (e.g. fluorine, chlorine, bromine, etc.), hydroxy group, C₁₋₄ alkylene-dioxy (e.g. methylenedioxy, etc.), etc.), ω-oxo-ω-(tetrahydrobenzazepinyl)C₁₋₆ alkyl (e.g. 1-oxo-1-(tetrahydrobenzazepinyl) methyl, 2-oxo-2-(tetrahdyrobenzazepinyl) ethyl, 3-oxo-3-(tetrahydrobenzazepinyl) propyl, etc.), the number of them being 1 or 2, preferably 1. As R³ and R⁴, most preferable examples are such cases as forming 4-(phenylmethyl)-piperazin-1-yl or 4-[(2-methylthiazol-4-yl)methyl]-piperazin-1-yl, taken together with the adjacent nitrogen atom.

Preferable benzene ring shown by ring A is unsubstituted one.

Referring to k and m, the case where k+m denotes a whole number of 2 to 6 is preferable, namely the case where the moiety 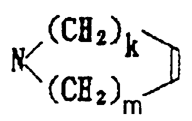 forms a 5- to 9-membered ring. Further, referring to the combination of k and m, it is preferable that, when k is 0, m is 2, 3, 4 or 5; when k is 1, m is 1, 2 or 3; and when k is 2, m is 2. More specifically, preferable examples of N-containing condensed heterocyclic ring represented by the formula: 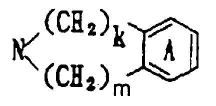 include 2,3-dihydro-1H-indole, 1,2,3,4-tetrahydroquinoline, 2,3,4,5-tetrahydro-1H-1-benzazepine, 2,3-dihydro-1H-isoindole, 1,2,3,4-tetrahydroisoquinoline, 2,3,4,5-tetrahydro-1H-2-benzazepine, 2,3,4,5-tetrahydro-1H-3-benzazepine, 1,2,3,4,5,6-hexahydro-1-benzazocine, 1,2,3,4,5,6-hexahydro-2-benzazocine, 1,2,3,4,5,6-hexahydro-3-benzazocine, 2,3,4,5,6,7-hexahydro-1H-1-benzazonine, 2,3,4,5,6,7-hexahydro-1H-2-benzazonine, 2,3,4,5,6,7-hexahydro-1H-3-benzazonine, 2,3,4,5,6,7-hexahydro-1H-4-benzazonine, etc., especially, 2,3,4,5-tetrahydro-1H-3-benzazepine, 2,3,4,5-tetrahydro-1H-2-benzazepine, 2,3-dihydro-1H-indole, etc.

Preferable ones of the condensed heterocyclic ring moiety in the above formulae shown by the formula: 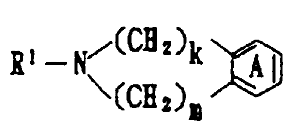 include the N-containing condensed heterocyclic rings shown by the following formulae: 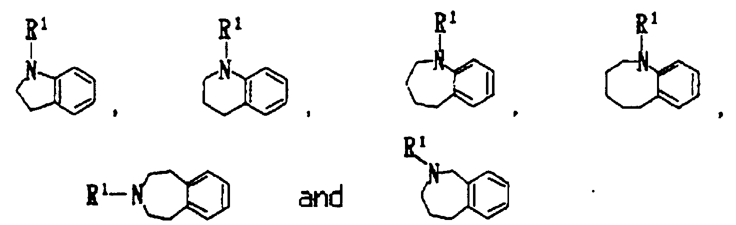 wherein R¹ is of the same meaning as defined above. In the above formulae, preferable examples of R¹ include H, C₁₋₄ alkyl groups (e.g. methyl,ethyl, n-propyl, i-propyl etc.), phenyl-C₁₋₃ alkyl groups which may be substituted by a C₁₋₄ alkoxy (e.g. phenylmethyl, (4-methoxyphenyl)methyl, phenylethyl, phenylpropyl, etc.) or C₁₋₃ alkyl-carbonyl (e.g. acetyl, etc.), etc.

Referring to n, when the group of the formula: 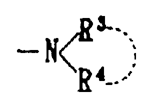 does not form a heterocyclic group, it is preferably a whole number of 3 to 8, and, when the group forms a heterocyclic group, it is preferably a whole number of 2 to 5.

As X¹, R¹-N< is preferable.

More specifically, the following compounds and salts thereof belonging to the compounds of the formula (I) or salts thereof are preferable.

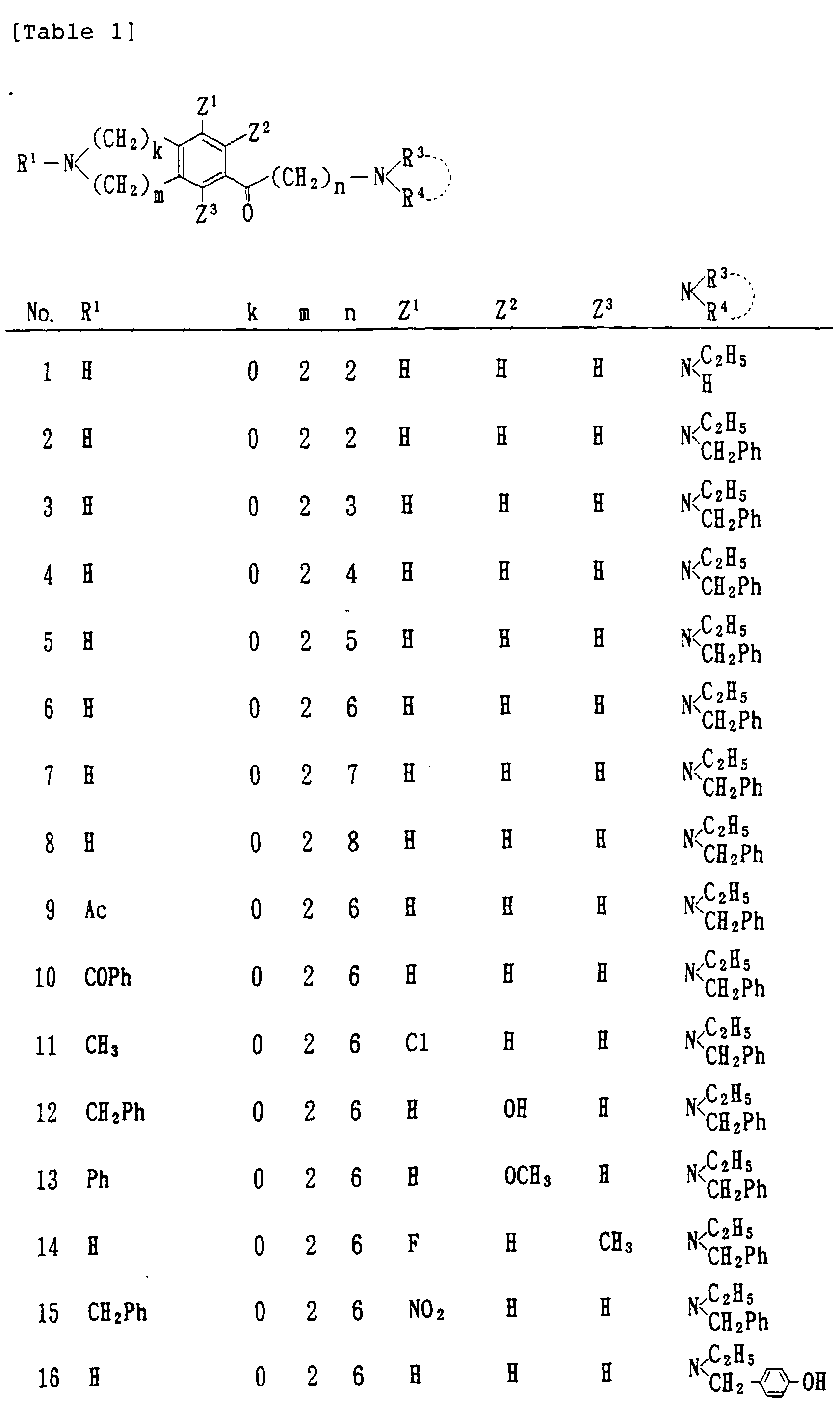

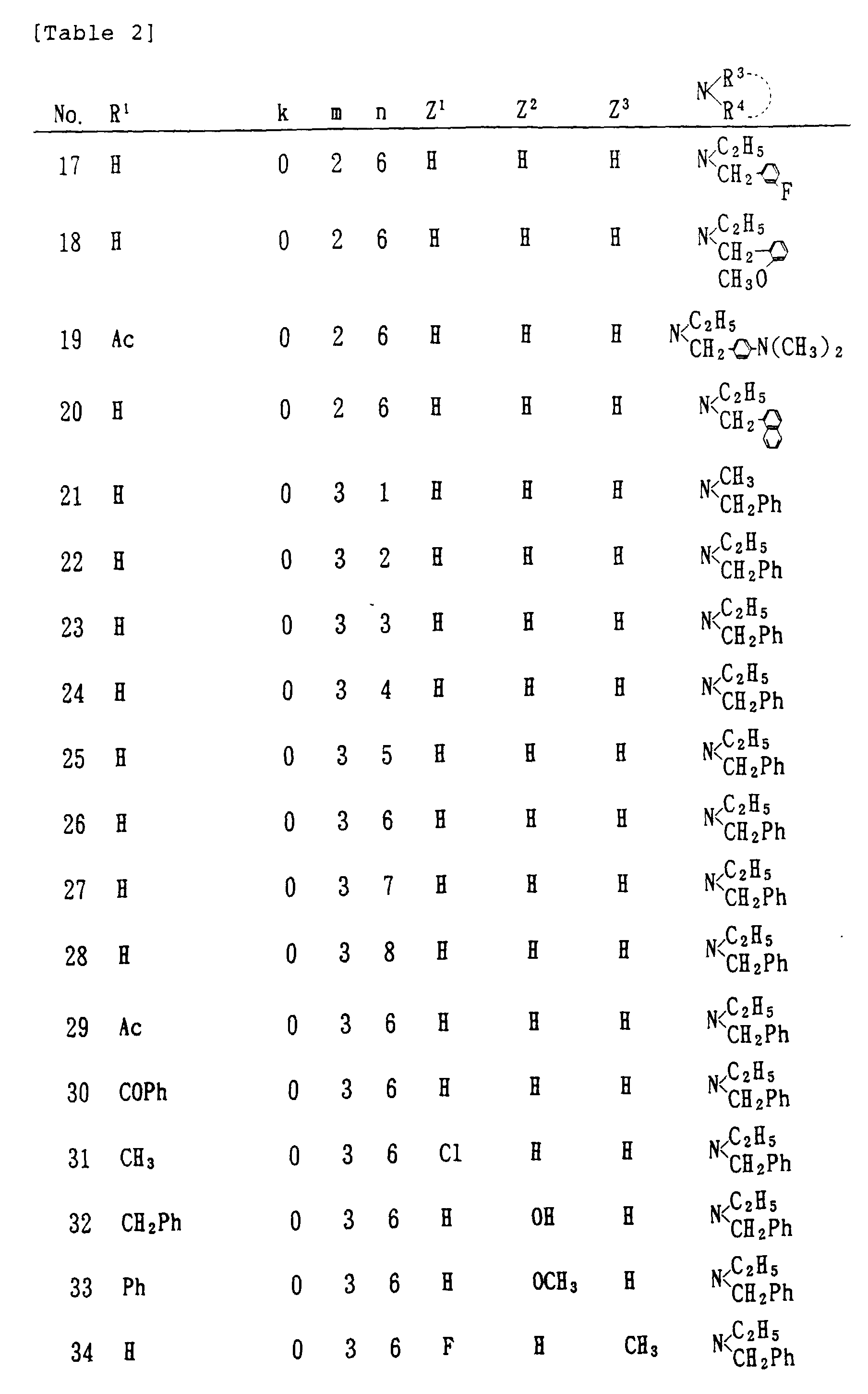

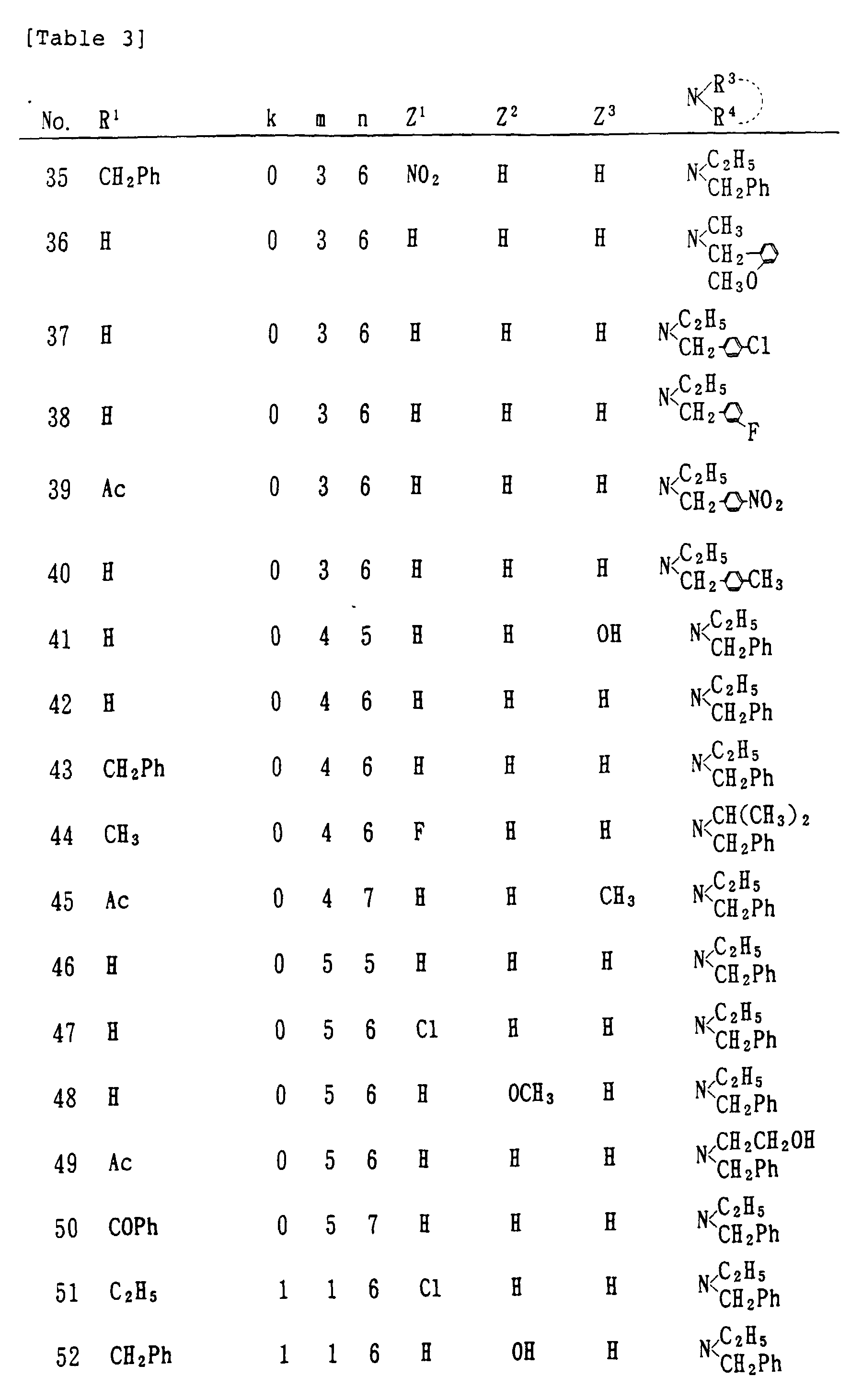

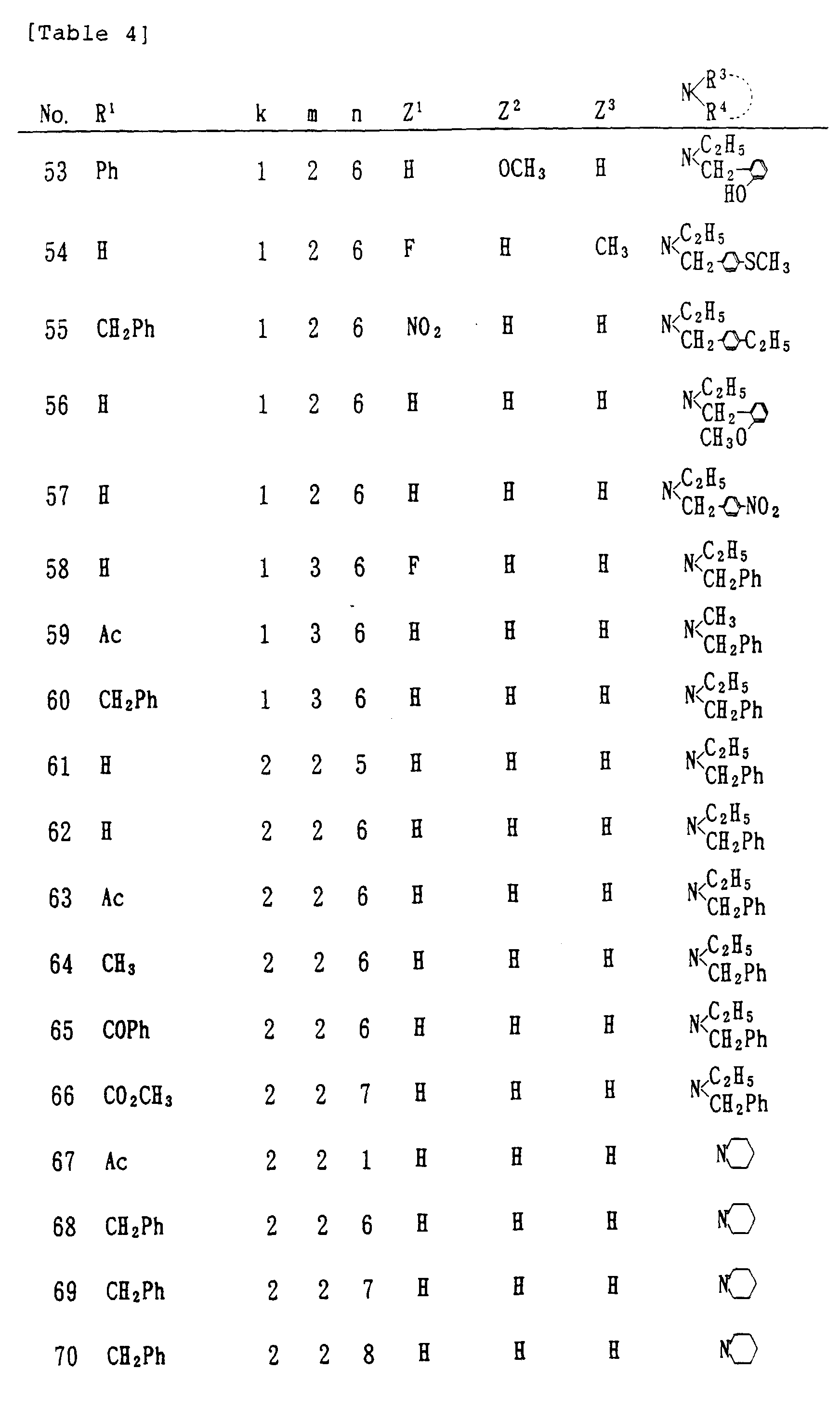

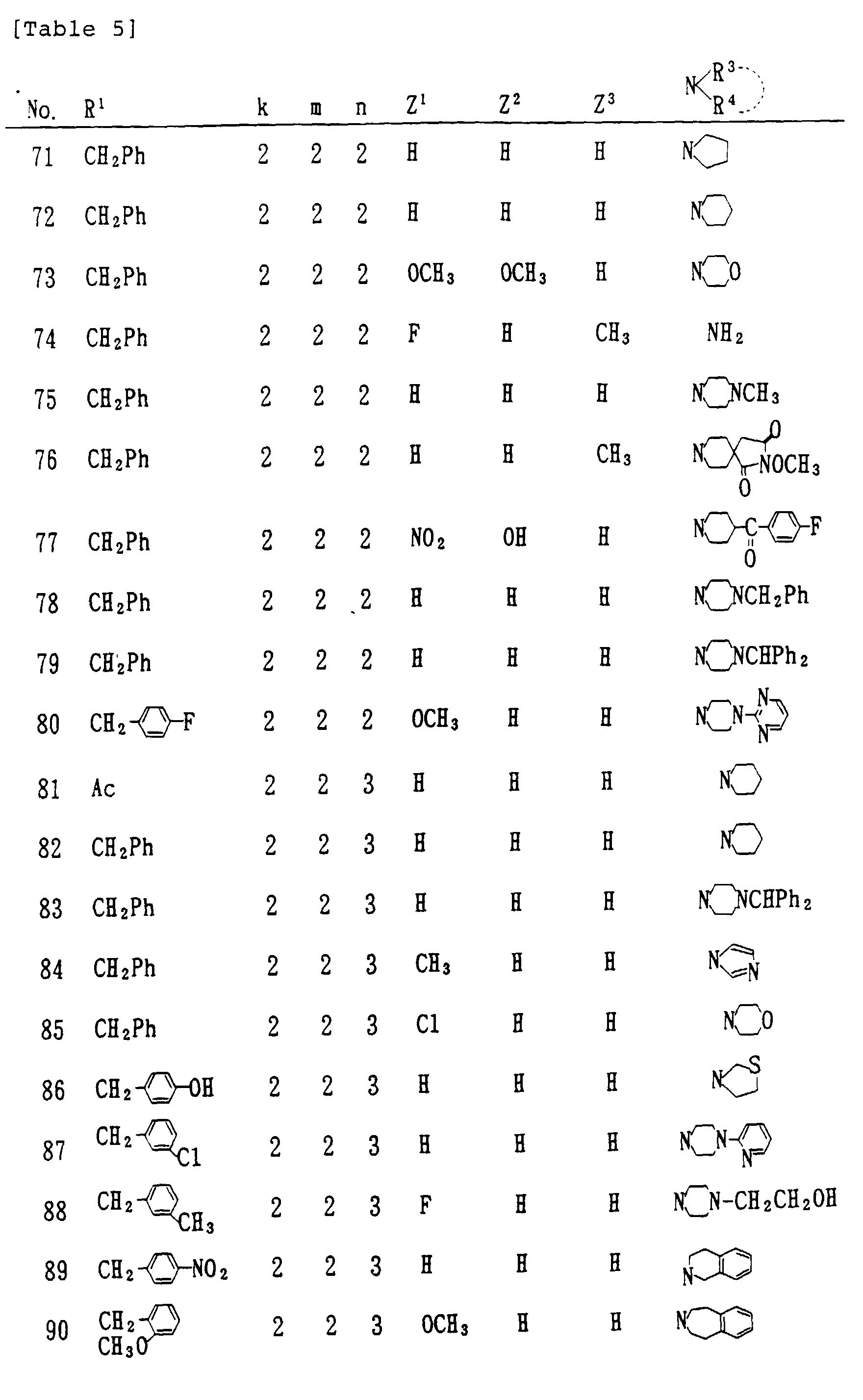

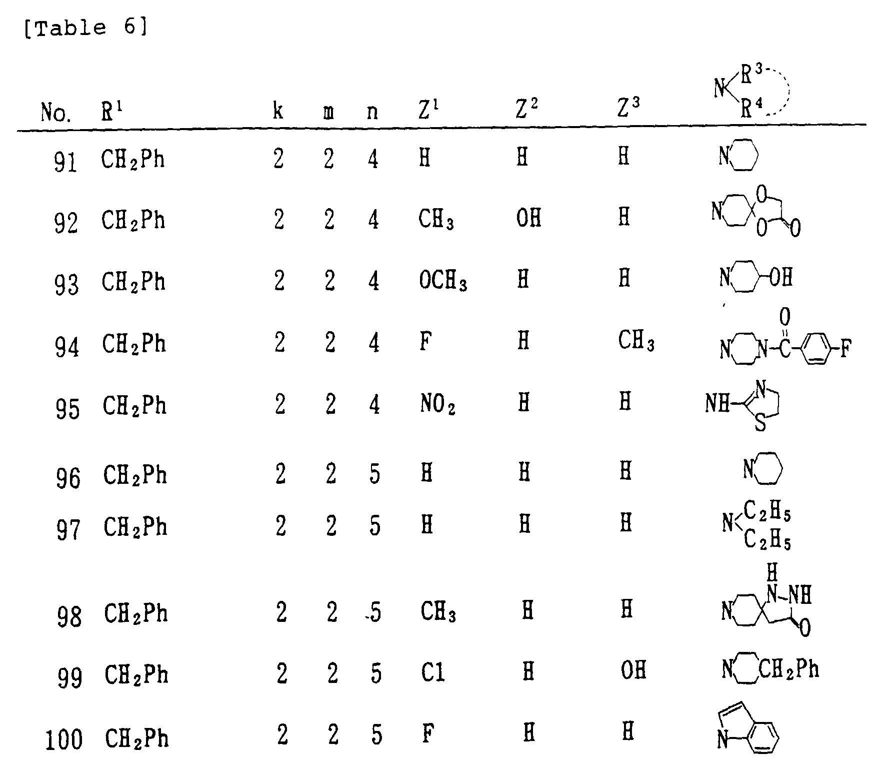

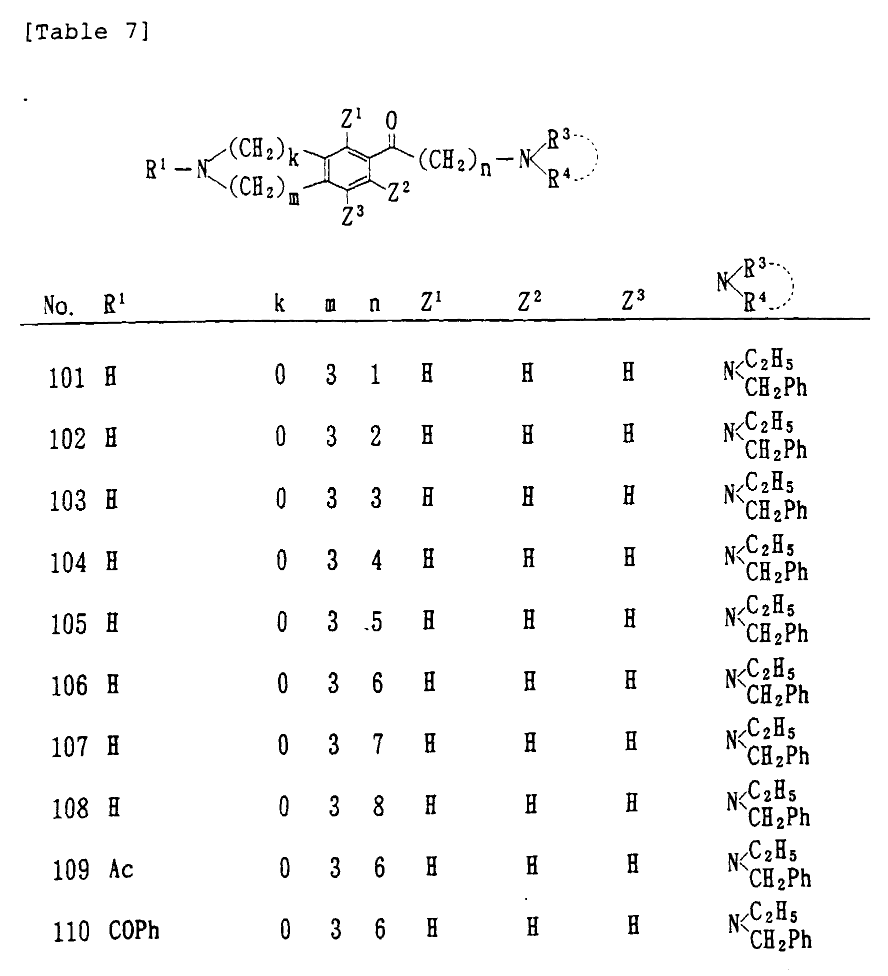

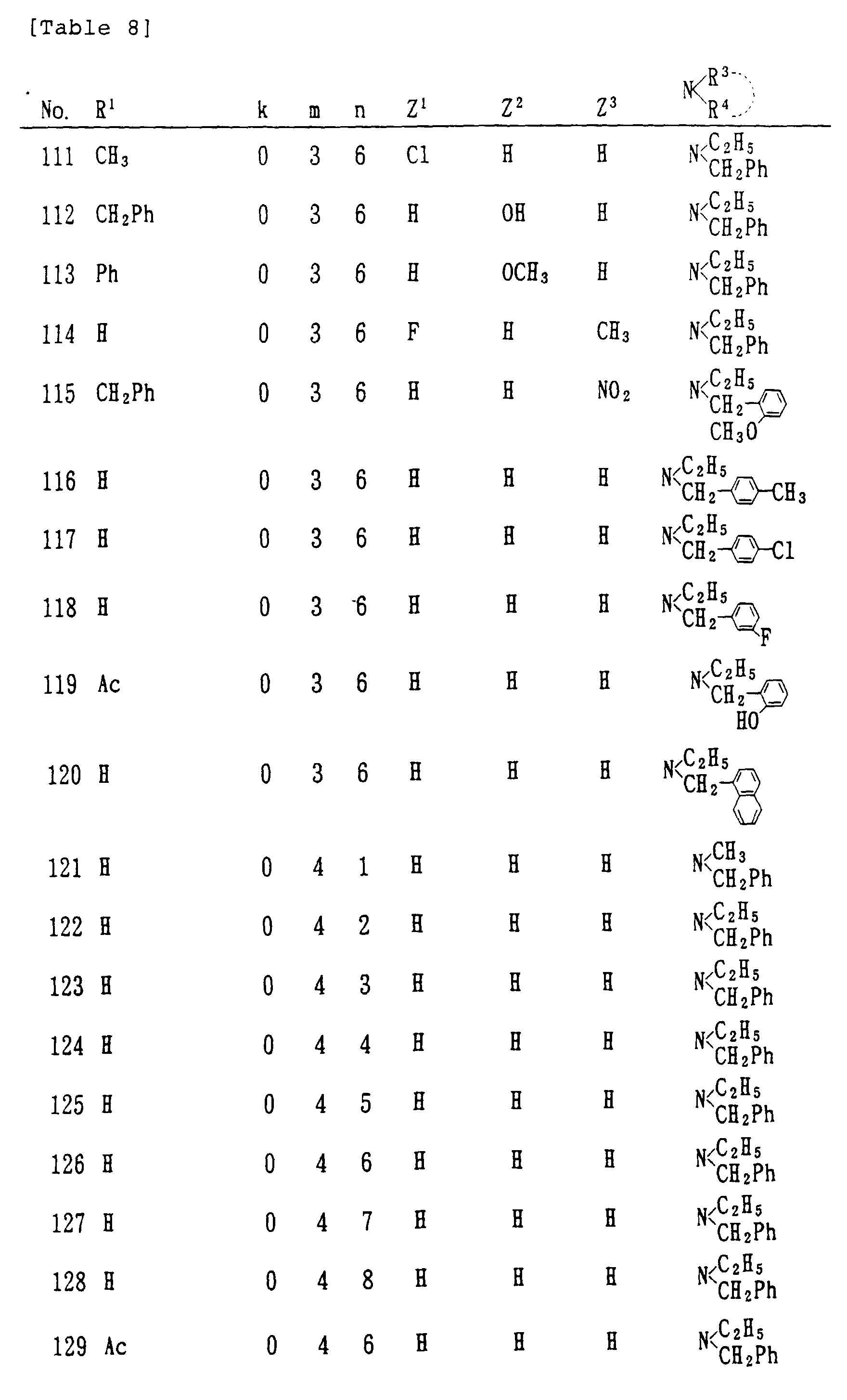

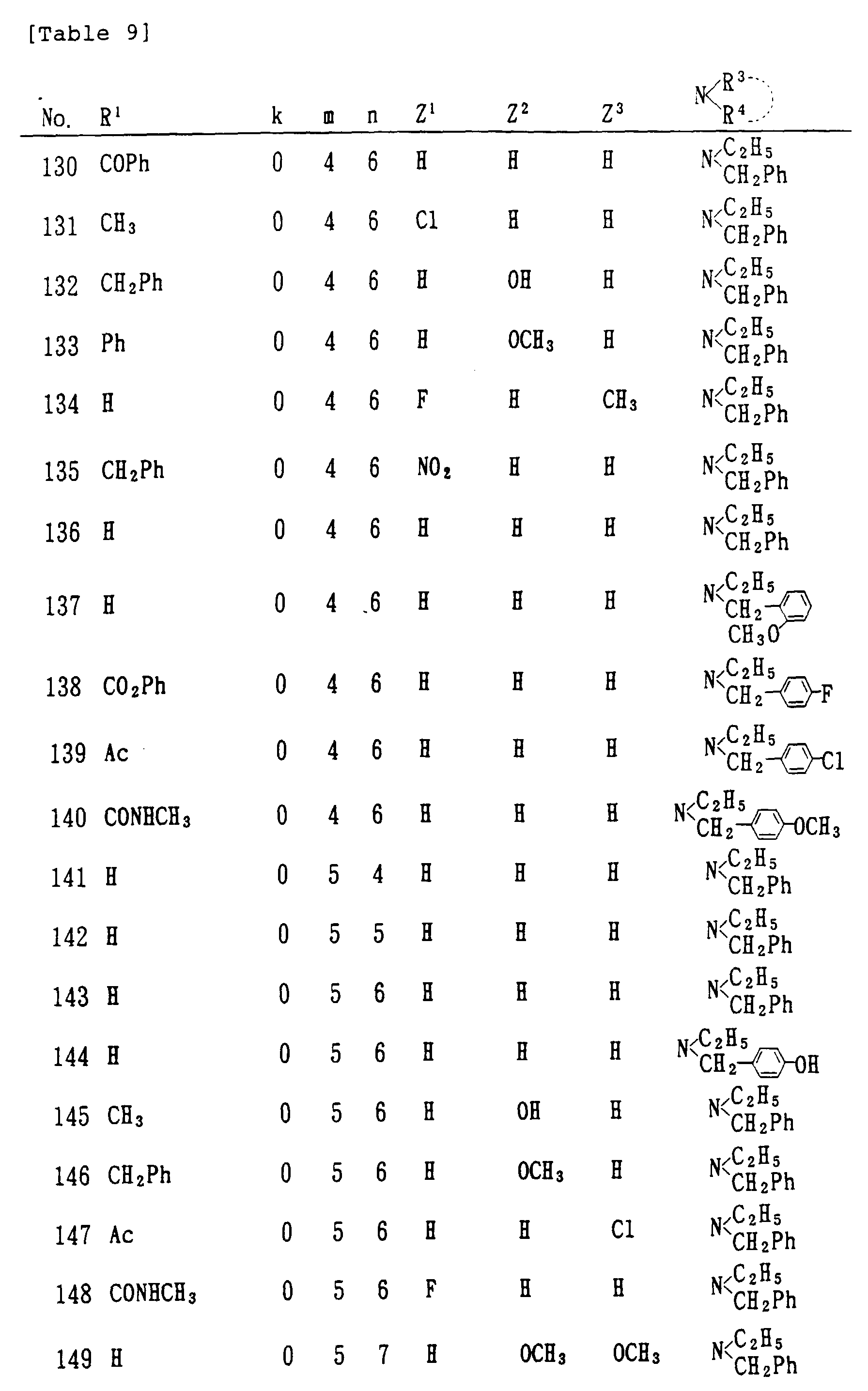

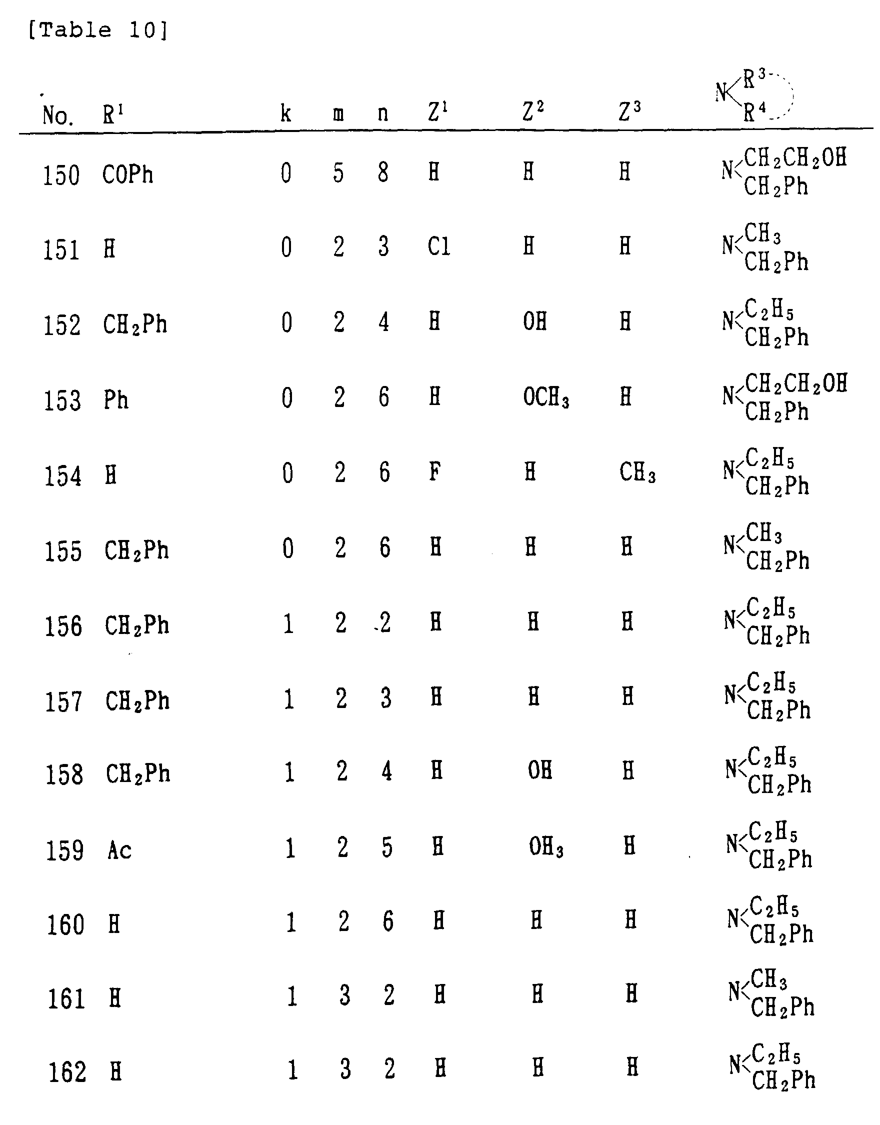

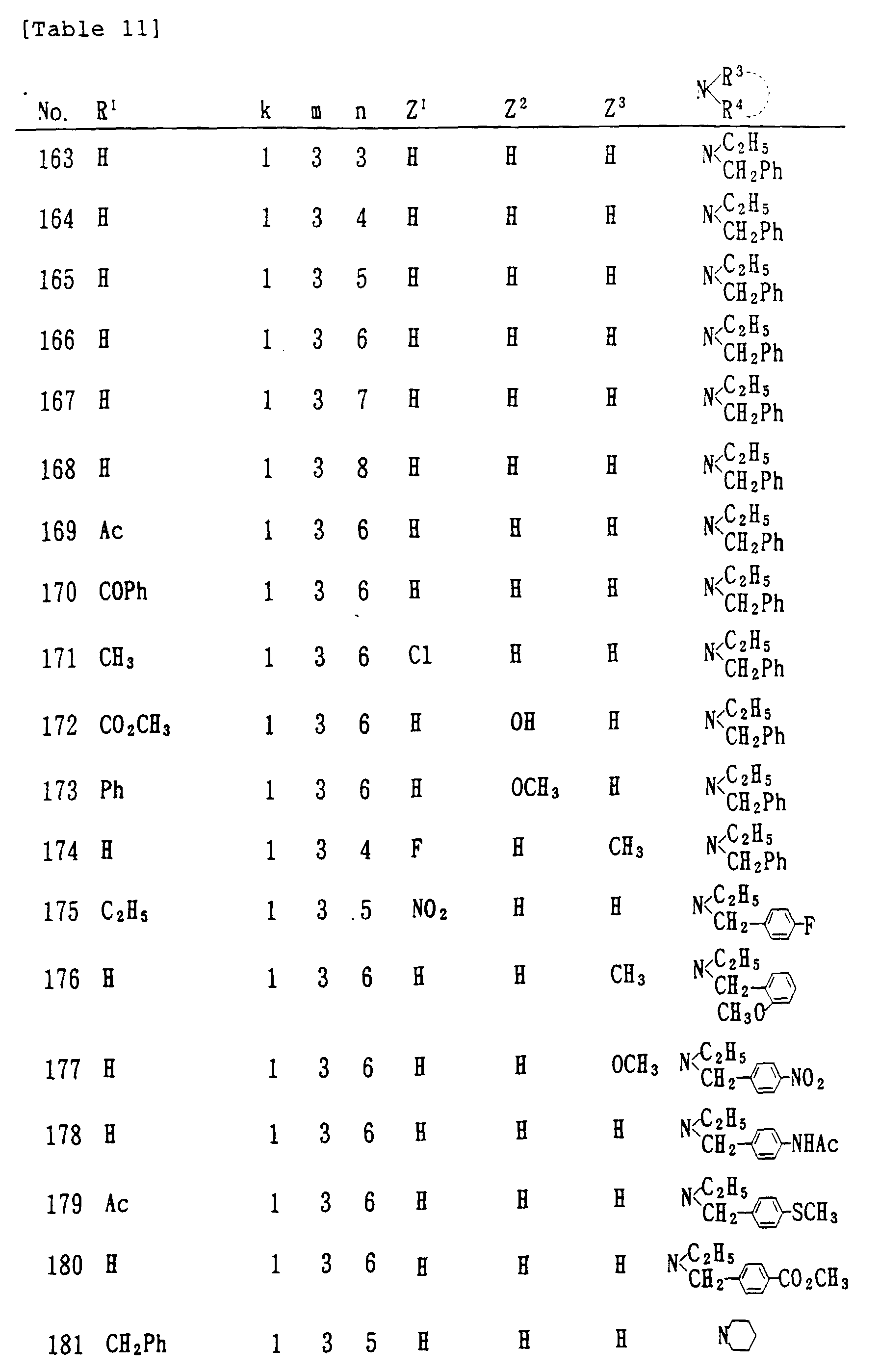

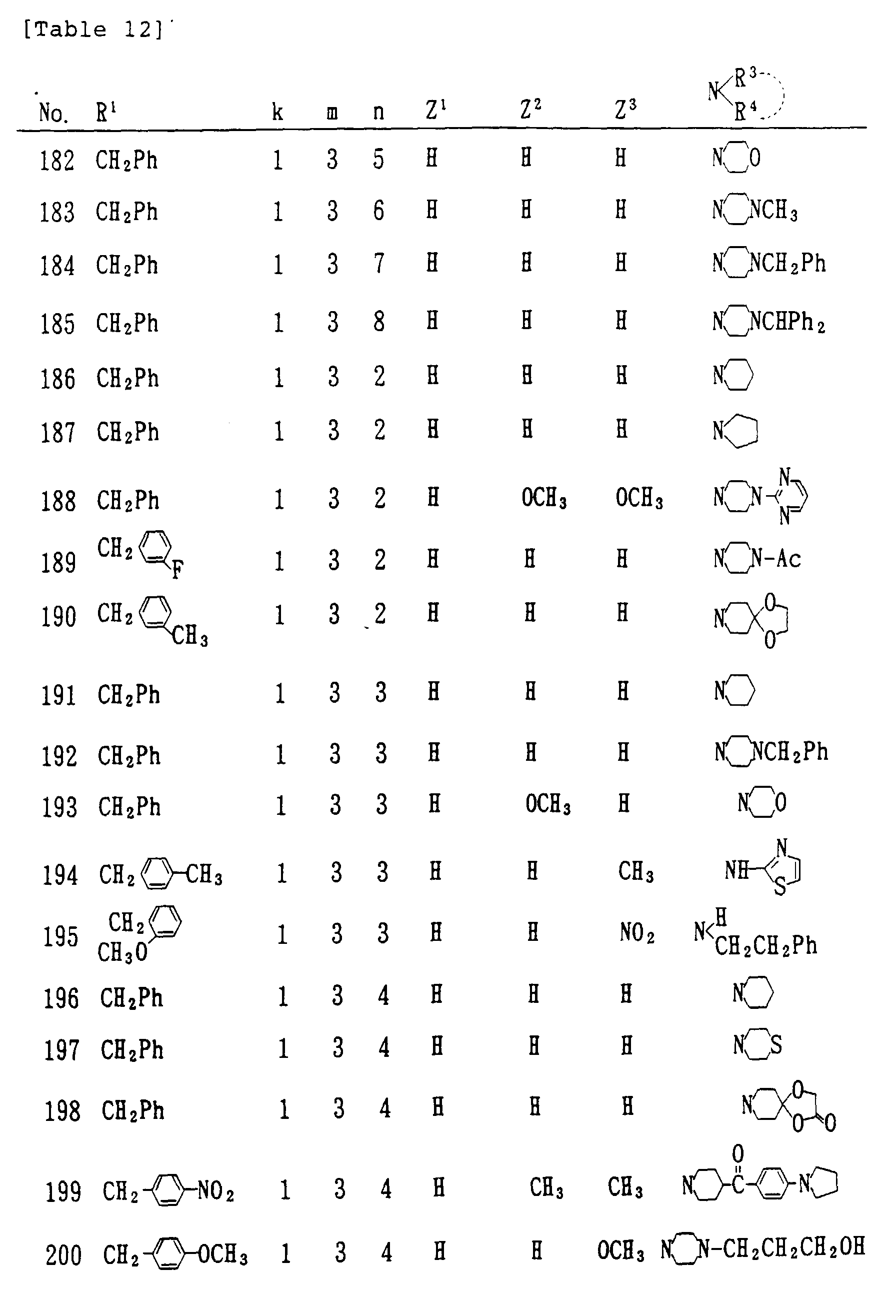

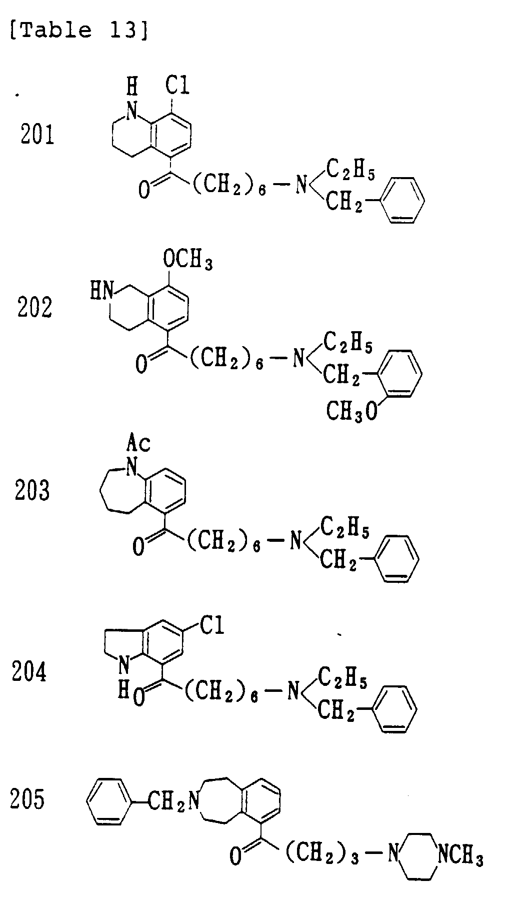

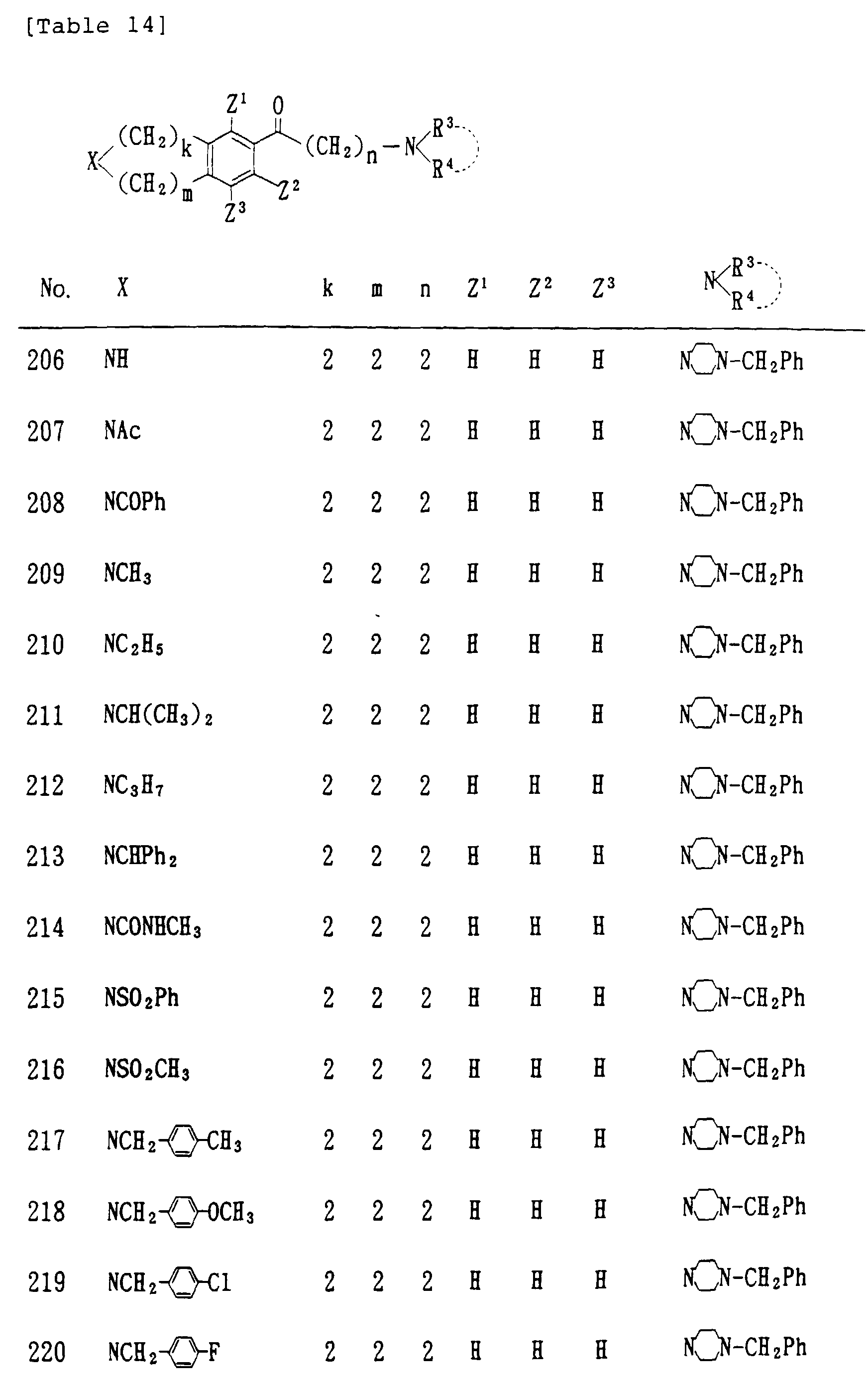

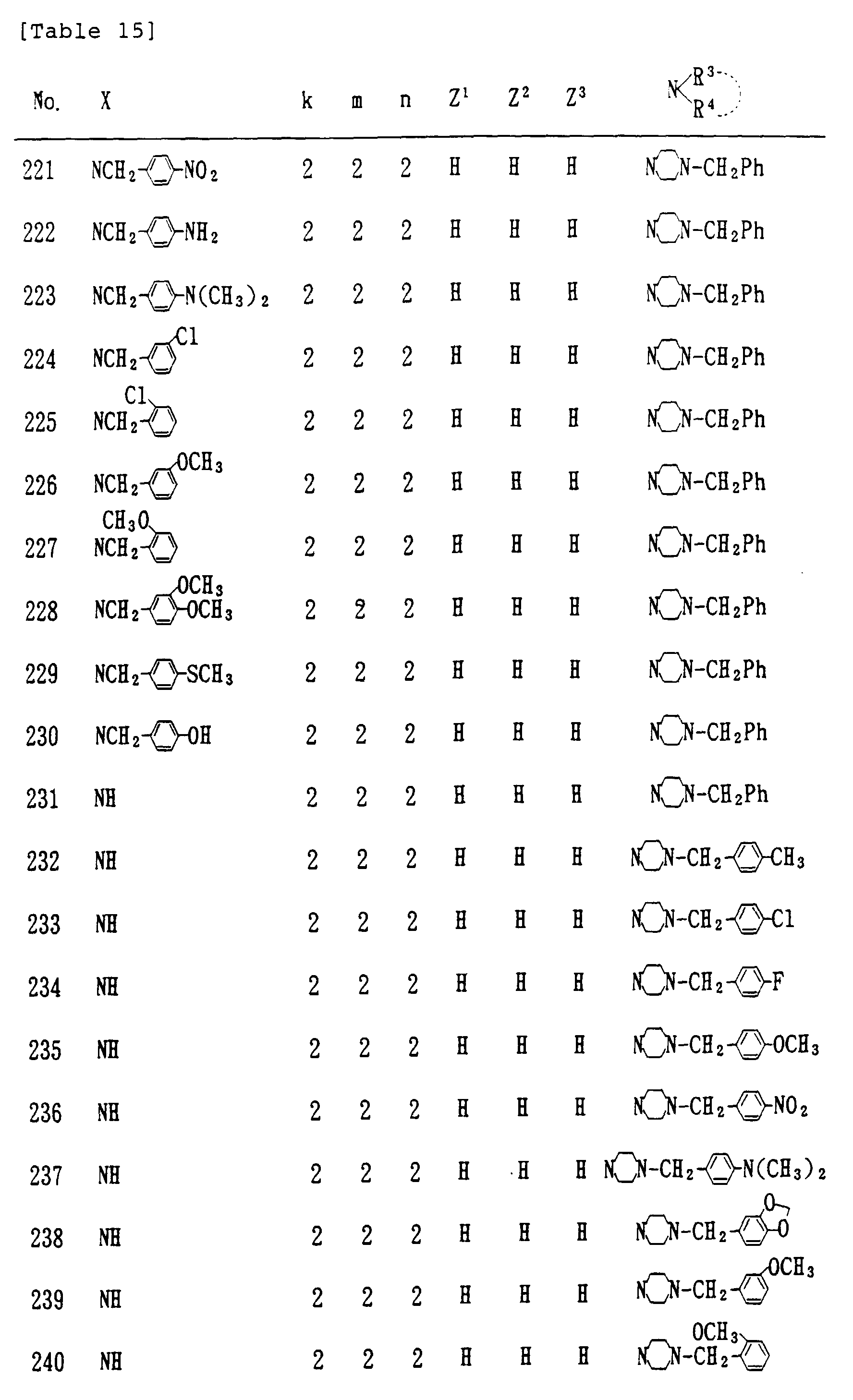

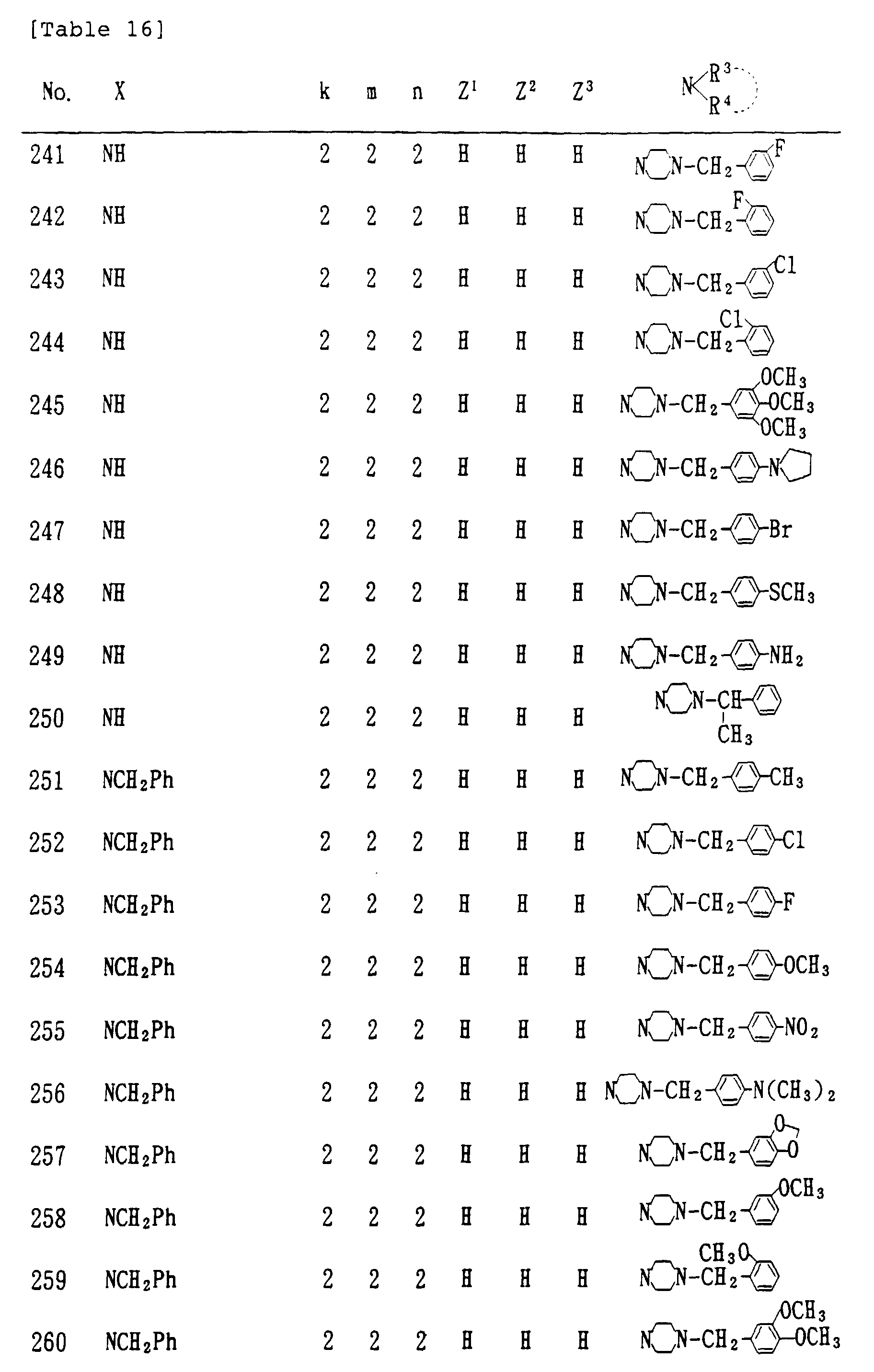

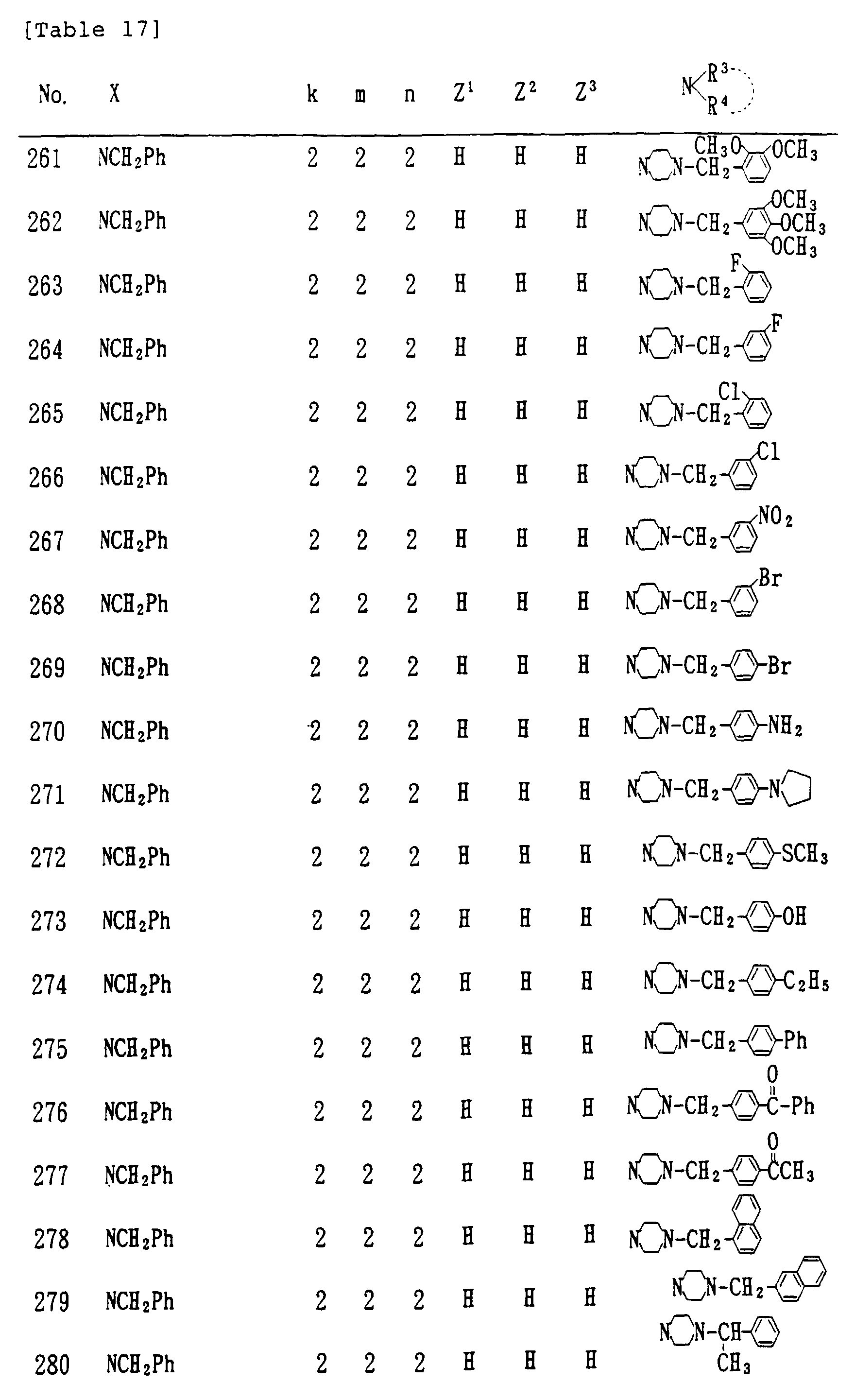

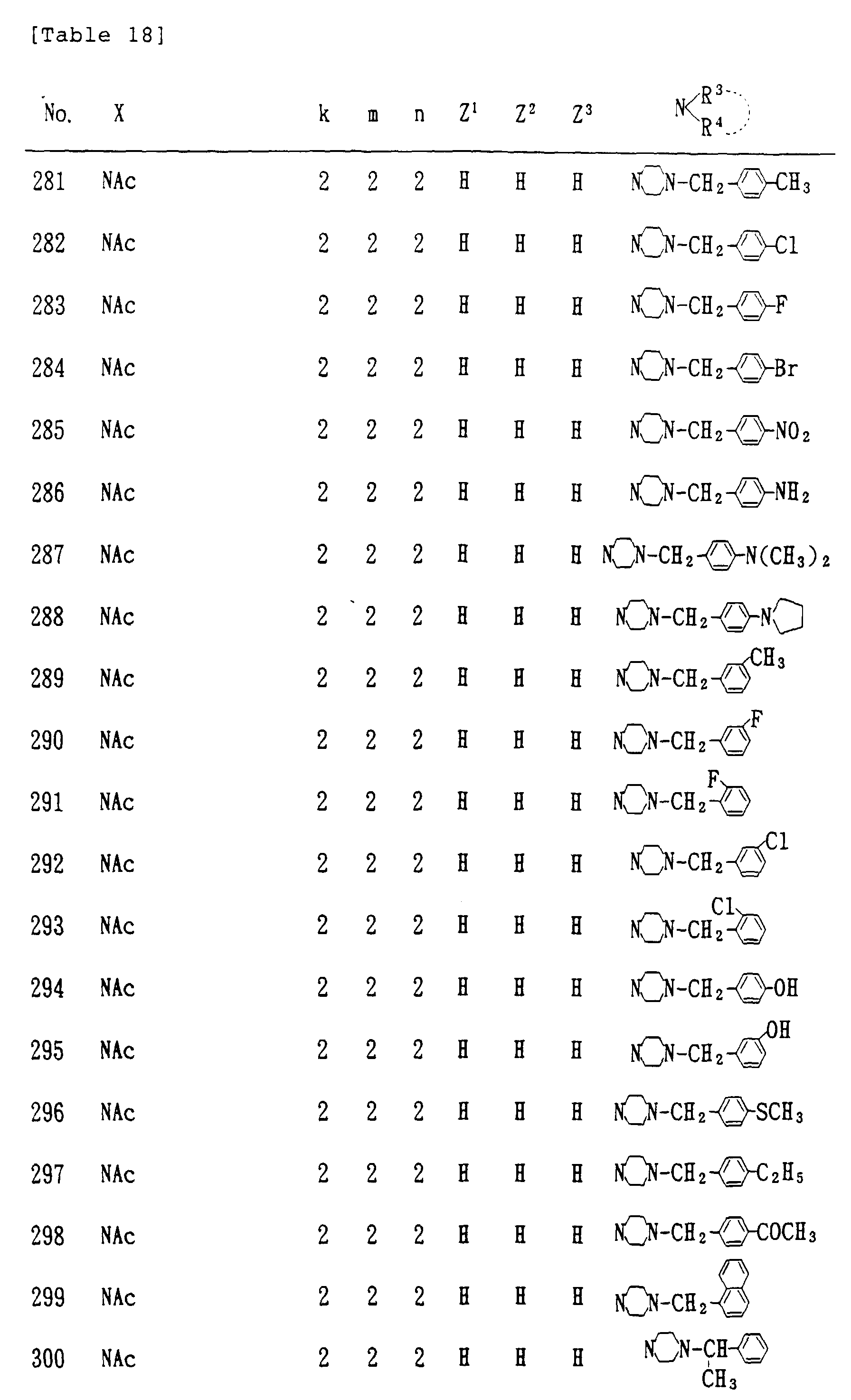

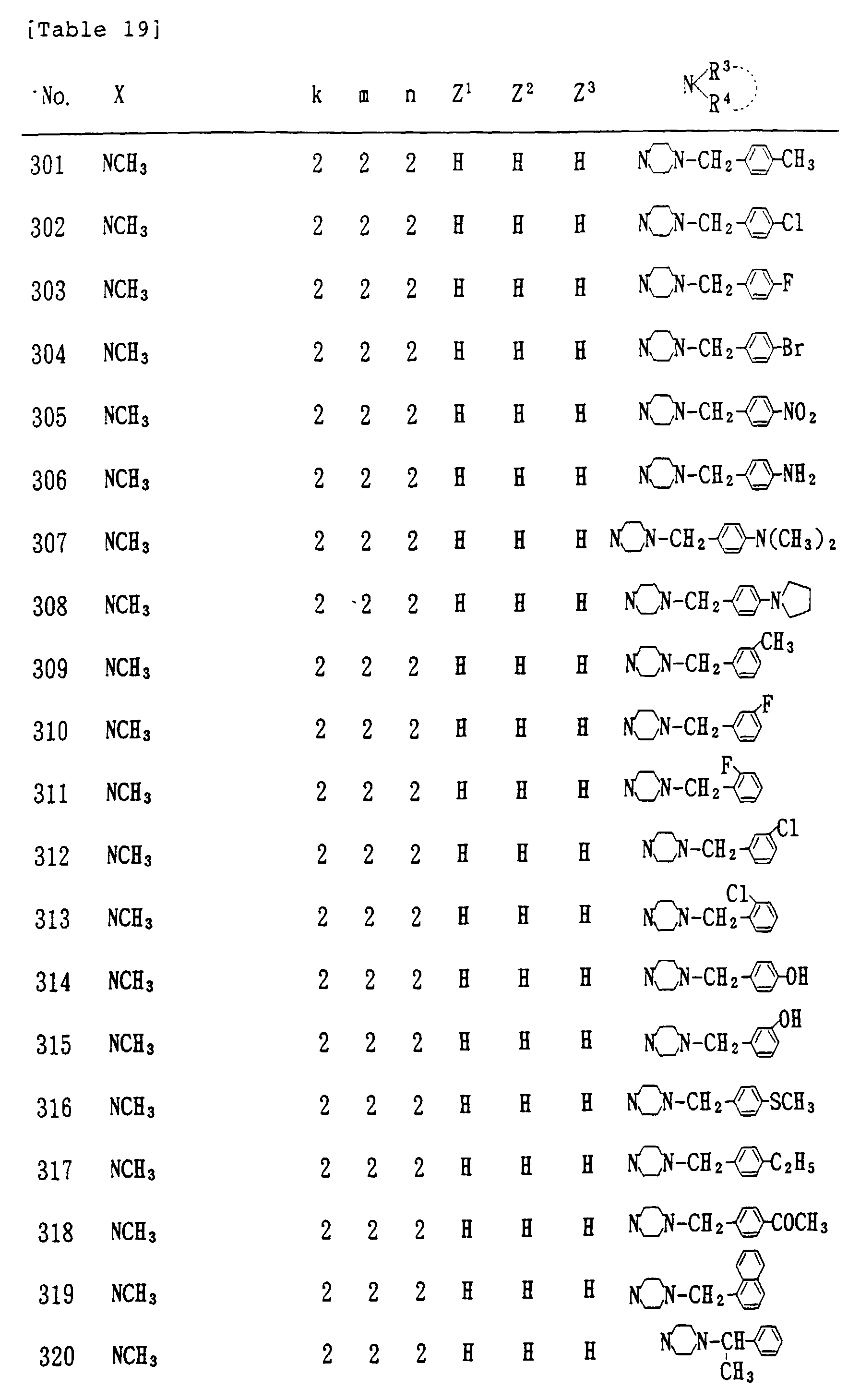

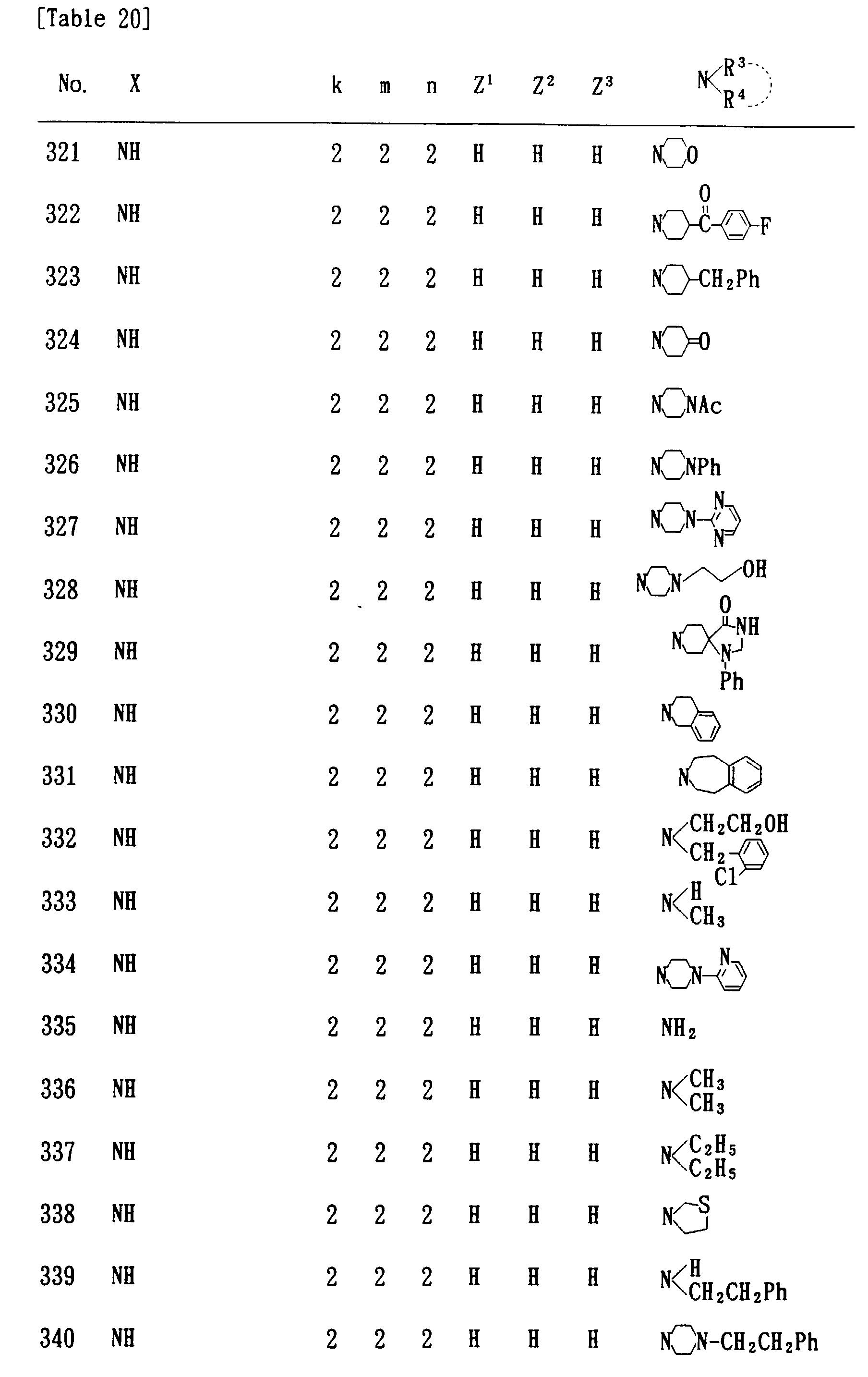

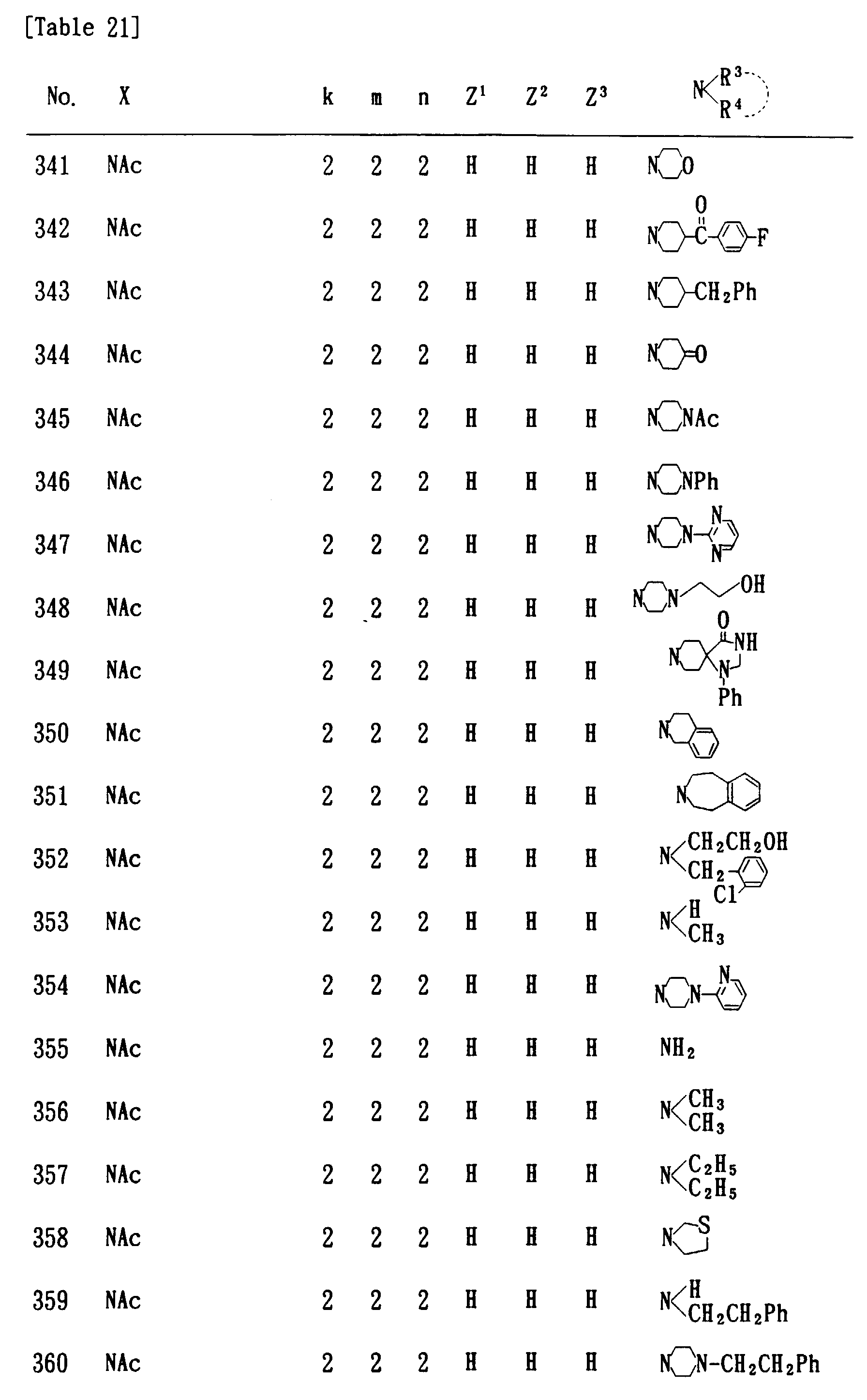

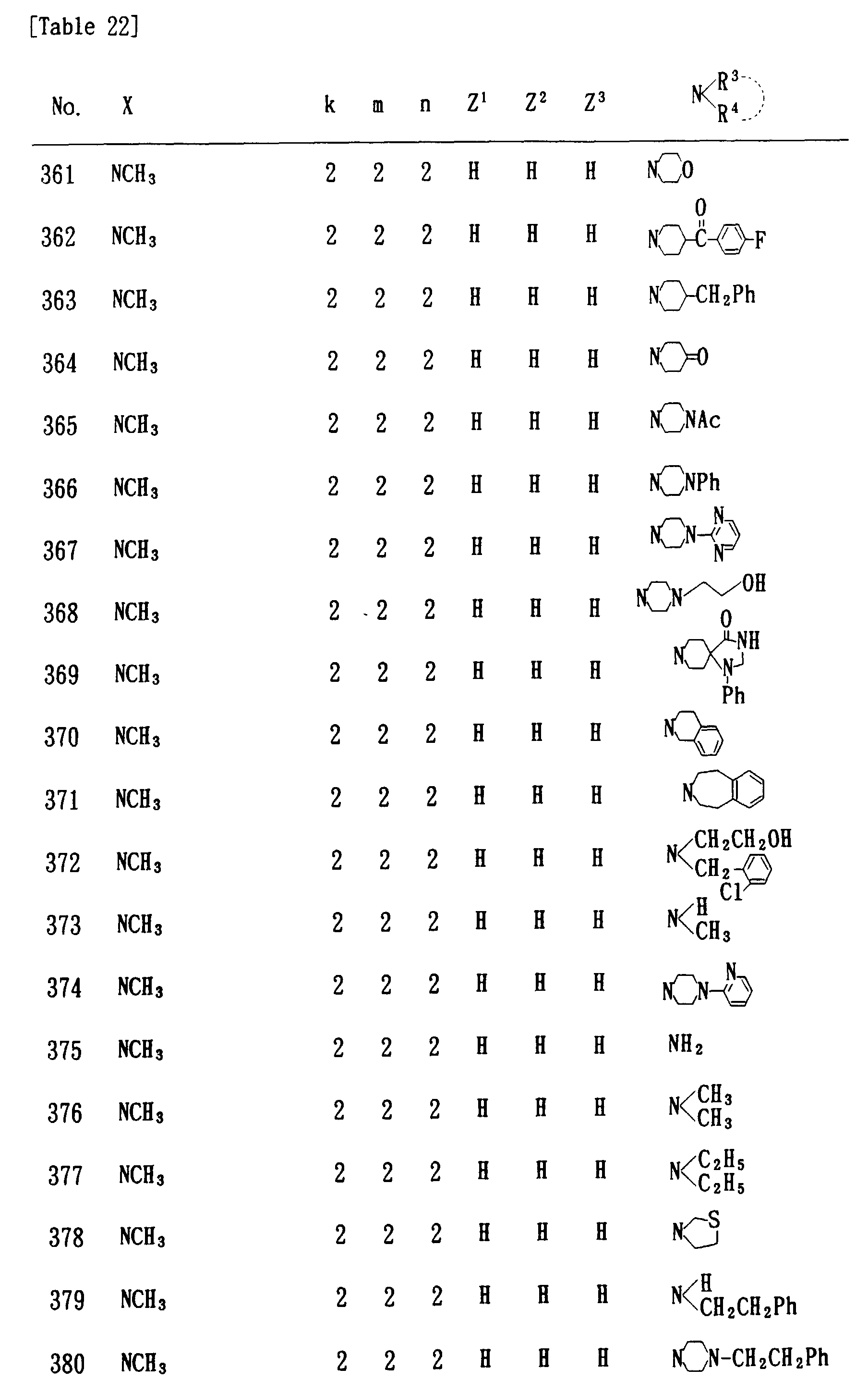

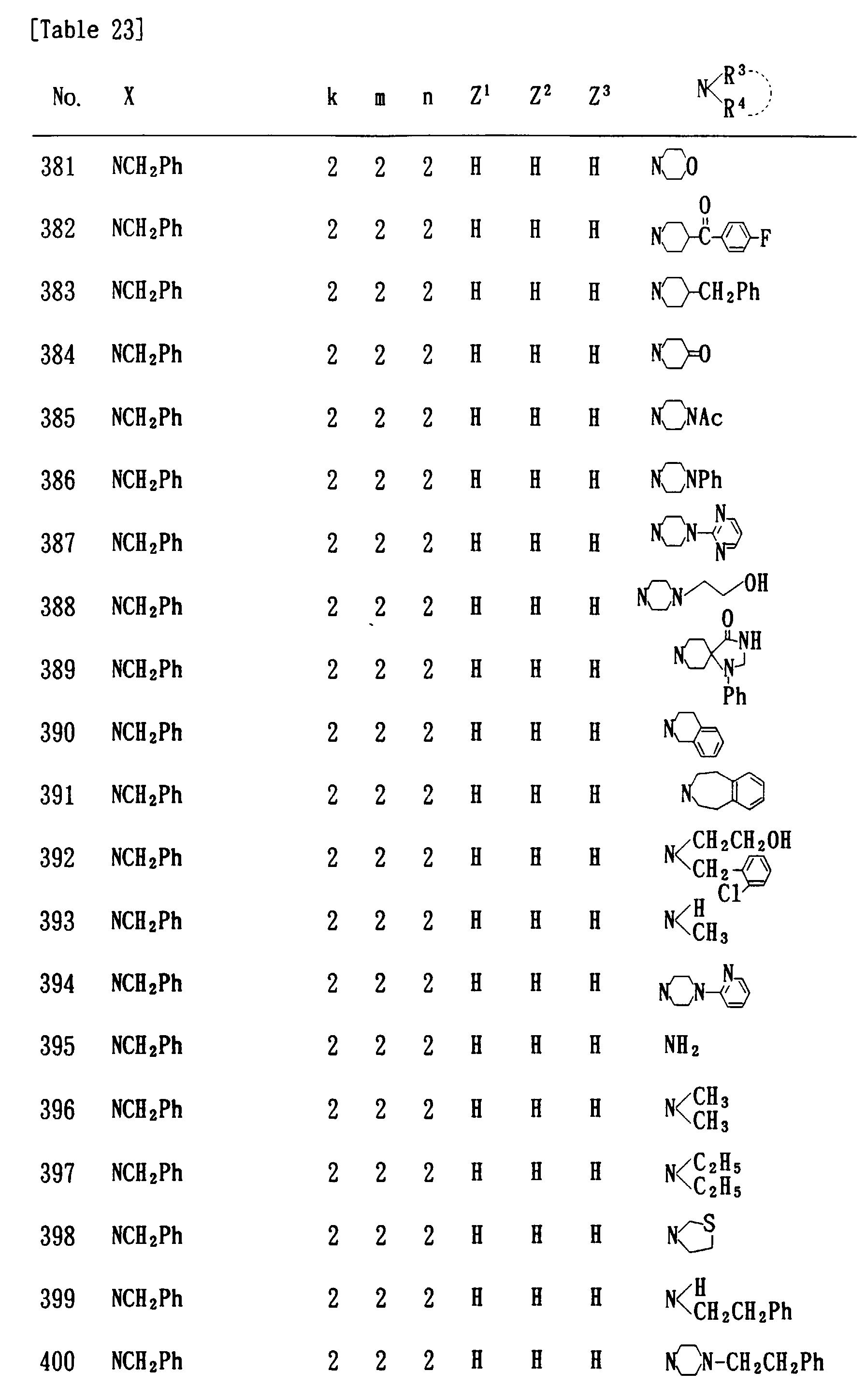

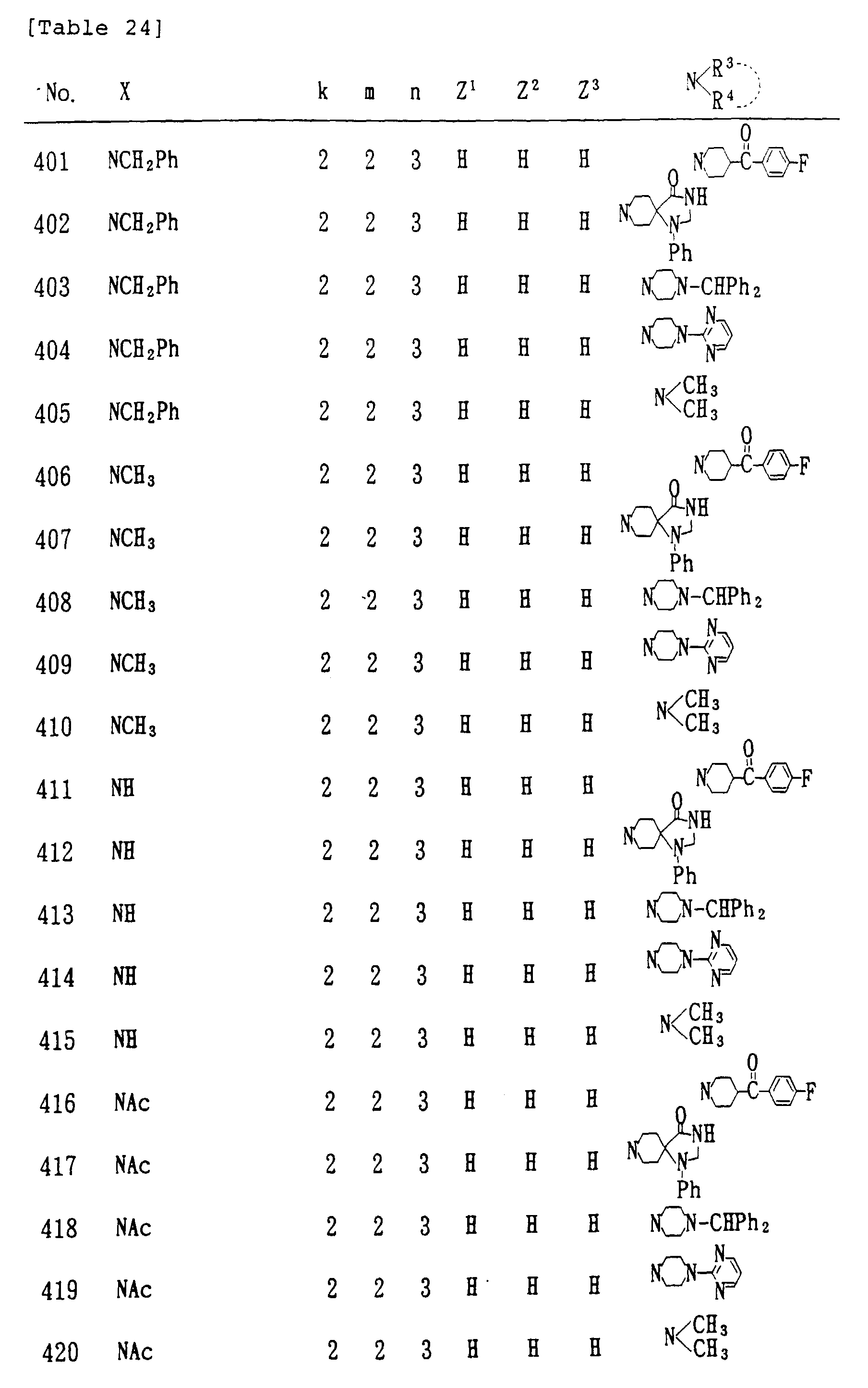

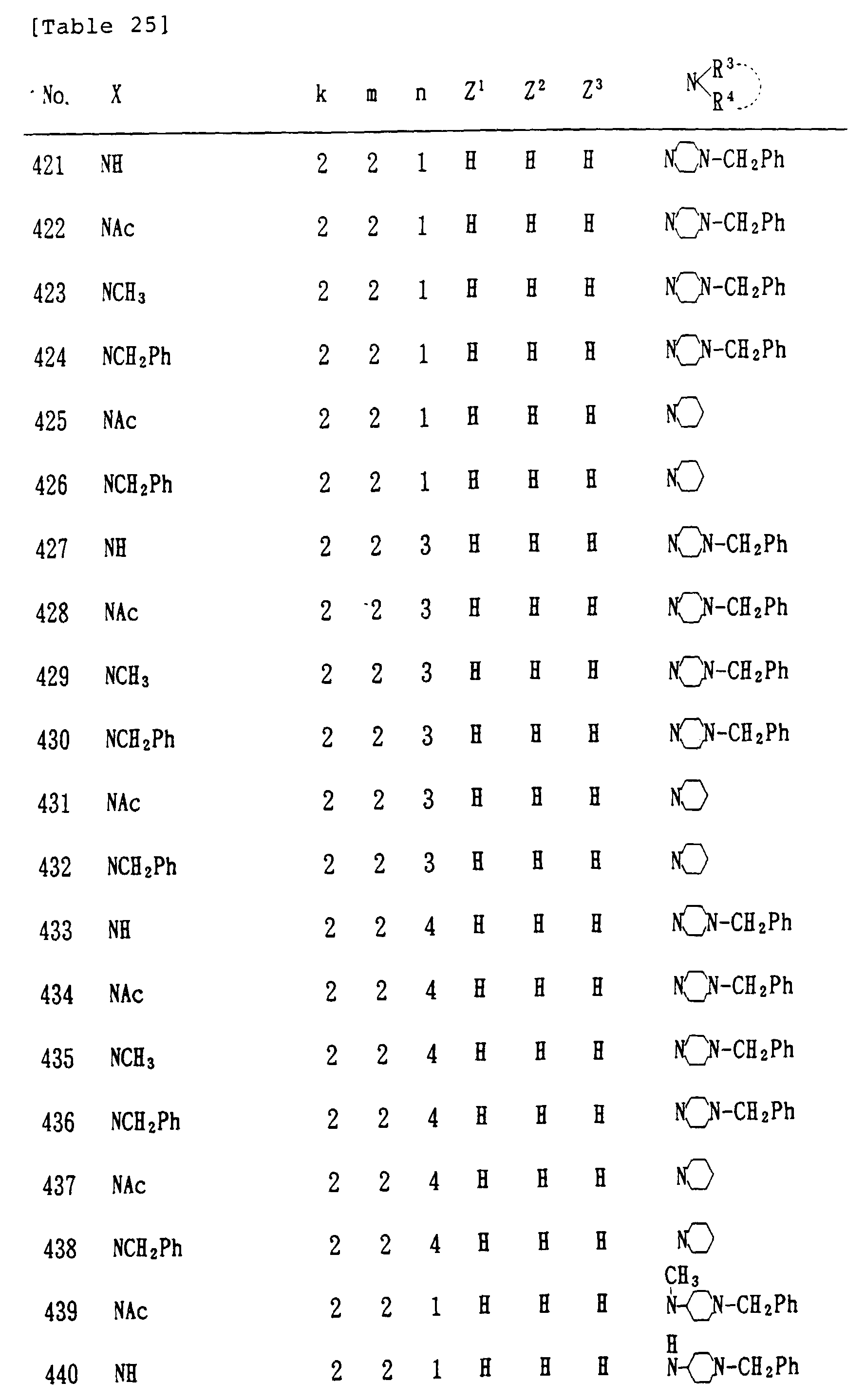

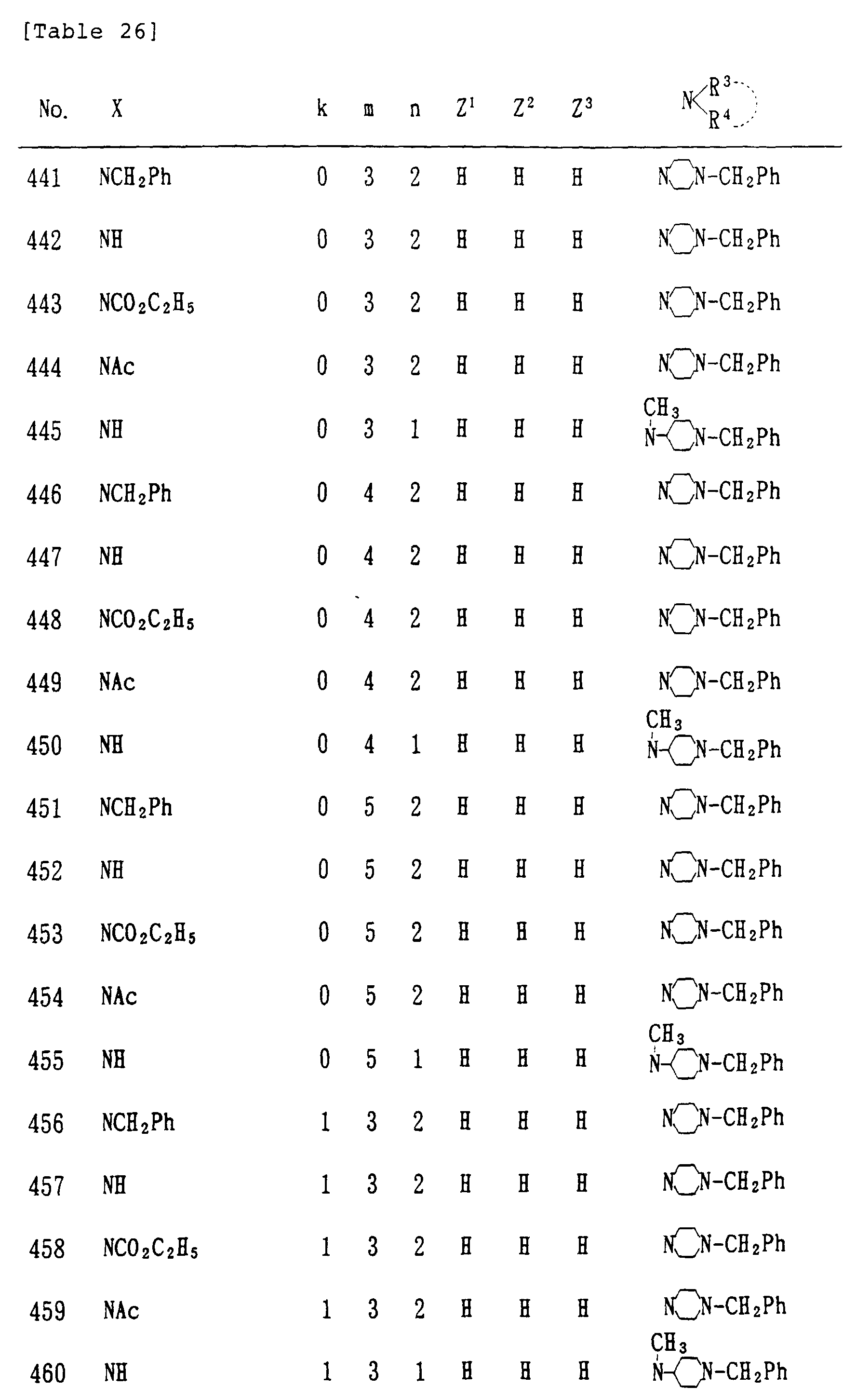

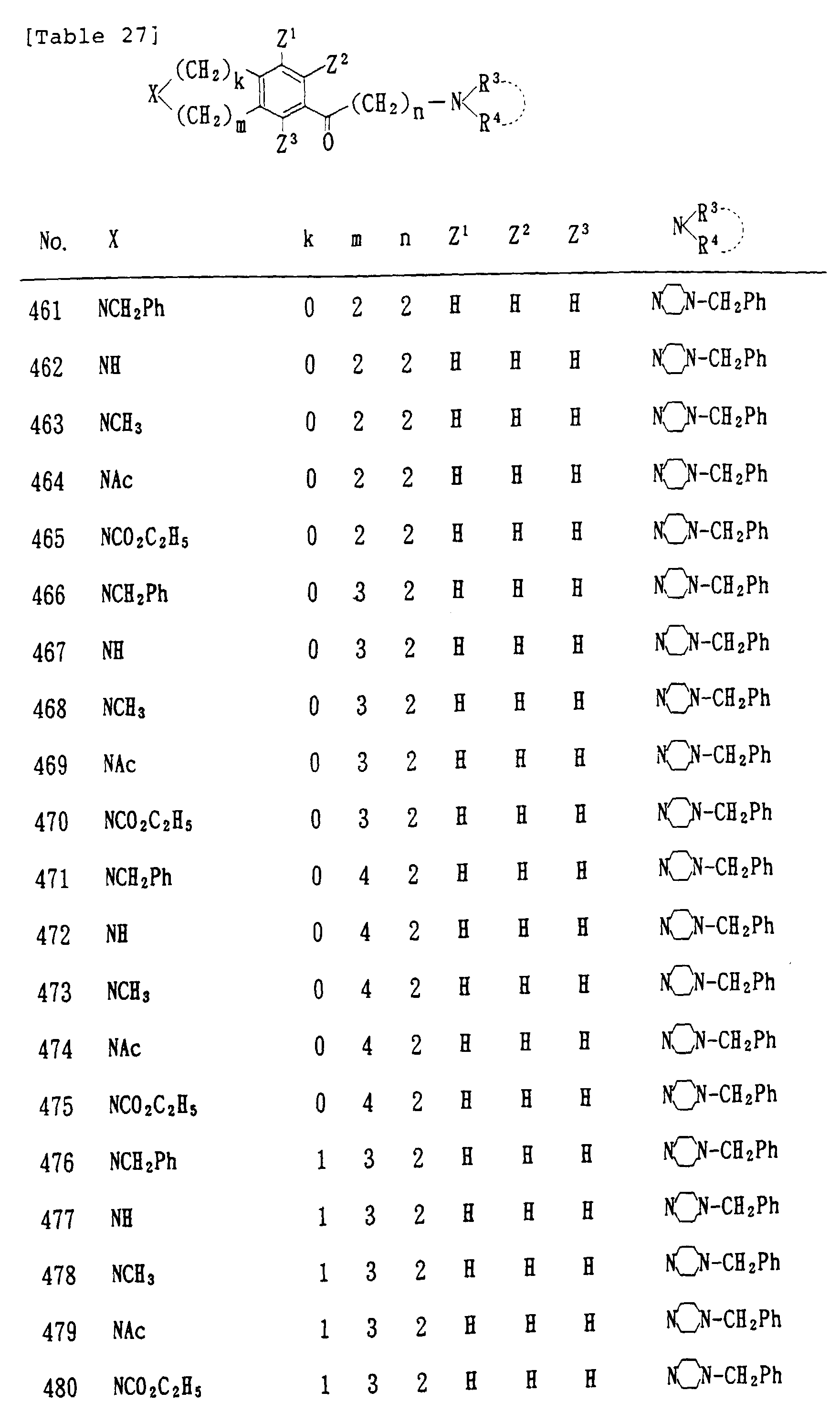

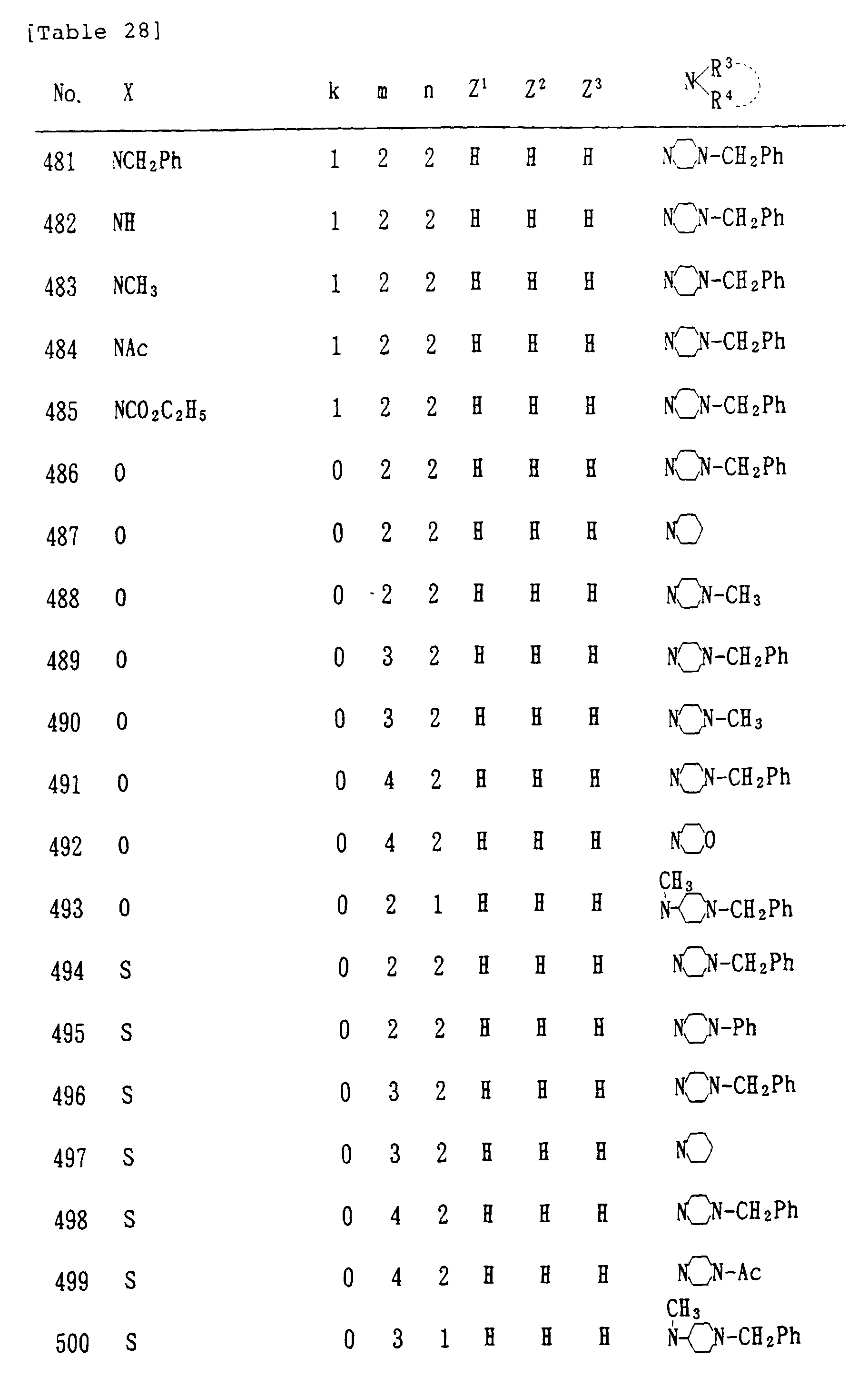

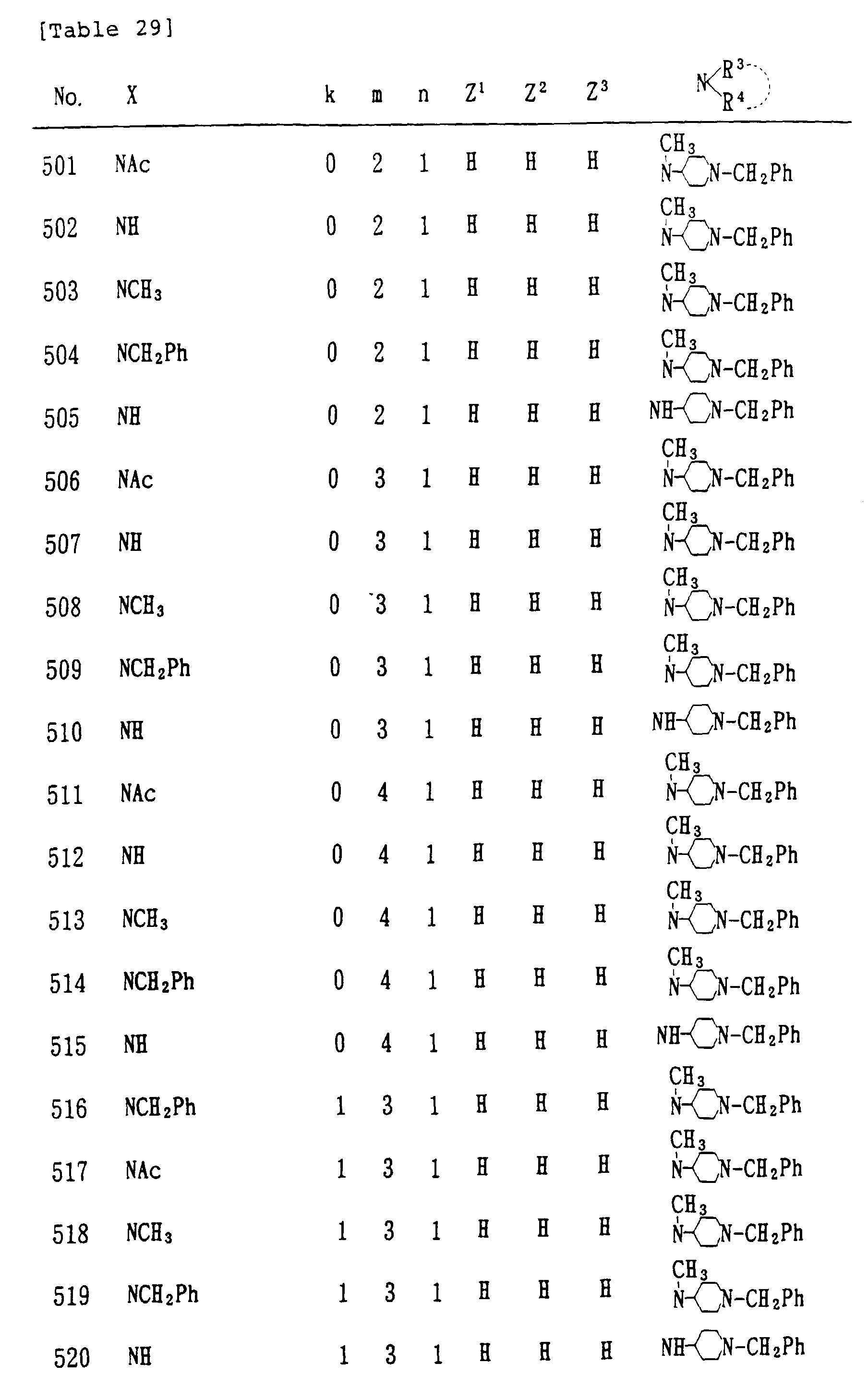

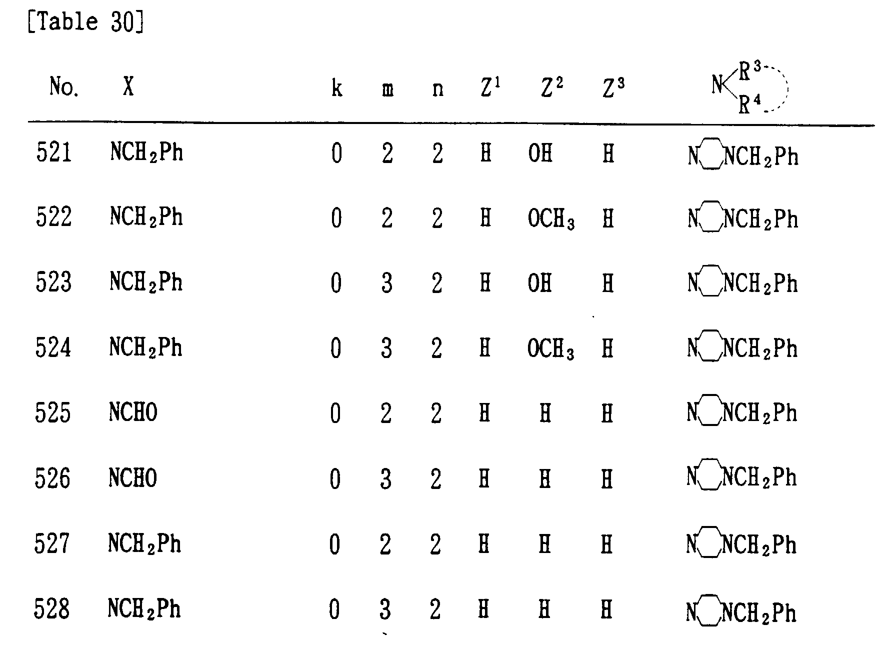

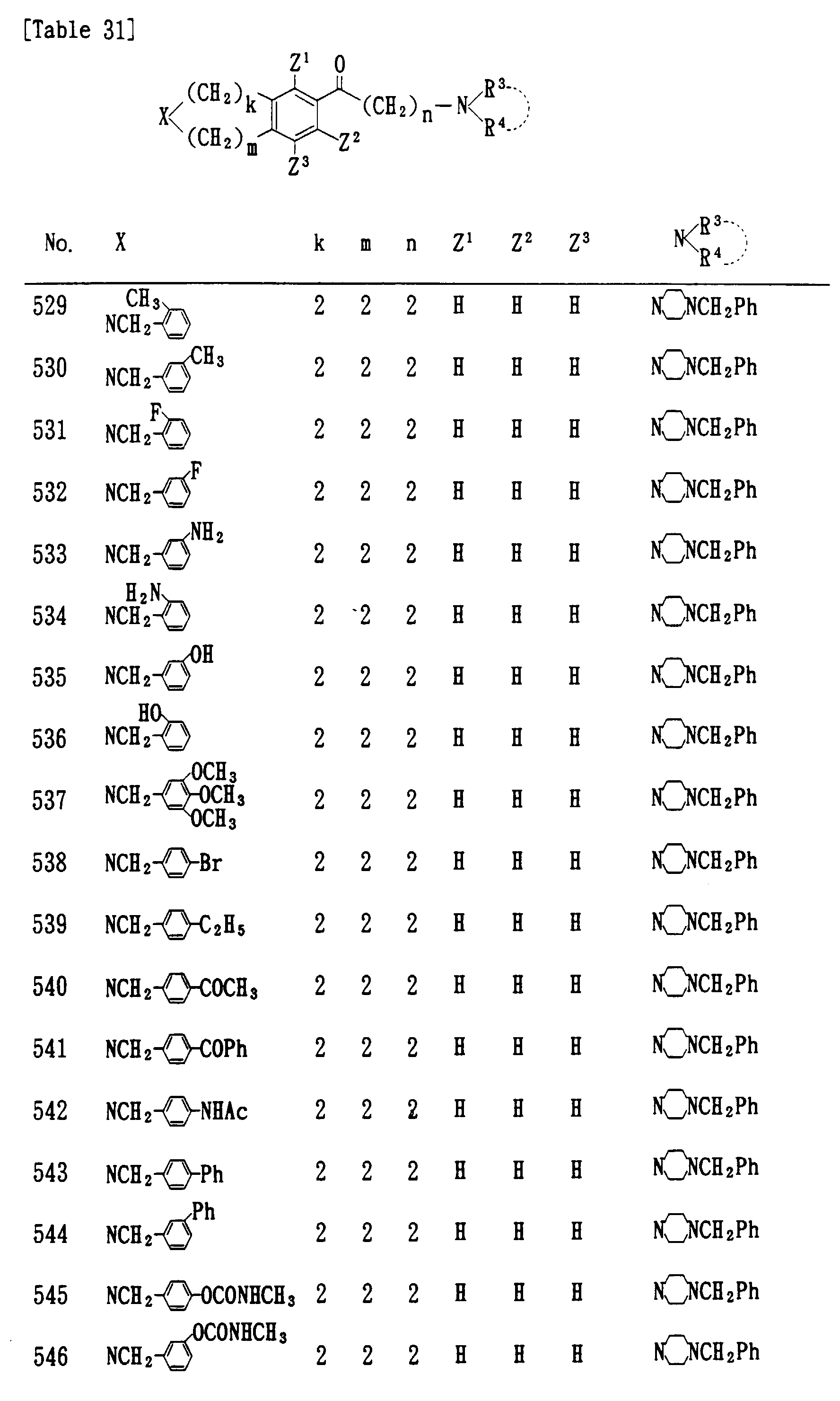

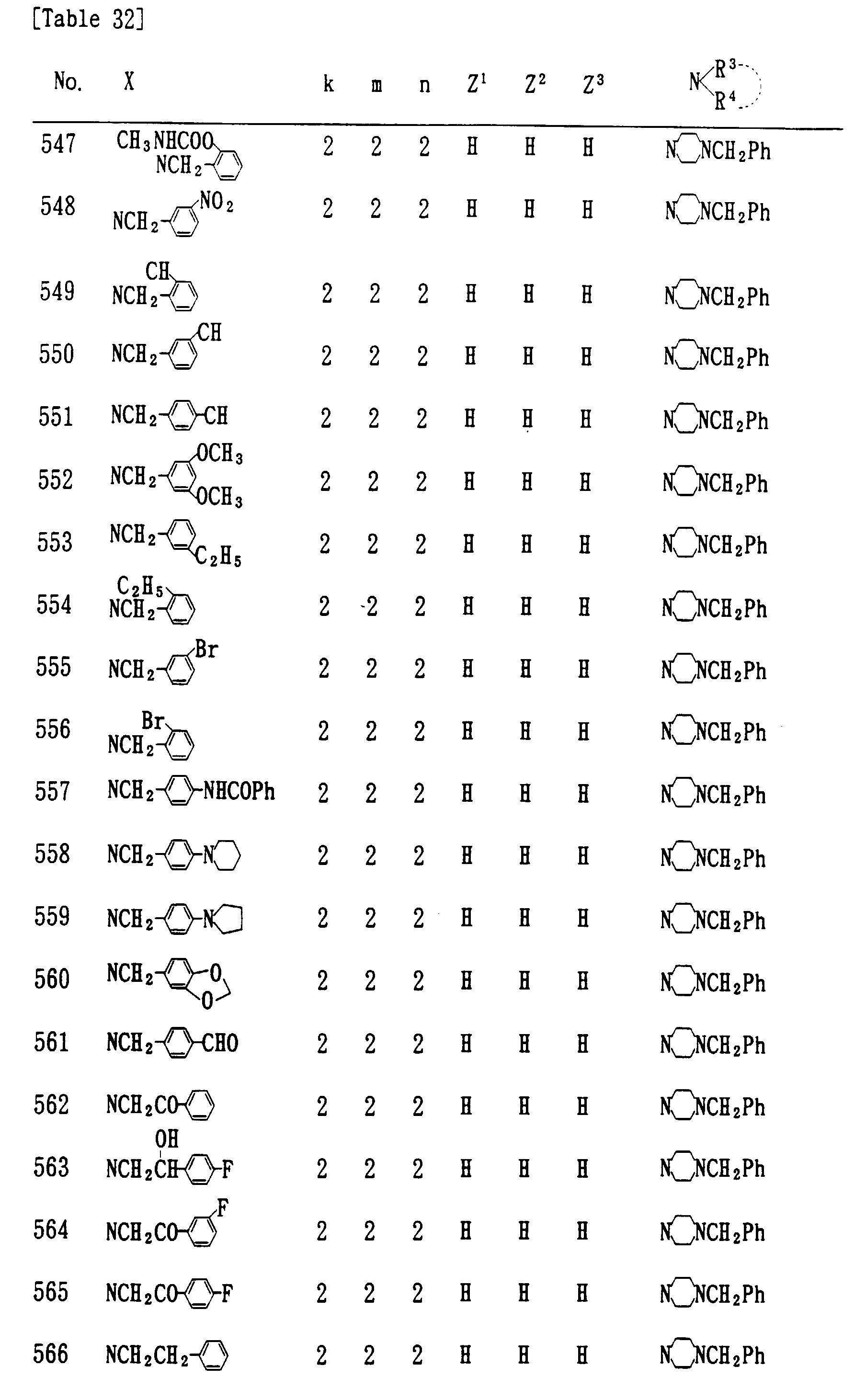

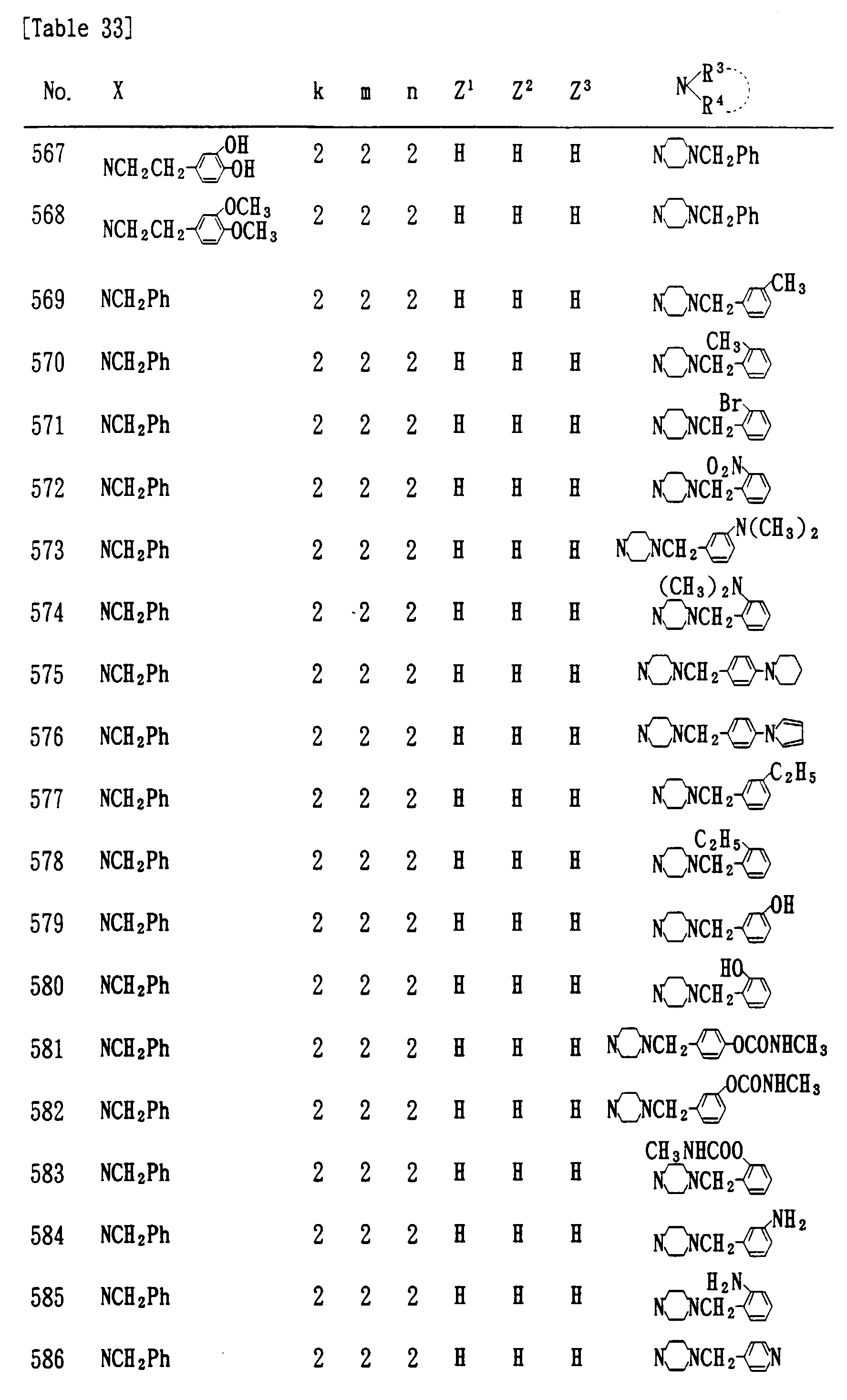

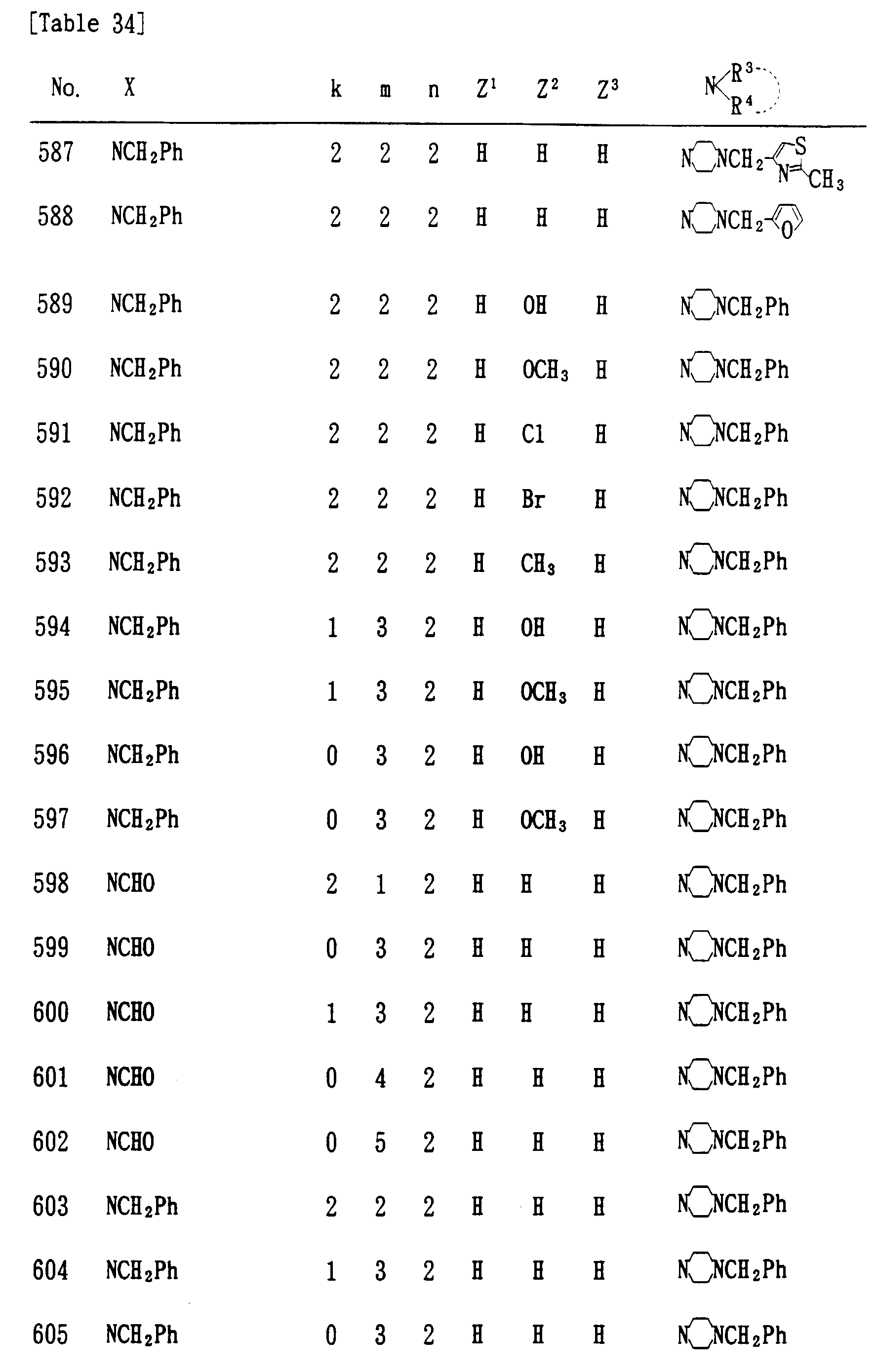

In the tables, Ac stands for acetyl group, and Ph stands for phenyl group.

As salts of the compounds [I] and [VII] of this invention, physiologically acceptable acid addition salts are especially preferable. Examples of these salts include salts with inorganic acids (e.g. hydrochloric acid, phosphoric acid, acid, hydrobromic acid, sulfuric acid), and salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid). And, in the case where the compounds [I] and [VII] of this invention have an acid group such as -COOH, they may form salts with inorganic bases (e.g. an alkali metal or alkaline earth metal such as sodium, potassium, calcium, magnesium etc., ammonia) or organic bases (e.g. tri-C₁₋₃ alkylamine such as triethylamine, etc.).

In the following, the process of producing the compound [I] or its salts of the present invention is described.

While the following description applies not only to the production of the compound [I] per se but also to the production of its salts, these compounds are simply referred to as the compound [I] inclusively in the description.

The compound [I] can be produced by, for example, reacting a compound represented by the formula: 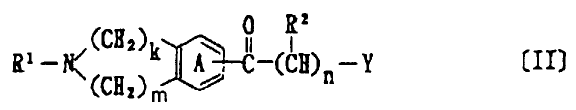 wherein each symbol has the same meaning as defined above, or a salt thereof, with a compound represented by the formula: 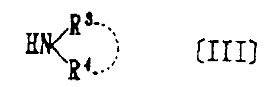 wherein each symbol has the same meaning as defined above or a salt thereof.

As the leaving group shown by Y in the formula [II], use is made of, for example, halogen atoms (e.g. chlorine, bromine, iodine, etc.), C₁₋₆ alkylsulfonyloxy (e.g. methanesulfonyloxy, ethanesulfonyloxy, etc.), C₆₋₁₀ arylsulfonyloxy (e.g. benzenesulfonyloxy, p-toluenesulfonyloxy, etc.), etc. Among them, halogen atoms are preferable, more specifically, chlorine, bromine, etc.

As salts of the compound [II] and [III], use is made of, for example, salts with inorganic acids (e.g. hydrochloric acid, phosphoric acid, hydrobromic acid, sulfuric acid) or those with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid). And, in the case where the compounds [II] and [III] of this invention have an acid group such as -COOH, they may form salts with inorganic bases (e.g. an alkali metal or alkaline earth metal such as sodium, potassium, calcium, magnesium, etc., ammonia) or organic bases (e.g. tri-C₁₋₃ alkylamine such as triethylamine, etc.).

The amount of the compound [III] or a salt thereof to be employed in this reaction ranges, usually, from 1.0 to 50.0 mol., preferably 1.0 to 10.0 mol., mol., relative to one mol. of the compound [II] or a salt thereof. This reaction can be conducted at temperatures ranging from 0°C to 120°C. The reaction time ranges, usually, from 10 minutes to 48 hours, preferably from 2 to 16 hours.

While this reaction can be conducted in the absence of solvent, it can be conducted, upon necessity, in a solvent. As the solvent, any one can be employed unless it hampers the reaction, exemplified by lower alcohols such as methanol, ethanol, propanol, isopropanol, n-butanol, t-butanol, etc., ethers such as dioxane, ether, tetrahydrofuran, etc., aromatic hydrocarbons such as toluene, benzene, xylene, etc., amides such as dimethylformamide, dimethylacetamide, hexamethylphosphonotriamide, etc., esters such as ethyl acetate, butyl acetate, etc. The amount the solvent to be employed ranges, usually, from 0.5 to 100 ml, preferably from 5 to 20 ml, relative to 1 mmol.of the compound [II] or salt thereof.

This reaction can also be conducted in the presence of a base, when necessary. Examples of the base to be employed include inorganic bases such as sodium carbonate, potassium carbonate, lithium carbonate, sodium hydroxide, potassium hydroxide, sodium methoxide, sodium ethoxide, sodium hydride, etc. and organic bases such as pyridine, 4-dimethylaminopyridine, triethylamine, etc. The amount of the base to be employed ranges, usually, from equimol. to excess amount, preferably from 1.0 to 5.0 mol. relative to one mol. of the compound [III] or a salt thereof.

It is also possible to promote this reaction by allowing an iodide (e.g. sodium iodide, potassium iodide, lithium iodide) to be present in the reaction system. The amount of the iodide to be employed ranges, usually from 1 to 5 mol., preferably 1.0 to 1.5 mol. relative to one mol. of the compound [II] or a salt thereof.

The compound [I], wherein R¹ stands for an optionally substituted hydrocarbon group or an optionally substituted acyl group, or a salt thereof can be produced by, for example, reacting a compound [I], wherein R¹ stands for H (R=H), or a salt thereof, with a compound represented by the formula

R¹'-Y² [VIII]

wherein R¹' stands for an optionally substituted hydrocarbon group or an optionally substituted acyl group, and Y² stands for a leaving group, or a salt thereof.

Examples of the salts of the compound [VIII] include those with inorganic acids (e.g. hydrochloric acid, phosphoric acid, hydrobromic acid, sulfuric acid) or organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid). Further, in the case where the compound [VIII] of this invention has an acid group such as -COOH, the compound [VIII] may be in the form of salt with an inorganic base (e.g. an alkali metal or alkaline earth metal such as sodium, potassium, calcium, magnesium, etc., ammonia) or an organic base (e.g. tri-C₁₋₃ alkylamine such as triethylamine, etc.).

As the leaving group shown by Y², use is made of, for example, a halogen atom (e.g. chlorine, bromine, iodine), C₁₋₆ alkylsulfonyloxy (e.g. methanesulfonyloxy, ethanesulfonyloxy), C₆₋₁₀ arylsulfonyloxy (e.g. benzenesulfonyloxy, p-toluenesulfonyloxy), etc. Especially, halogen atoms are preferable.

As the optionally substituted hydrocarbon groups shown by R¹', use is made of, for example, the optionally substituted hydrocarbon groups shown by R¹ as described in the foregoing. As the optionally substituted acyl groups shown by R¹', use is made of, for example, the optionally substituted acyl groups shown by R¹ as described in the foregoing.

Further, the compound [I] (R=H) or a salt thereof can be produced also by subjecting a compound [I], wherein R¹ stands for an optionally substituted acyl group, or a salt thereof to hydrolysis with an acid or a base, in the same manner as in conventional hydrolysis.

The starting compound [II] or a salt thereof can be produced by reacting a compound represented by the formula: 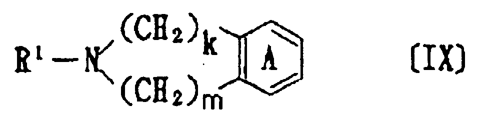 wherein each symbol has the same meaning as defined above, or a salt thereof, with a compound represented by the formula: 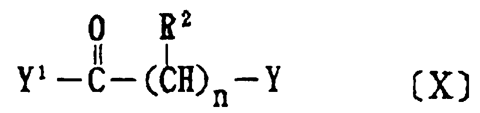 wherein Y¹ stands for a leaving group, and other symbols are of the same meanings as defined above.

As salts of the compound [IX], use is made of, for example, salts with an inorganic acid (e.g. hydrochloric acid, phosphoric acid, hydrobromic acid, sulfuric acid) or an organic acid (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid). Further, in the case where the compound [IX] of this invention has an acid group such as -COOH, the compound [IX] may be in the form of a salt with an inorganic base (e.g. an alkali metal or an alkaline earth metal such as sodium, potassium, calcium, magnesium, etc., ammonia) or an organic base (e.g. tri-C₁₋₃ alkylamine such as triethylamine, etc.).

As the leaving group shown by Y¹, use is made of, for example, halogen atoms (e.g. chlorine, bromine, iodine), C₁₋₆ alkylsulfonyloxy (e.g. methanesulfonyloxy, ethanesulfonyloxy), C₆₋₁₀ arylsulfonyloxy (e.g. benzenesulfonyloxy, p-toluenesulfonyloxy), etc. Especially, halogen atoms are preferable.

The compound [IX] or salts thereof can be produced in accordance with a per se known method or a method analogous thereto, for example, descriptions on J. Org. Chem. 34, 2235 (1969), J. Org Chem. 54, 5574 (1989), Tetrahedron Lett. 35, 3023 (1977), and Bull. Chem. Soc. Jpn. 56, 2300 (1983).

The compound [X] can be produced by a per se known method or a method analogous thereto.

The reaction of the compound [IX] or a salt thereof with the compound [X] can be conducted by using, for example, usually about 1 to 20 mol., preferably about 1 to 5 mol., of of the compound [X] or a salt thereof relative to 1 mol. of the compound [IX], in the presence of, for example, a Lewis acid.

This reaction can be conducted without using a solvent or, upon necessity, in a solvent. As the solvent, any one which is conventionally usable in chemical reactions, unless it hampers the reaction, can be employed, as exemplified by organic solvents including hydrocarbons (e.g. pentane, hexane, benzene, toluene, nitrobenzene, etc.), halogenated hydrocarbons (e.g. dichloromethane, chloroform, 1,2-dichloroethane, carbon tetrachloride, etc.), ethers (e.g. ethyl ether, tetrahydrofuran, dioxane, dimethoxyethane, etc.), nitro alkanes (e.g. nitromethane, propionitrile, etc.), and carbon disulfide. Especially, dichloromethane, 1,2-dichloroethane, nitrobenzene and carbon disulfide are preferable. The amount of the solvent ranges usually from 0.5 to 100 ml, preferably from 5 to 20 ml, relative to 1 mmol of the compound [IX] or a salt thereof.

The reaction temperature ranges usually from from about -30°C to about 150°C, preferably from about 20°C to about 100°C. The reaction time ranges usually from 0.5 to 72 hours, preferably from 1 to 16 hours.

And, as the Lewis acid to be employed in this reaction, use is made of, for example, aluminum chloride, zinc chloride, titanium chloride, tin(IV) chloride, boron trifluoride, iron(II) chloride, iron(III) chloride, antimony pentachloride (V), bismuth chloride (III), silver chloride (II), hydrogen fluoride, sulfuric acid, polyphosphoric acid, etc. Among them, aluminum chloride and the like are preferable. The amount of the Lewis acid to be employed ranges usually from 1 to 10 mol., preferably from 2 to 10 mol., relative to 1 mol. of the compound [IX] or a salt thereof.

In the above reaction, the position where the group of the compound [X] 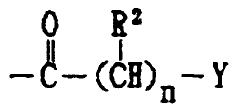 is introduced into the compound [IX] or a salt thereof may be any of the positions on ring A on which the substitution can take place. For example, in the case where the skeleton of the compound [IX] or a salt thereof is 1,2,3,4-tetrahydroquinoline (provided that the ring A is unsubstituted), the group of the compound [X] is introduced principally into 6-position, while such compounds as having the group introduced at any other positions (5-position, 7-position, 8-position) can be produced and separated as well.

The compound [II] or salts thereof obtained thus above can be isolated and refined by a conventional means such as concentration, pH change, phasic transfer, solvent extraction, fractional distillation, distillation, crystallization, recrystallization and chromatography, while they may be fed to the subsequent process as the material in the state of reaction mixture without isolation.

The stating compound [III] or salts thereof can be produced by a per se known method or a method analogous thereto.

The compound [VIII] or salts thereof can be produced by a per se known method or a method analogous thereto.

And, among the compounds [I], those where n is 2, i.e. the compound of the formula: 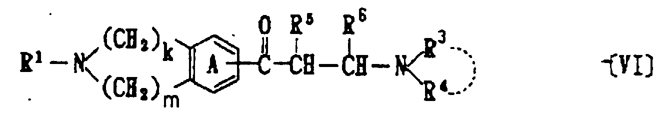 wherein each symbol is of the same meaning as defined above, or a salt thereof, can be produced also by reacting a compound of the formula: 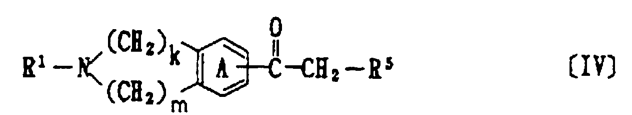 wherein each symbol is of the same meaning as defined above, or a salt thereof, with a compound represented by the formula:

R⁶-CHO [V]

wherein R⁶ is of the same meaning as defined above and a compound represented by the formula: 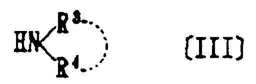 wherein each symbol is of the same meaning as defined above, or a salt thereof.

As salts of the compound [IV], use is made of, for example, those with inorganic acids (e.g. hydrochloric acid, phosphoric acid, hydrobromic acid, sulfuric acid) or with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid). Further, in the case where the compound [IV] of this invention has an acid group such as -COOH, the compound [IV] may be in the form of a salt with an inorganic base (e.g. alkali metal or alkaline earth metal such as sodium, potassium, calcium, magnesium, etc., ammonia) or with an organic base (e.g. tri-C₁₋₃ alkylamine such as triethylamine, etc.).

This reaction can be conducted in substantially the same manner as Mannich reaction described in Organic Reaction, Vol. 1, p 303 to 341. More specifically, this reaction is conducted by reacting, for example, the compound [V] and the compound [III] or a salt thereof, with the compound [IV] or a salt thereof in a ratio of usually 0.9 to 10, preferably 1.0 to 3.0, equivalents of the former relative to 1 equivalent of the latter. Wile this reaction can be carried out usually at temperatures ranging from room temperature to under heating (10 to 150°C), it is conducted preferably at temperatures ranging from 80 to 120°C. The reaction time ranges usually from 1 to 48 hours, preferably from 2 to 24 hours. This reaction can usually be conducted in the absence or presence of solvent. As the solvent, any one to be used in general for Mannich reaction can be employed, unless it hampers this reaction. For example, alcohols such as ethanol are often employed. The amount of the solvent ranges usually from 0.5 to 200 ml, preferably from 5 to 40 ml, relative to 1 mmol. of the compound [IV] or a salt thereof. Further, this reaction can be conducted, when desired, in the presence of an inorganic acid such as hydrochloric acid. The acid is used in a catalytic amount relative to the compound [IV] or a salt thereof (0.001 to 0.05 equivalent relative to 1 equivalent of the compound [IV] or a salt thereof). In the case where the compound [III] or [IV] to be employed for the reaction is not in the form of salt, however, it is preferable to use the acid in an excess amount sufficient for allowing these compounds to form salts.

The compound [IV] or salts thereof and the compound [V] can be produced by a per se known method or a method analogous thereto.

Further, the compound [I] or a salt thereof can be produced also by first hydrolizing the ester moiety (COOR²) of, for example, a compound represented by the formula: 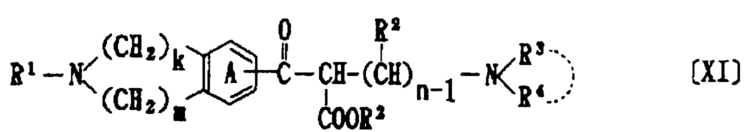 wherein each symbol is of the same meaning as defined above or a salt thereof, which can be obtained by the method described above, then by subjecting the reaction mixture to decarboxylation. The hydrolysis and decarboxylation can be conducted in the same manner as per se known methods.

And, the compound [I] or a salt thereof can be produced also by subjecting, for example, a compound, wherein R¹ is carboxylic acid acyl, or a salt thereof to reduction in a conventional manner. In this reaction, it is preferable that, upon necessity, the functional group (e.g. ketone) of the compound [I] or a salt thereof (R¹ = carboxylic acid acyl group) is first protected, in the form of acetal with, for example, ethylene glycol or any other alcohol (e.g. methanol, ethanol, etc.), then the thus protected compound is subjected to reduction, and then the reaction mixture is subjected to deprotection with an acid or base or by heating.

And, in each of the above-mentioned reactions, in the case where the starting compound has, as substituents, amino group, carboxyl group, hydroxyl group, etc., these groups may be protected with such protecting groups as generally used in peptide chemistry, and the object compound can be obtained by, upon necessity, removing these protecting groups after the reaction.

As the protecting groups of amino group, use is made of, for example, formyl, optionally substituted C₁₋₆ alkyl-carbonyl groups (e.g. acetyl, ethylcarbonyl, etc.), benzoyl, C₁₋₆ alkyloxy-carbonyl (e.g. methoxy-carbonyl, ethoxycarbonyl, etc.), phenyloxycarbonyl (e.g. phenoxycarbonyl, etc.), C₇₋₁₅ aralkyloxy-carbonyl (e.g. benzyloxycarbonyl, fluorenyloxycarbonyl, etc.), trityl, phthaloyl, etc. As substituents on these groups, use is made of halogen atoms (e.g. fluorine, chlorine, bromine, iodine, etc.), C₁₋₆ alkyl-carbonyl (e.g. methyl-carbonyl, ethylcarbonyl, butylcarbonyl, etc.), nitro group, among others, and the number of these substituents ranges from 1 to 3. As the protecting group of carboxyl group, use is made of, for example, an optionally substituted C₁₋₆ alkyl (e.g. methyl, ethyl, n-propyl, i-propyl, n-butyl, tert-butyl, etc.), phenyl, trityl, silyl, etc. As substituents on these groups, use is made of halogen atoms (e.g. fluorine, chlorine, bromine, iodine, etc.), formyl, C₁₋₆ alkyl carbonyl (e.g. methylcarbonyl, ethylcarbonyl, butylcarbonyl, etc.), nitro, etc., and the number of these substituents ranges from 1 to 3. As the group protecting hydroxyl group, use is made of, for example, an optionally substituted C₁₋₆ alkyl (e.g. methyl, ethyl, n-propyl, i-propyl, n-butyl, tert-butyl, etc.), phenyl, C₇₋₁₀ aralkyl (e.g. benzyl, etc.), formyl, C₁₋₆ alkyl carbonyl (e.g. acetyl, ethylcarbonyl, etc.), phenyloxycarbonyl, C₇₋₁₀ aralkyl-carbonyl (e.g. benzyloxycarbonyl, etc.), pyranyl, furanyl, silyl, etc. As substituents on these groups, use is made of halogen atom (e.g. fluorine, chlorine, bromine, iodine, etc.), C₁₋₆ alkyl, phenyl, C₇₋₁₀ aralkyl, nitro group, etc., and the number of these substituents ranges from 1 to 4.

And, as the means of removing these protecting groups, use is made of per se known means or those analogous thereto, as exemplified by those which comprise processing with an acid, a base, reduction, UV-ray, hydrazine, phenyl hydrazine, sodium N-methyldithiocarbamate, tetrabutylammonium fluoride, palladium acetate, etc.

Further, the compound [VII] of this invention or salts thereof can be produced by substantially the same method as described above for the production of the compound [I] or salts thereof, using a compound wherein the group 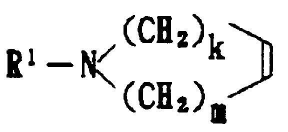 in, for example, the starting compound [II], [IV], [IX] or a salt thereof, is a group

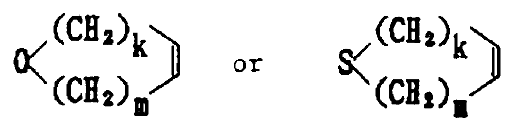

The compound [I] or salts thereof and the compound [VII] or salts thereof thus obtained above can be isolated and refined by conventional separating means such as recrystallization, distillation, chromatography, etc. In the case where the compound [I] and the compound [VII] obtained thus above are in the free form, they can be led to salts by per se conventional means or those analogous thereto. Conversely, when the compound [I] or [VII] is obtained in the form of salt, it can be led to the free compound or any other salt by per se conventional means or those analogous thereto.

The compound [I] or salts thereof and the compound [VII] or salts thereof include their stereoisomers due to the presence of asymmetric carbon atoms. These isomers can be resolved into corresponding optically active compounds by means of a conventional optical resolution.

The compound [I] or salts thereof and the compound [VII] or salts thereof of the present invention act on the central nervous system of mammals, have strong cholinesterase inhibitory activity, and exhibit excellent antiamnestic effects on various amnesia-inducing actions in man and animals (e.g. mice, etc.). Further, the compound [I] or salts thereof and the compound [VII] or salts thereof of the present invention have monoamine (e.g. norepinephrine, serotonin, etc.) reuptake inhibitory activity, and exhibit excellent antidepressant activity, etc. in man and animals (e.g. mice, etc.).

The compound [I] or salts thereof and the compound [VII] or salts thereof of the present invention are remarkably excellent in separation of effects on central nervous system from those on peripheral nervous system, as compared with physostigmine and, at the antiamnestic and antidepressant dose level, do not cause peripheral nervous system effects such as spasm, salivation, diarrhea, etc. or, if they do, only slightly. Moreover, they are characterized by a long duration of effects and low toxicity, ensuring a remarkably high efficacy when administered orally. The acute toxicity of the compound [I] or salts thereof and the compound [VII] or salts thereof of the present invention is not less than 100 mg/kg. Therefore, the compounds of this invention are useful as a safely administrable agent for improving the cerebral function of mammalian animals including human beings.

Diseases on which the compounds of this invention are effective include senile dementia, Alzheimer's diseases, Huntington's chorea, hyperkinesia and mania. The compounds of this invention can be used for the prophylaxis or therapy of these diseases. Further, the compounds of this invention can be also used for the prophylaxis or therapy of depression, hypobulia, affective disorders, lack of spontaneity, etc. which are symptoms related to the above-mentioned diseases.

The compounds of this invention are usually formulated with pharmaceutically acceptable carriers or excipients, which can be administered orally or non-orally to man and other mammalian animals. Such pharmaceutical preparations may be those for oral administration (e.g. powders, tablets, granules and capsules) and for non-oral administration (e.g. suppositories, injections). These pharmaceutical compositions can be prepared by per se known methods. While the dosage depends on the type and symptom of diseases to be treated, in the case of oral administration to general adult humans (60 kg body weight), it ranges from about 0.01 mg to about 50 mg, preferably from 0.1 to 30 mg, more preferably from 0.5 to 10 mg per day.

By the following working examples, reference examples, formulation examples and experimental examples, the present invention will be illustrated in more concrete manner, but they should by no means be construed as defining the metes and bounds of this invention.

In the experimental examples and reference examples, elution in the procedure of column chromatography was carried out under observation by means of TLC (Thin Layer Chromatography) unless otherwise specified. In the TLC observation, 60F₂₅₄ manufactured by Merck was employed as the TLC plate, the solvent employed as elution solvent for the column chromatography was employed as the developer, and a UV detector was employed for detection. As an adjunctive detection procedure, the spot on the TLC plate was sprayed with 48% HBr, heated to hydrolize, sprayed with a ninhydrin reagent and heated again, then the change to a red - reddish purple was regarded as positive reaction. The fractions containing the object compound were pooled. Unless otherwise specified, Merck Kieselgel 60 (70 to 230 mesh) was employed as the silica gel for the column.

Incidentally, the term "ambient temperature" or "room temperature" generally means usually temperatures ranging from about 5°C to 40°C, and the term "atmospheric pressure" means the neighborhood of one atmospheric pressure.

And, unless otherwise specified, % denotes percentage by weight.

### Reference Example 1

### 4-Chloro-1-(3-acetyl-2,3,4,5-tetrahydro-1H-3-benzazepin-7-yl)-1-butanone

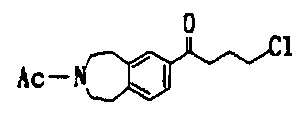

In 30 ml of dichloromethane, was dissolved 3.79 g of 3-acetyl-2,3,4,5-tetrahydro-1H-3-benzazepine. To the solution were added 3.38 g of 4-chlorobutyryl chloride and 4.00 g of aluminum chloride. The mixture was stirred for 2 hours at room temperature (about 20°C). The reaction mixture was poured into 50 ml of ice-water. The organic layer was separated, which was washed successively with 50 ml of 0.5N aqueous solution of sodium hydroxide and 50 ml of pure water, then dried over anhydrous sodium sulfate. The solvent was distilled off to leave 5.40 g of an oily residue. The residue was purified by means of a silica gel column chromatography (developing solvent, ethyl acetate - dichloromethane 1:1 (V/V)) to afford 2.92 g of the title compound as colorless crystals, m.p.103-106°C.

| Elemental Analysis for C₁₆H₂₀ClNO₂: | | | |
|---|---|---|---|
| Calcd.: | C, 65.41; | H, 6.86; | N, 4.77 |
| Found : | C, 65.33; | H, 6.91; | N, 4.69 |

### Reference Example 2

By conducting substantially the same procedure as in Reference Example 1, compounds shown in Table 35 were obtained.

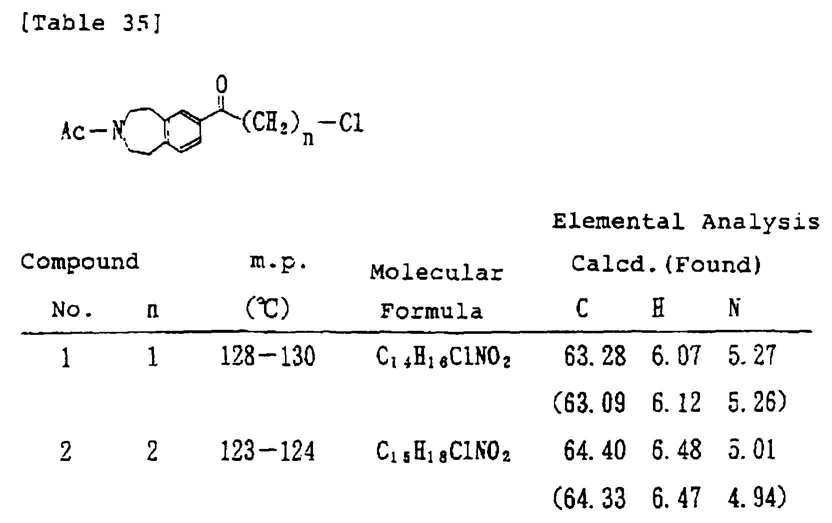

### Reference Example 3

### 7-Acetyl-3-(phenylmethyl)-2,3,4,5-tetrahydro-1H-3-benzazepine

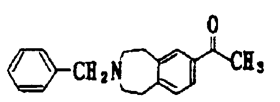

To 7.36 g of 2,3,4,5-tetrahydro-1H-3-benzazepine and 10.37 g of potassium carbonate, was added 75 ml of ethanol. To the mixture, was added dropwise 8.38 g of benzyl bromide under ice-cooling in the course of 10 minutes, followed by stirring for 2 hours at 25°C, then the solvent was distilled off. To the residue were added 100 ml of pure water and 100 ml of dichloromethane, then the organic layer was separated and dried over sodium sulfate, followed by distilling off the solvent to leave a crystalline residue.

The residue was dissolved in 20 ml of methanol, to which was added 19 ml of 4N methanolic hydrochloric acid. Methanol was then distilled off, and the residue was suspended in 100 ml of ethyl acetate. Resulting crystals were collected by filtration, then the crystals were washed with 30 ml of ethyl acetate. The crystals were dried under reduced pressure to give 11.94 g of 3-(phenylmethyl)-2,3,4,5-tetrahydro-1H-3-benzazepine hydrochloride.

10.95 g of the above-mentioned compound was dissolved in 80 ml of 1,2-dichloroethane, to which were added 10.67 g of aluminum chloride and 4.71 g of acetyl chloride, then the mixture was stirred for 30 minutes under reflux. The reaction mixture was poured into 150 ml of ice-water, then pH of the aqueous layer was adjusted to 10, followed by addition of 150 ml of dichloromethane. The organic layer was separated and dried over sodium sulfate. The solvent was then distilled off to leave 13.0 g of an oily residue. The residue was purified by means of a silica gel column chromatography (developing solvent, ethyl acetate - dichloromethane 1:2) to afford 10.44 g of the title compound as colorless crystals, m.p.89-91°C.

| Elemental Analysis for C₁₉H₂₁NO: | | | |
|---|---|---|---|
| Calcd.: | C, 81.68; | H, 7.58; | N, 5.01 |
| Found : | C, 81.49; | H, 7.61; | N, 4.86 |

### Reference Example 4

### Ethyl β-(1-acetyl-2,3,4,5-tetrahydro-1H-1-benzazepin-8-yl)-β-oxopropionate

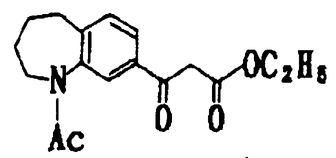

(1) To a solution of 18.9 g of 1-acetyl-2,3,4,5-tetrahydro-1H-1-benzazepine in 20 ml of carbon disulfide were first added 30.8 g of aluminum chloride then 7.8 ml of acetyl chloride gradually at room temperature. The mixture was heated for 16 hours under reflux. The reaction mixture was poured into ice water, which was subjected to extraction with dichloromethane. The extract solution was dried over anhydrous sodium sulfate, then the solvent was distilled off. The residue was purified by means of chromatography (developing solvent; dichloromethane - ethyl acetate = 5:1(V/V)) to give 13.5 g of pale yellow crystals. Recrystallization from diethyl ether - hexane affords 12.4 g of 1,8-diacetyl-2,3,4,5-tetrahydro-1H-1-benzazepine as colorless crystals, m.p.105-108°C.

| Elemental Analysis for C₁₄H₁₇NO₂: | | | |
|---|---|---|---|
| Calcd.: | C, 72.70; | H, 7.41; | N, 6.06 |
| Found : | C, 72.82; | H, 7.36; | N, 6.00 |

(2) To a refluxed solution of 3.83 g of diethyl carbonate and 1.04 g of sodium hydride (oil free) in 50 ml of tetrahydrofuran was added dropwise, under nitrogen atmosphere, a solution of 5 g of 1,8-diacetyl-2,3,4,5-tetrahydro-1H-1-benzazepine in 50 ml of tetrahydrofuran. The mixture was heated for 3 hours under reflux, then the reaction mixture was poured into ice-water, which was subjected to extraction with dichloromethane. The extract solution was dried over anhydrous sodium sulfate, then the solvent was distilled off. The residue was purified by means of chromatography (developing solvent; dichloromethane - ethyl acetate = 5:1(V/V)) to afford 3.5 g of the title compound as a colorless oily product.

| Elemental Analysis for C₁₇H₂₁NO₄: | | | |
|---|---|---|---|
| Calcd.: | C, 67.31; | H, 6.98; | N, 4.62 |
| Found : | C, 67.14; | H, 6.83; | N, 4.63 |

### Reference Example 5

By substantially the same procedure as in Reference Example 1, compounds listed in Table 36 were obtained.

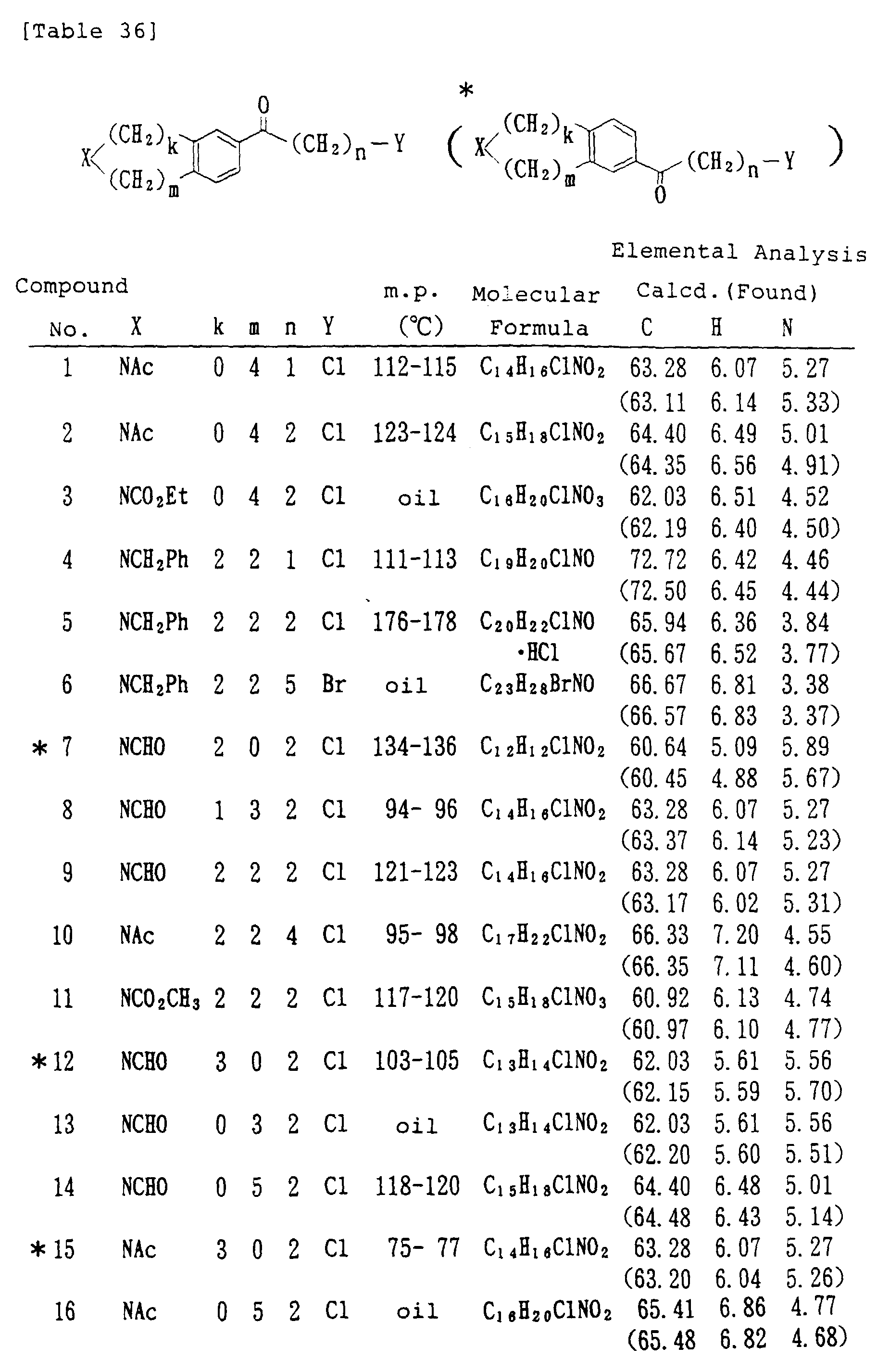

### Reference Example 6

### 1-(3-Methyl-2,3,4,5-tetrahydro-1H-3-benzazepin-7-yl)2-propen-1-one hydrochloride

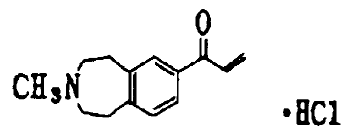

In 30 ml of 1,2-dichloroethane, was dissolved 1.28 g of 3-methyl-2,3,4,5-tetrahydro-1H-3-benzazepine hydrochloride. To the solution were added, at room temperature, 2.1 g of aluminum chloride and 0.66 ml of 3-chloropropionyl chloride, then the mixture was stirred for 2 hours. The reaction mixture was poured into 50 ml of ice-water, then the pH of the aqueous solution was adjusted to not lower than 9 with a 40% aqueous solution of sodium hdyroxide, followed by extraction with 50 ml of dichloromethane. The organic layer was washed with 50 ml of pure water, which was then dried over anhydrous sodium sulfate. The solvent was distilled off under reduced pressure to give an oily residue, which was crystallized from dichloromethane-ether to afford 0.83 g of the title compound as pale yellow crystals, m.p.120-123°C.

| Elemental Analysis for C₁₄H₁₇NO·HCl·H₂O: | | | |
|---|---|---|---|
| Calcd.: | C, 62.33; | H, 7.47; | N, 5.19 |
| Found : | C, 62.53; | H, 7.65; | N, 5.13 |

### Reference Example 7

By substantially the same procedure as in Reference Example 1, the compounds shown in Table 37 were obtained.

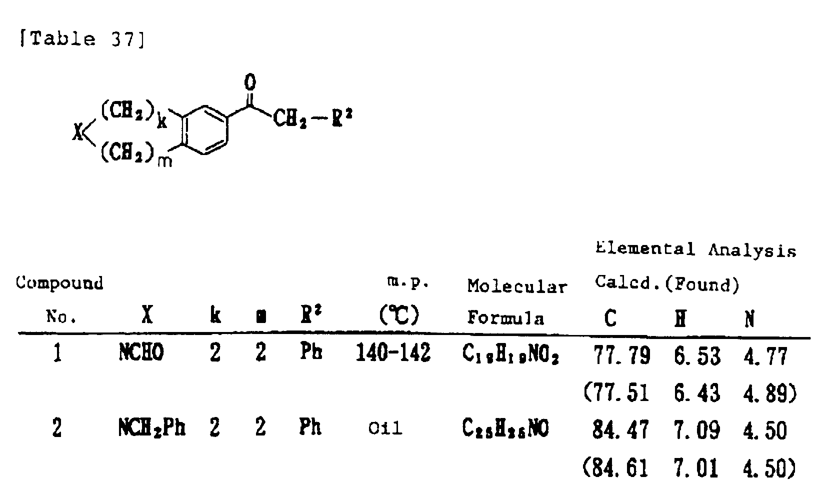

### Reference Example 8

### 4-Bromo-1-(3-formyl-2,3,4,5-tetrahydro-1H-3-benzazepin-7-yl)-2-phenyl-1-butanone

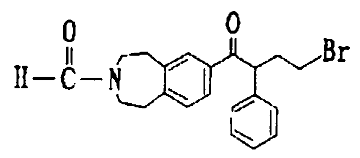

In 50 ml of dimethylformamide was dissolved 2.93 g of the compound No.1 obtained in Reference Example 7, to which was added 0.29 g of 60% sodium hydride. The mixture was stirred for 30 minutes at room temperature, to which was added 4.33 ml of 1,2-dibromoethane, then the mixture was stirred for further 5 hours. The reaction mixture was poured into 150 ml of pure water, which was subjected to extraction with 150 ml of ethyl acetate. The organic layer was washed twice with 150 ml each portion of a saturated aqueous saline solution, which was dried over sodium sulfate, followed by distilling off the solvent under reduced pressure to leave an oily residue. The oily residue was subjected to a silica gel (150 g) column chromatography, eluting with dichloromethane - ethyl acetate (4:1), to afford 1.05 g of the title compound as a colorless oily product.

| Elemental Analysis for C₂₁H₂₂BrNO₂: | | | |
|---|---|---|---|
| Calcd.: | C, 63.01; | H, 5.54; | N, 3.50 |
| Found : | C, 63.07; | H, 5.62; | N, 3.53 |

### Reference Example 9

Using the compound obtained in Reference Example 7, substantially the same procedure as in Reference Example 8 was followed to give the compound shown in Table 38.

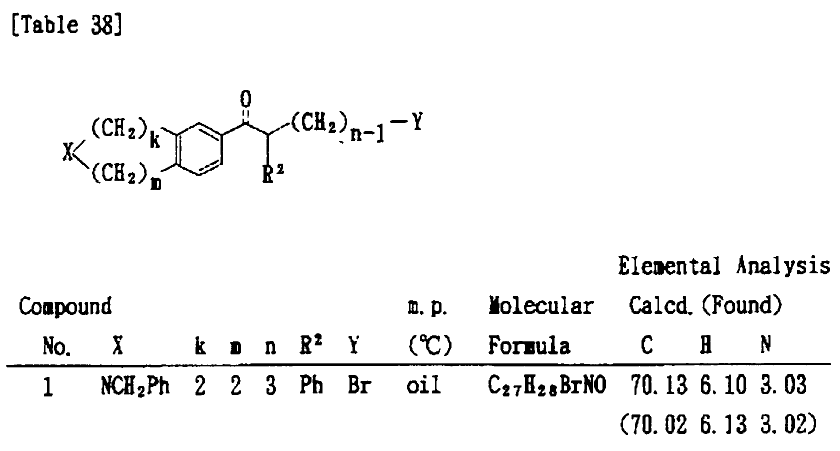

### Working Example 1

### 1-(3-Acetyl-2,3,4,5-tetrahydro-1H-3-benzazepin-7-yl)-4-(piperidin-1-yl)-1-butanone

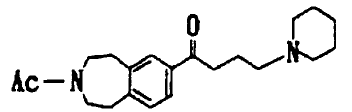

To a solution of 2.64 g of the compound obtained in Reference Example 1 in 50 ml of toluene, were added 1.50 g of piperidine and 20 mg of KI. The mixture was stirred for 12 hours under reflux. The reaction mixture was cooled, to which was then added 50 ml of pure water. The organic layer was separated, from which the solvent was distilled off to leave an oily residue. The residue was purified by means of alumina chromatography (developing solvent; ethyl acetate) to afford 2.15 g of the title compound as colorless powder.

| Elemental Analysis for C₂₁H₃₀N₂O₂: | | | |
|---|---|---|---|
| Calcd.: | C, 73.65; | H, 8.83; | N, 8.18 |
| Found : | C, 73.60; | H, 8.74; | N, 8.09 |

### Working Example 2

### 1-[3-(Phenylmethyl)-2,3,4,5-tetrahydro-1H-3-benzazepin-7-yl]-4-(piperidin-1-yl)-1-butanone dihydrochloride

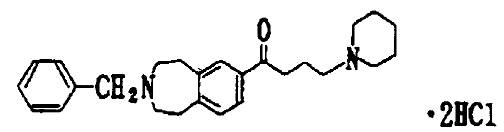

To 2.10 g of the compound obtained in Working Example 1 was added 21 ml of conc. hydrochloric acid, and the mixture was heated for 16 hours under reflux. Excess volume of the conc. hydrochloric acid was distilled off under reduced pressure. To the residue was added 50 ml of water, and the mixture was washed with 50 ml of dichloromethane. The aqueous layer was made basic with an aqueous solution of sodium hydroxide, which was subjected to extraction with dichloromethane. The extract solution was dried over anhydrous sodium sulfate, then the solvent was distilled off to leave 1.80 g of an oily substance.

To a suspension of 1.80 g of the above-mentioned oily substance and 1.30 g of potassium carbonate in 20 ml of ethanol was added dropwise a solution of 0.97 g of benzyl bromide in 5 ml of ethanol. The mixture was stirred for 4 hours at room temperature. The solvent was distilled off under reduced pressure. To the residue was added 30 ml of water, and the mixture was subjected to extraction with dichloromethane. The extract solution was dried over anhydrous sodium sulfate, followed by distilling off the solvent to give 1.37 g of the free form of the title compound as colorless powder, m.p.90-92°C. 1.0 g of the thus obtained free compound was dissolved in methanol, to which was added 1.5 ml of 4N-methanolic hydrochloride. The solvent was distilled off under reduced pressure to leave a solid substance, which was recrystallized from methanol - ethyl acetate to afford 0.93 g of the title compound as colorless crystals, m.p.210-215°C (decomp.)

| Elemental Analysis for C₂₆H₃₄N₂O·2HCl: | | | |
|---|---|---|---|
| Calcd.: | C, 67.38; | H, 7.83; | N, 6.04 |
| Found : | C, 67.12; | H, 7.81; | N, 6.00 |

### Working Example 3

Using the compound obtained in Reference Example 2, the procedure of Working Example 1 was followed to give compounds shown in Table 39.

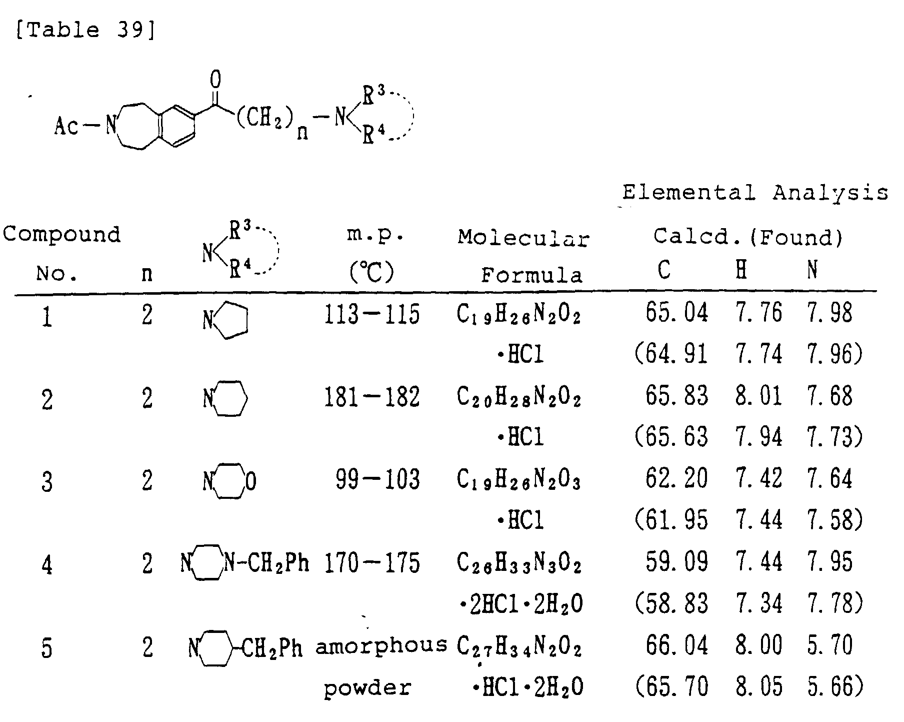

### Working Example 4

Using the compound obtained in Working Example 3, the procedure of Working Example 2 was followed to give the compound shown in Table 40.

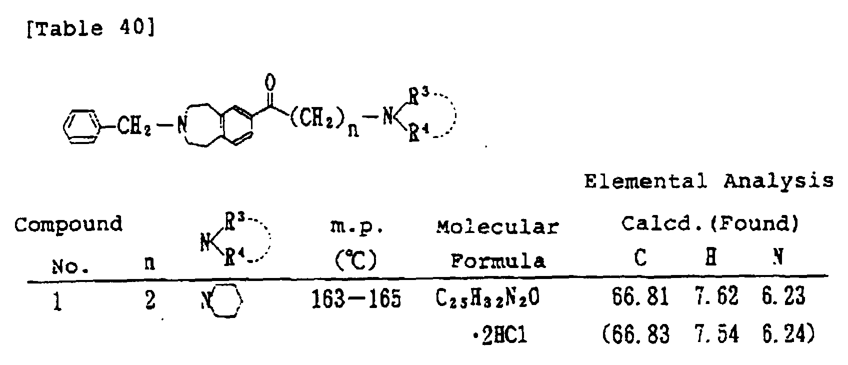

### Working Example 5

### 1-[3-(Phenylmethyl)-2,3,4,5-tetrahydro-1H-3-benzazepin-7-yl]-3-(piperidin-1-yl)-1-propanone dihydrochloride

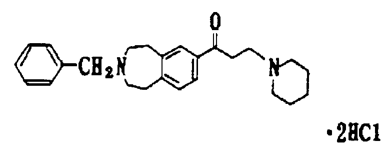

To a solution of 1.40 g of the compound obtained in Reference Example 3, 1.22 g of piperidine hydrochloride, 1.5 g of paraformaldehyde in 50 ml of ethanol was added 0.7 ml of conc. hydrochloride, and the mixture was stirred for 24 hours under reflux. The solvent was distilled off. To the residue were added 50 ml of ethyl acetate and 50 ml of pure water. The aqueous layer was separated, whose pH was adjusted to not lower than 10, followed by extraction with 50 ml of ethyl acetate. The extract solution was dried over anhydrous sodium sulfate, from which the solvent was distilled off to leave 2.15 g of an oily residue.

The residue was purified by means of alumina chromatography (developing solvent; dichloromethane - ethyl acetate = 9:1) to give 1.4 g of an oily substance. The oily substance was dissolved in 15 ml of methanol, to which was added 2.0 ml of 4N methanolic hydrochloric acid. The solvent was distilled off, and the crystalline residue was recrystallized from ethanol - ethyl acetate to afford 1.05 g of the title compound as colorless crystals, m.p.163-165°C.

| Elemental Analysis for C₂₅H₃₂N₂O·2HCl: | | | |
|---|---|---|---|
| Calcd.: | C, 66.81; | H, 7.62; | N, 6.23 |
| Found : | C, 66.65; | H, 7.64; | N, 6.17 |

### Working Example 6

Using the compound obtained in Reference Example 3, the procedure of Working Example 5 was followed to give compounds shown in Table 41.

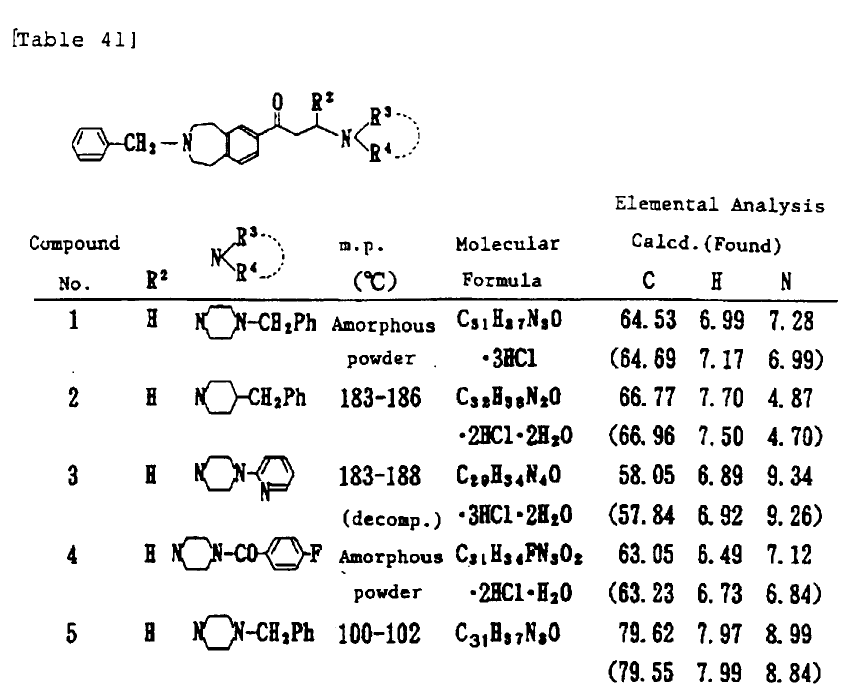

### Working Example 7

### 7-[N-ethyl-N-(phenylmethyl)amino]-1-(2,3,4,5-tetrahydro-1H-1-benzazepin-8-yl)-1-heptanone fumarate

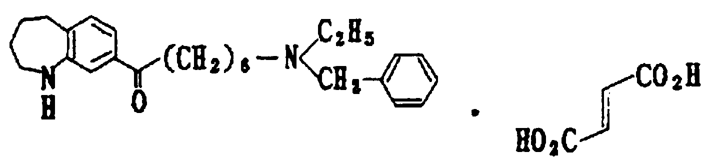

(1) A mixture of 1.7 g of ethyl β-(1-acetyl-2,3,4,5-tetrahydro-1H-1-benzazepin-8-yl)-β-oxopropionate, 2.6 g of 1,5-dibromopentane, 0.93 g of potassium carbonate and 50 ml of acetone was heated for 16 hours under reflux. The reaction mixture was left standing for cooling, then the resulting solid matter was filtered off. From the filtrate was distilled off the solvent. The residue was purified by means of column chromatography (developing solvent; dichloromethane - ethyl acetate = 10:1(V/V)) to afford 1.8 g of ethyl β-(1-acetyl-2,3,4,5-tetrahydro-1H-1-benzazepin-8-yl)-α-(5-bromopentyl)-β-oxopropionate as a viscous oily substance.

| Elemental Analysis for C₂₂H₃₀BrNO₄: | | | |
|---|---|---|---|
| Calcd.: | C, 58.41; | H, 6.68; | N, 3.10 |
| Found : | C, 58.26; | H, 6.63; | N, 3.04 |

(2) A solution of 0.5 g of the compound obtained in (1) and 0.3 g of N-ethylbenzylamine in 10 ml of toluene was heated for 24 hours under reflux. The reaction mixture was left standing for cooling, then the resulting solid matter was filtered off. From the filtrate was distilled off the solvent. To the residue was added 30 ml of conc. hydrochloric acid. The mixture was heated for 24 hours under reflux, then excess volume of conc. hydrochloric acid was distilled off under reduced pressure. To the residue was added a 5% aqueous solution of sodium hydroxide, which was subjected to extraction with dichloromethane. The extract solution was dried over anhydrous sodium sulfate, from which the solvent was distilled off. The residue was purified by means of column chromatography (developing solvent; ethyl acetate - methanol=20:1(V/V)) to give 55 mg of a colorless oily product, which was processed with 16 mg(one equivalent) of fumaric acid to afford 60 mg of the title compound as an amorphous powder.

| Elemental Analysis for C₂₆H₃₆N₂O·C₄H₄O₄: | | | |
|---|---|---|---|
| Calcd.: | C, 70.84; | H, 7.93; | N, 5.51 |
| Found : | C, 70.59; | H, 8.04; | N, 5.47 |

### Working Example 8

### 1-(3-Acetyl-2,3,4,5-tetrahydro-1H-3-benzazepin-7-yl)-2-(piperidin-1-yl)-1-ethanone hydrochloride

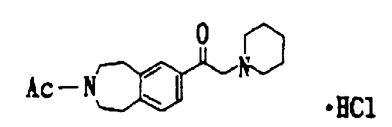

In 10 ml of dichloromethane was dissolved 1.50 g of 1-(3-acetyl-2,3,4,5-tetrahydro-1H-3-benzazepin-7-yl)-2-chloro-1-ethanone obtained in Reference Example 2. To the solution was added 1.7 ml of piperidine, and the mixture was stirred for one hour at room temperature. The reaction mixture was poured into an aqueous solution of potassium carbonate, which was subjected to extraction with dichloromethane. The extract was dried over anhydrous sodium sulfate, from which was distilled off the solvent to leave an oily residue. To the residue was dissolved in 5 ml of methanol, to which was added 1.7 ml of 4N methanolic hydrochloric acid. Then the solvent was distilled off to leave 1.50 g of the title compound as colorless powder.

| Elemental Analysis for C₁₉H₂₆N₂O₂·HCl: | | | |
|---|---|---|---|
| Calcd.: | C, 65.04; | H, 7.76; | N, 7.98 |
| Found : | C, 64.92; | H, 7.81; | N, 7.87 |

### Working Example 9

### 1-[3-(Phenylmethyl)-2,3,4,5-tetrahydro-1H-3-benzazepin-7-yl]-2-(piperidin-1-yl)-1-ethanone dihydrochloride

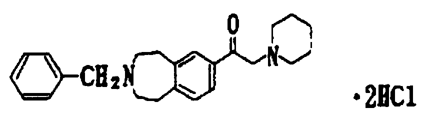

To 1.40 g of the compound obtained in Working Example 8 was added 20 ml of conc. hydrochloric acid. The mixture was stirred for 13 hours under reflux. The reaction mixture was cooled to 25°C, to which was added 50 ml of pure water. The mixture was washed with 50 ml of dichloromethane. The aqueous layer was made basic with an aqueous solution of sodium hdyroxide, which was subjected to extraction with 50 ml of dichloromethane. The extract was dried over anhydrous sodium sulfate, from which was distilled off the solvent to leave 1.15 g of an oily product.

To a suspension of 0.50 g of the above-mentioned oily product and 0.31 g of potassium carbonate in 10 ml of ethanol was added dropwise a solution of 0.30 g of benzyl bromide in 5 ml of ethanol, and the mixture was stirred for 16 hours at room temperature. The solvent was distilled off. To the residue were added 20 ml of dichloromethane and 20 ml of pure water. The organic layer was separated, dried over anhydrous sodium sulfate, followed by distilling off the solvent. The residue was dissolved in 10 ml of methanol, to which was added 1.1 ml of 4N methanolic hydrochloric acid. The solvent was then distilled off to leave a solid matter. Recrystallization from methanol - ethyl acetate afforded 0.2 g of the title compound as colorless crystals, m.p.236-239°C (decomp.).

| Elemental Analysis for C₂₄H₃₀N₂O·2HCl: | | | |
|---|---|---|---|
| Calcd.: | C, 66.20; | H, 7.41; | N, 6.43 |
| Found : | C, 66.11; | H, 7.39; | N, 6.38 |

### Working Example 10

Using the compounds obtained in Reference Example 1 or Reference Example 5, the procedure of Working Example 1 was followed to give compounds shown in Table 42, Table 43, Table 44 and Table 45.

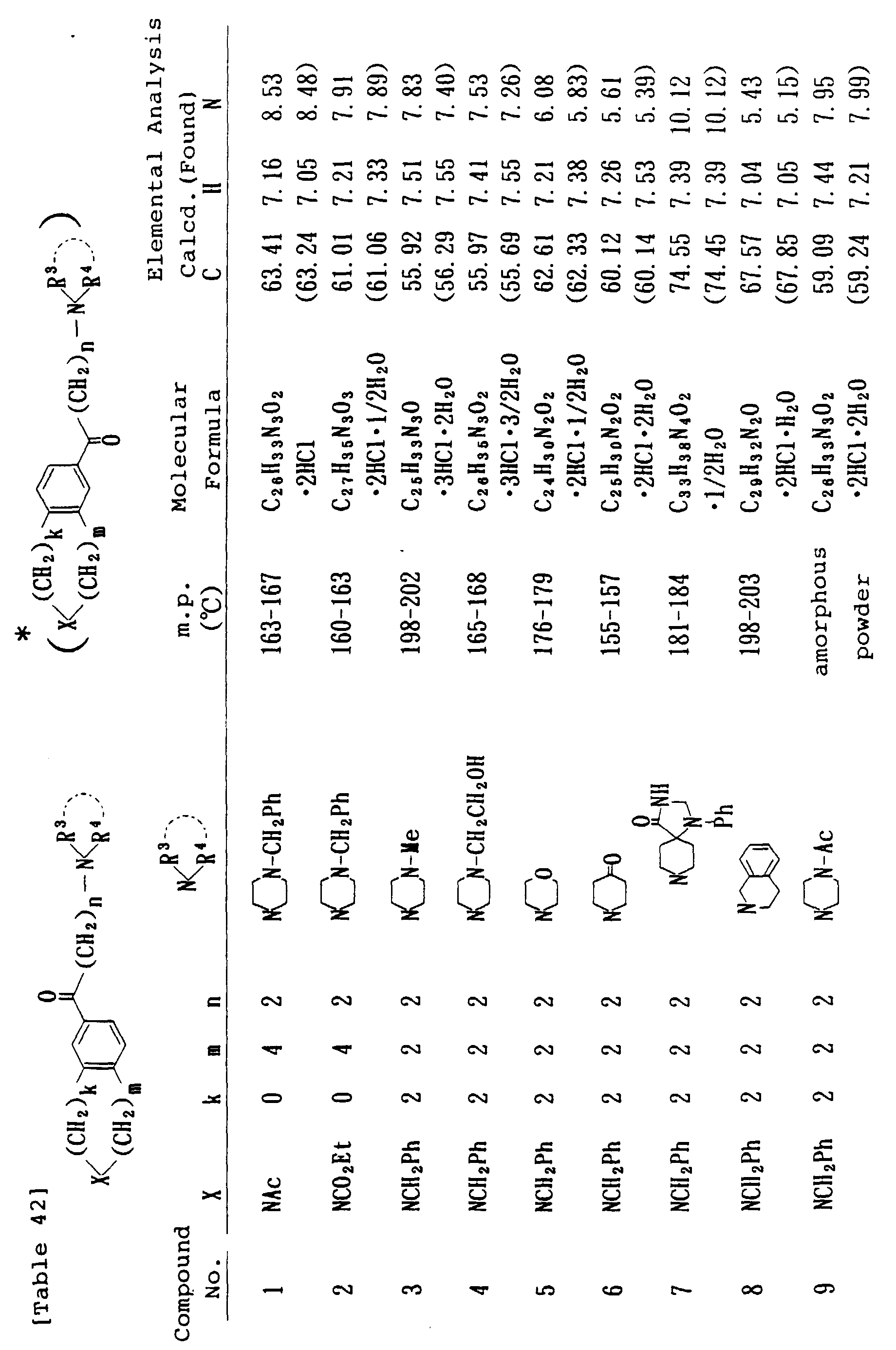

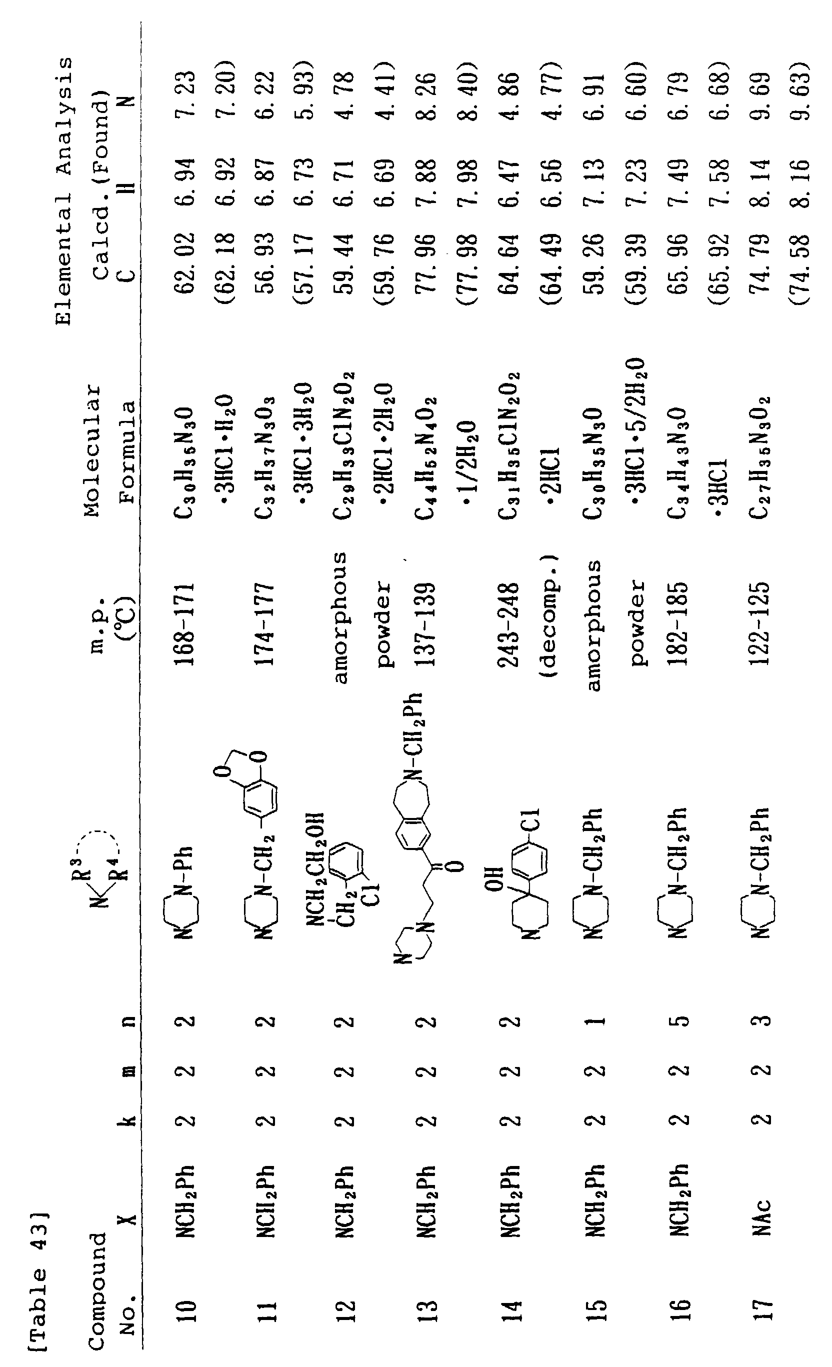

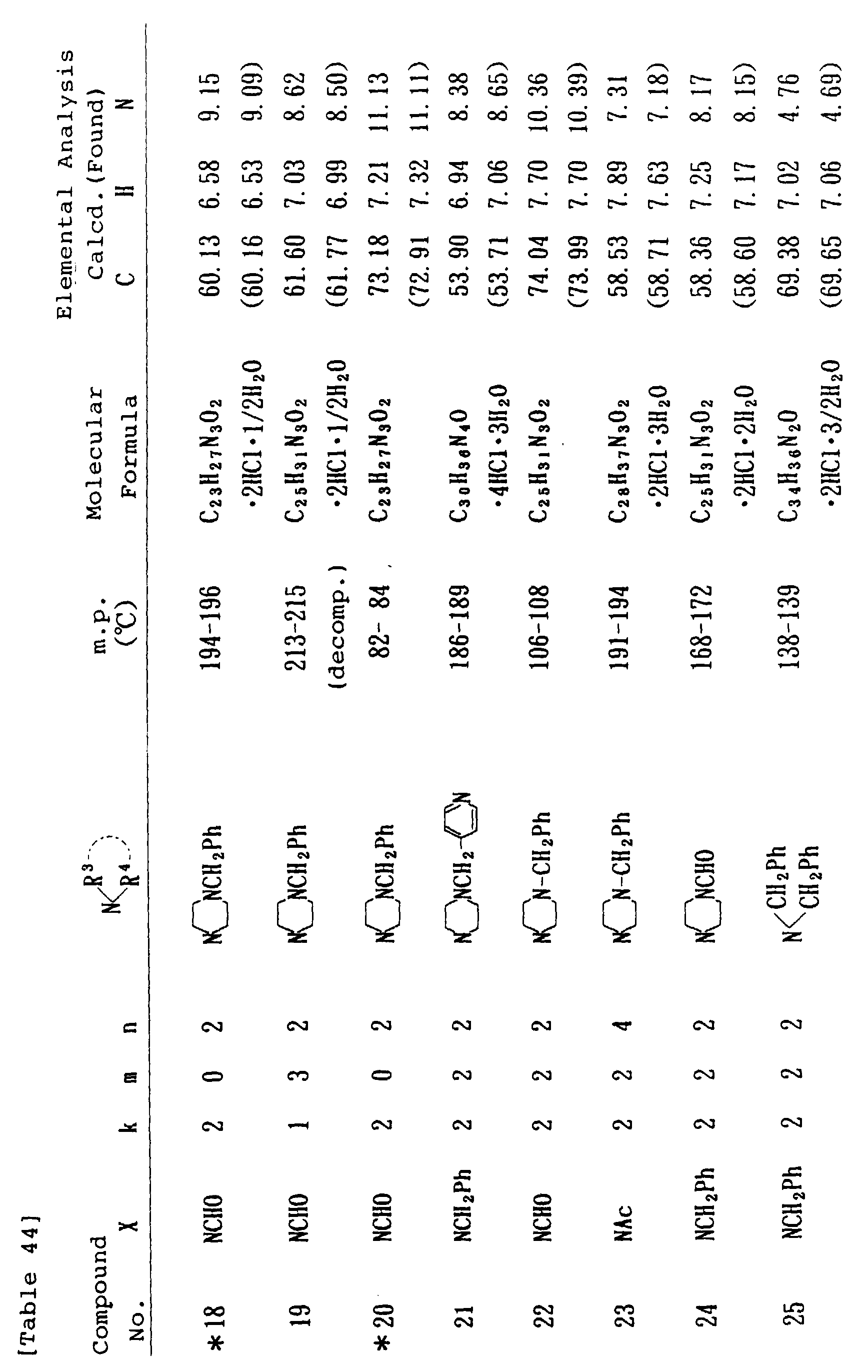

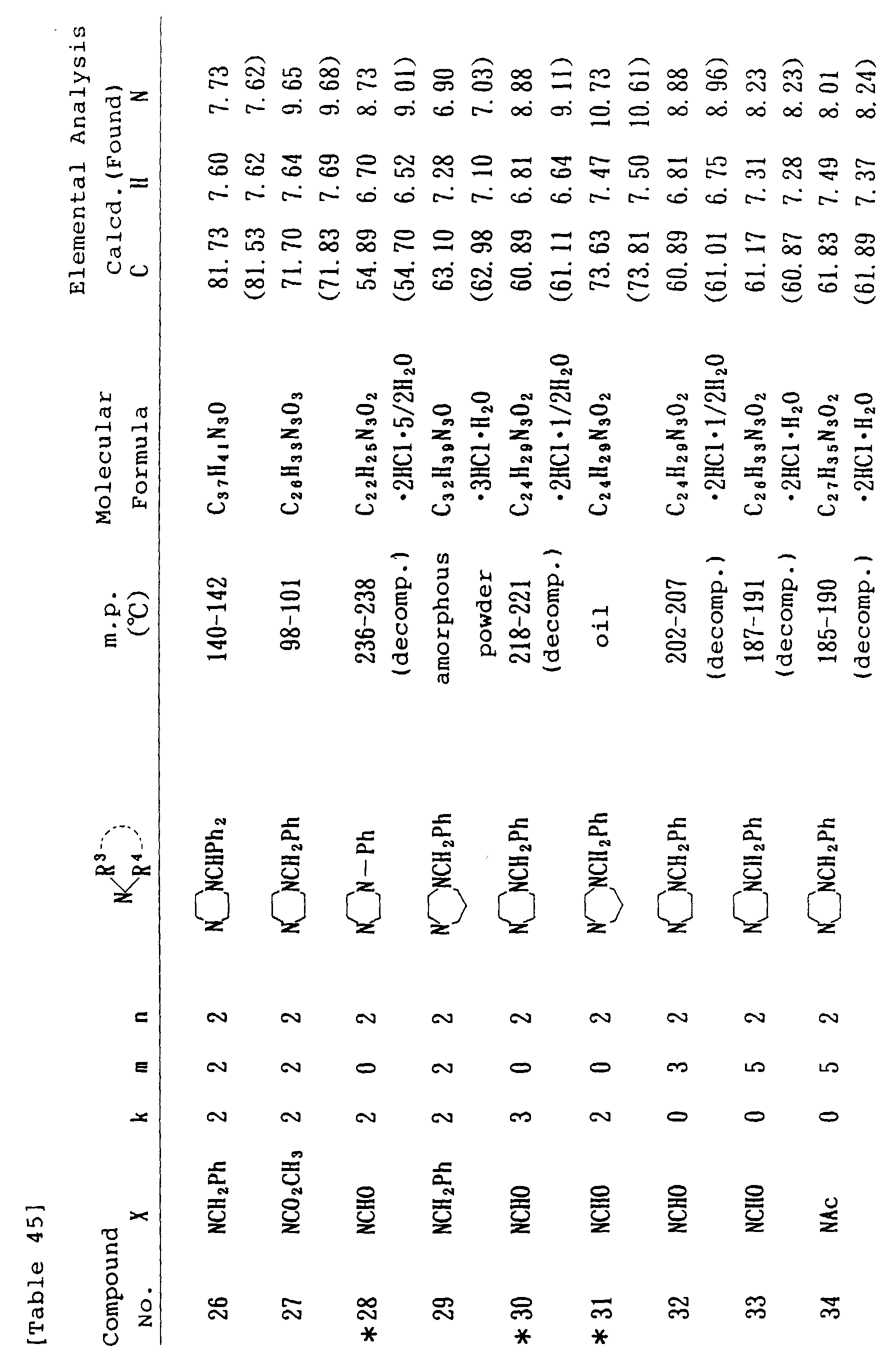

### Working Example 11

### 3-[4-(Phenylmethyl)piperazin-1-yl]-1-(2,3,4,5-tetrahydro-1H-1-benzazepin-8-yl)-1-propanone trihydrochloride

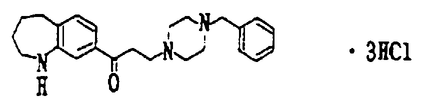

In 100 ml of 6N hydrochloric acid was dissolved 2.0 g of the compound No.2 in Working Example 10. The solution was heated for 16 hours under reflux. Hydrochloric acid was distilled off under reduced pressure. The residue was dissolved in water, which was neutralized with a 5% aqueous solution of sodium hydroxide, followed by extraction with dichloromethane. The extract solution was dried over anhydrous sodium sulfate, from which was distilled off the solvent. The residue was purified by means of column chromatography (developing solvent; ethyl acetate - methanol = 40:1 (v/v)) to give 1.2 g of the starting compound (free base) and 0.3 g of the title compound (free base). The title compound (free base) (0.3 g) was treated with triequivalent hydrochloric acid to afford 0.35 g of the title compound (trihydrochloride) as colorless powder, m.p.145-149°C.

| Elemental Analysis for C₂₄H₃₁N₃O·3HCl: | | | |
|---|---|---|---|
| Calcd.: | C, 59.20; | H, 7.04; | N, 8.63 |
| Found : | C, 59.04; | H, 7.20; | N, 8.53 |

### Working Example 12

### 1-(2,3,4,5-Tetrahydro-1H-3-benzazepin-7-yl)-4-[4-(phenylmethyl)piperazin-1-yl)-1-butanone trihydrochloride

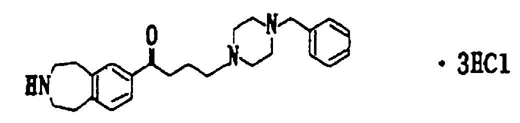

To 2.17 g of 1-(3-acetyl-2,3,4,5-tetrahydro-1H-3-benzazepin-7-yl)-4-(phenylmethyl)piperazin-1-yl]-1-butanone, i.e. Compound No. 17 in Working Example 10, was added 50 ml of conc. hydrochloric acid. The mixture was heated for 24 hours under reflux. Excess amount of conc. hydrochloric acid was distilled off under reduced pressure. To the residue was added 50 ml of water. The mixture was washed with 50 ml of dichloromethane. The aqueous layer was made basic with an aqueous solution of sodium hydroxide, which was subjected to extraction with dichloromethane. The extract solution was dried over anhydrous sodium sulfate. The solvent was distilled off to give 2.0 g of an oily product.

The above-mentioned oily product was dissolved in methanol, to which was added 4.5 ml of 4N-methanolic hydrochloric acid. The solvent was distilled off to leave colorless crystals. The crystals were suspended in 50 ml of ethyl acetate - ether (1:1), followed by collecting the crystals by filtration to give 2.1 g of the title compound as colorless crystals, m.p.201-204°C.

| Elemental Analysis for C₂₅H₃₃N₃O·3HCl·H₂O: | | | |
|---|---|---|---|
| Calcd.: | C, 57.86; | H, 7.38; | N, 8.10 |
| Found : | C, 57.46; | H, 7.36; | N, 8.28 |

### Working Example 13

Using the compound obtained in Working Example 10, the procedure of Working Example 11 (procedure A) or the procedure of Working Example 12 (procedure B) was followed to give compounds shown in Table 46.

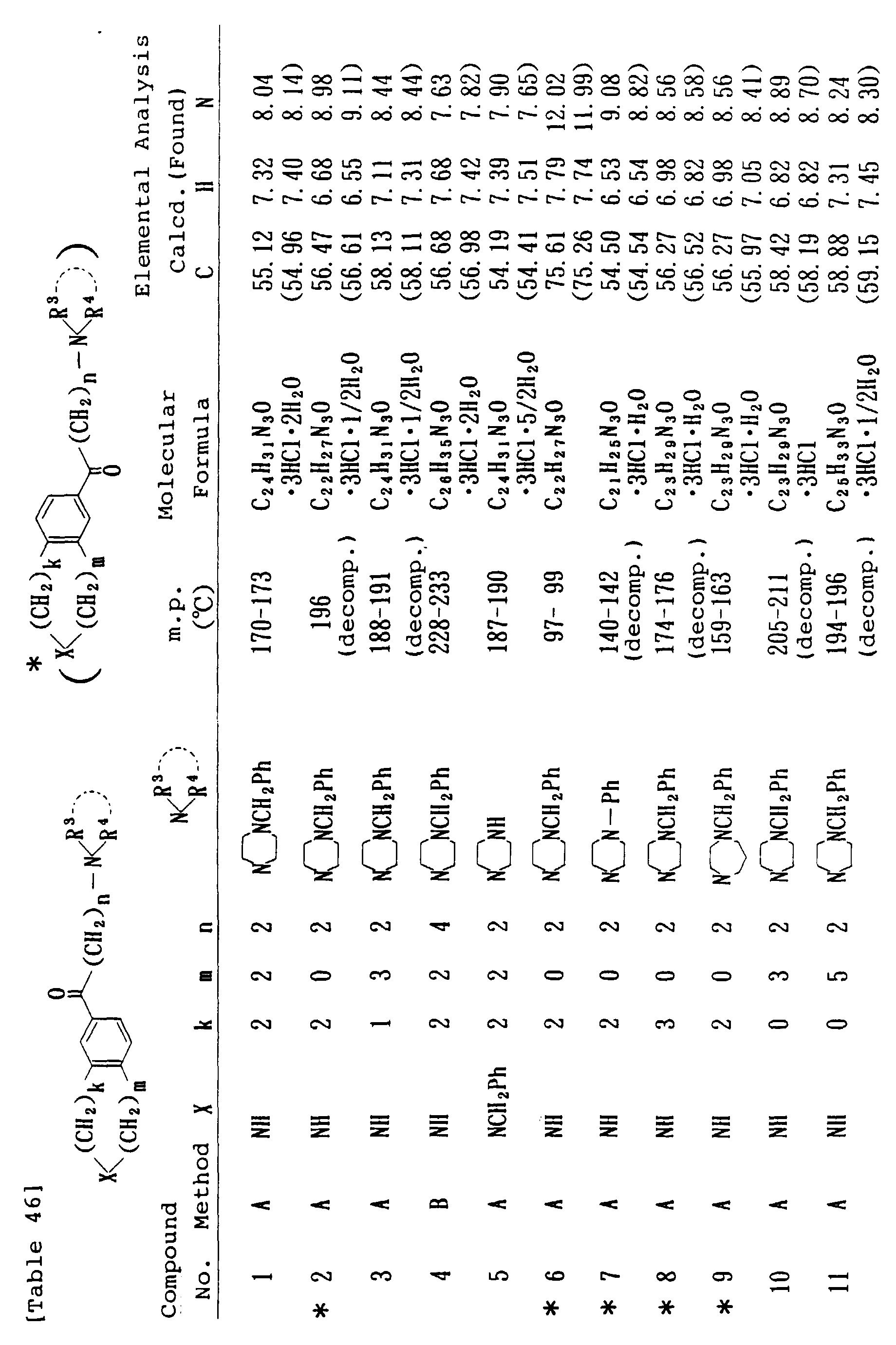

### Working Example 14

By using the compound obtained in Working Example 13, the procedure of Working Example 1 was followed to give compounds shown in Tables 47 - 51.

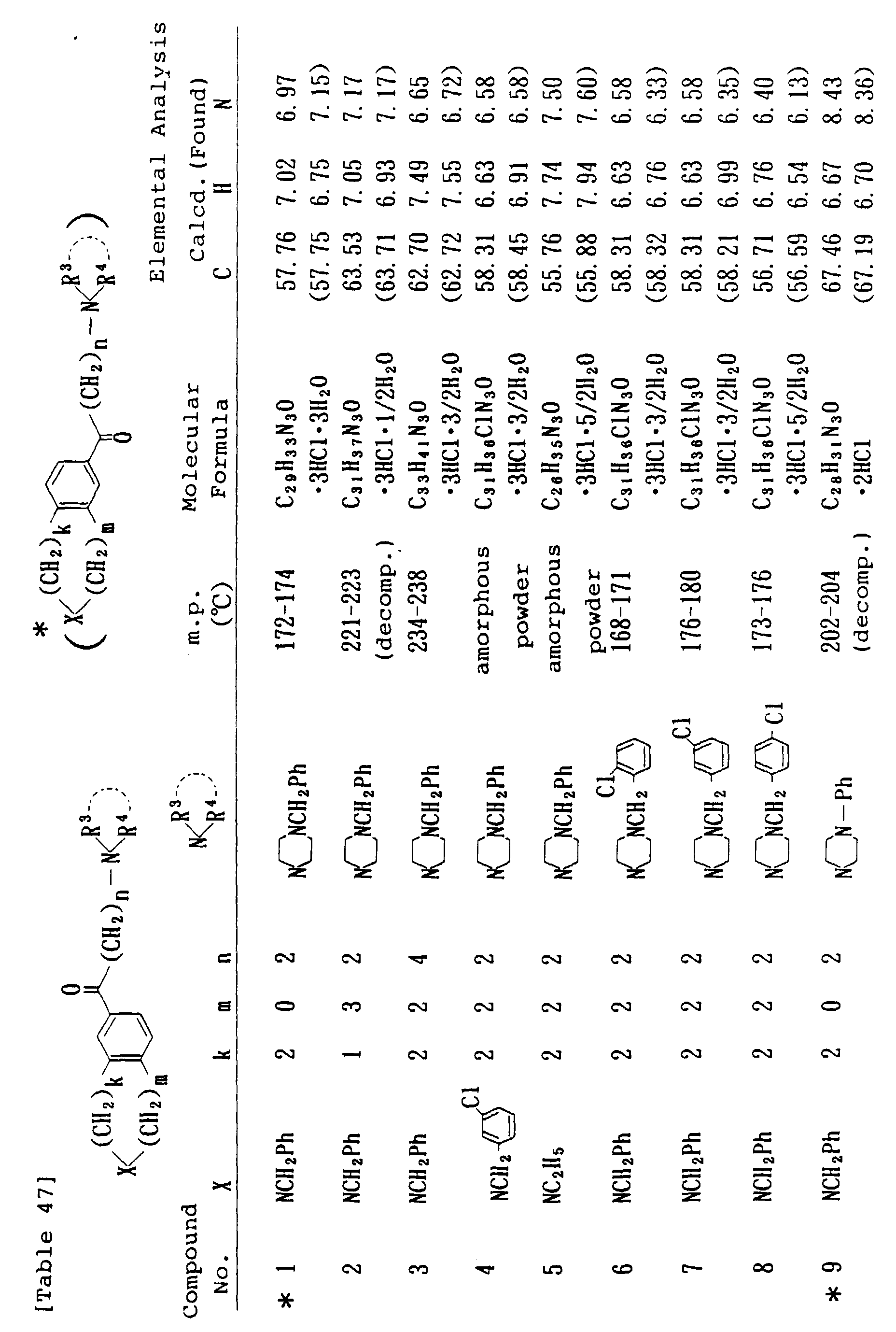

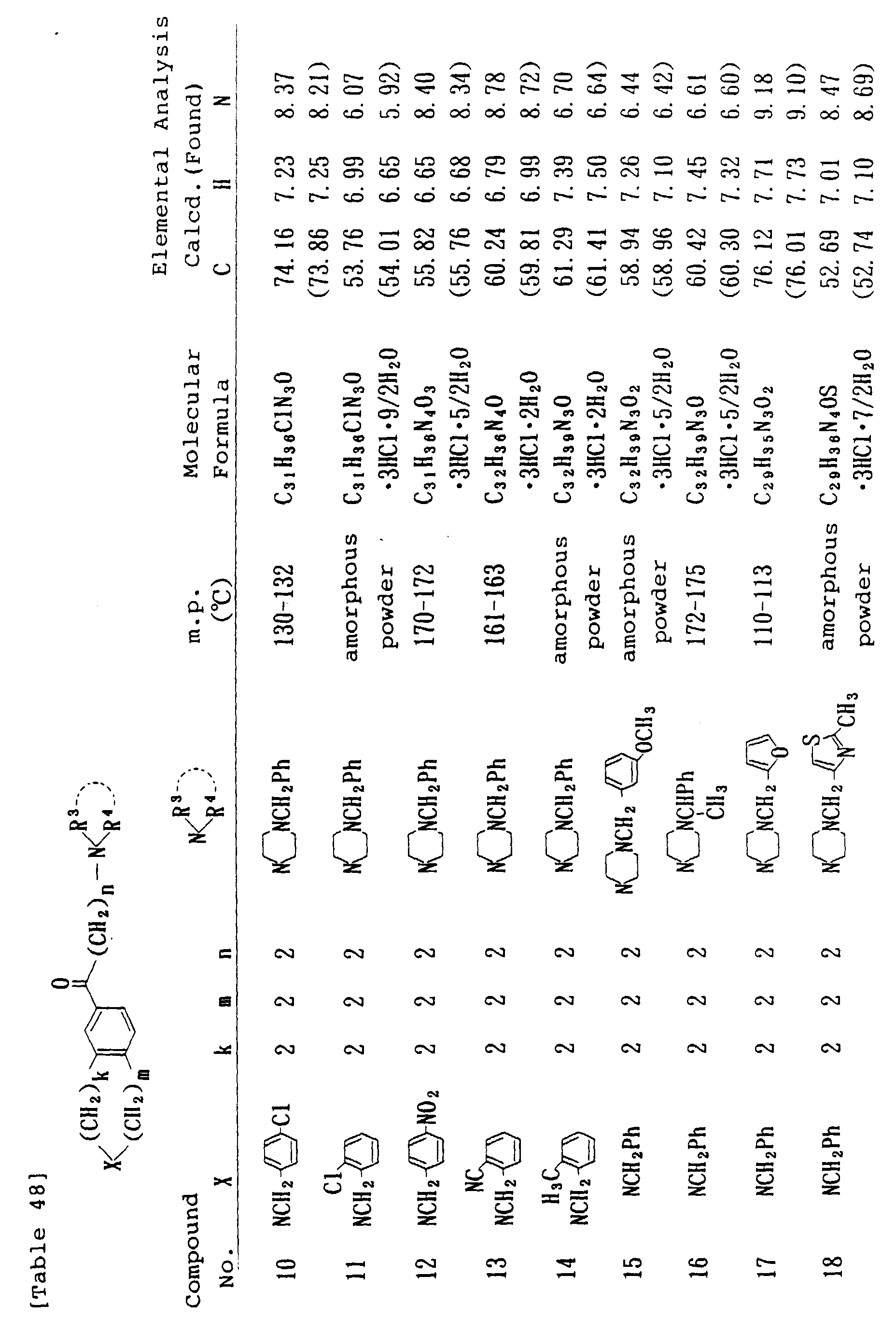

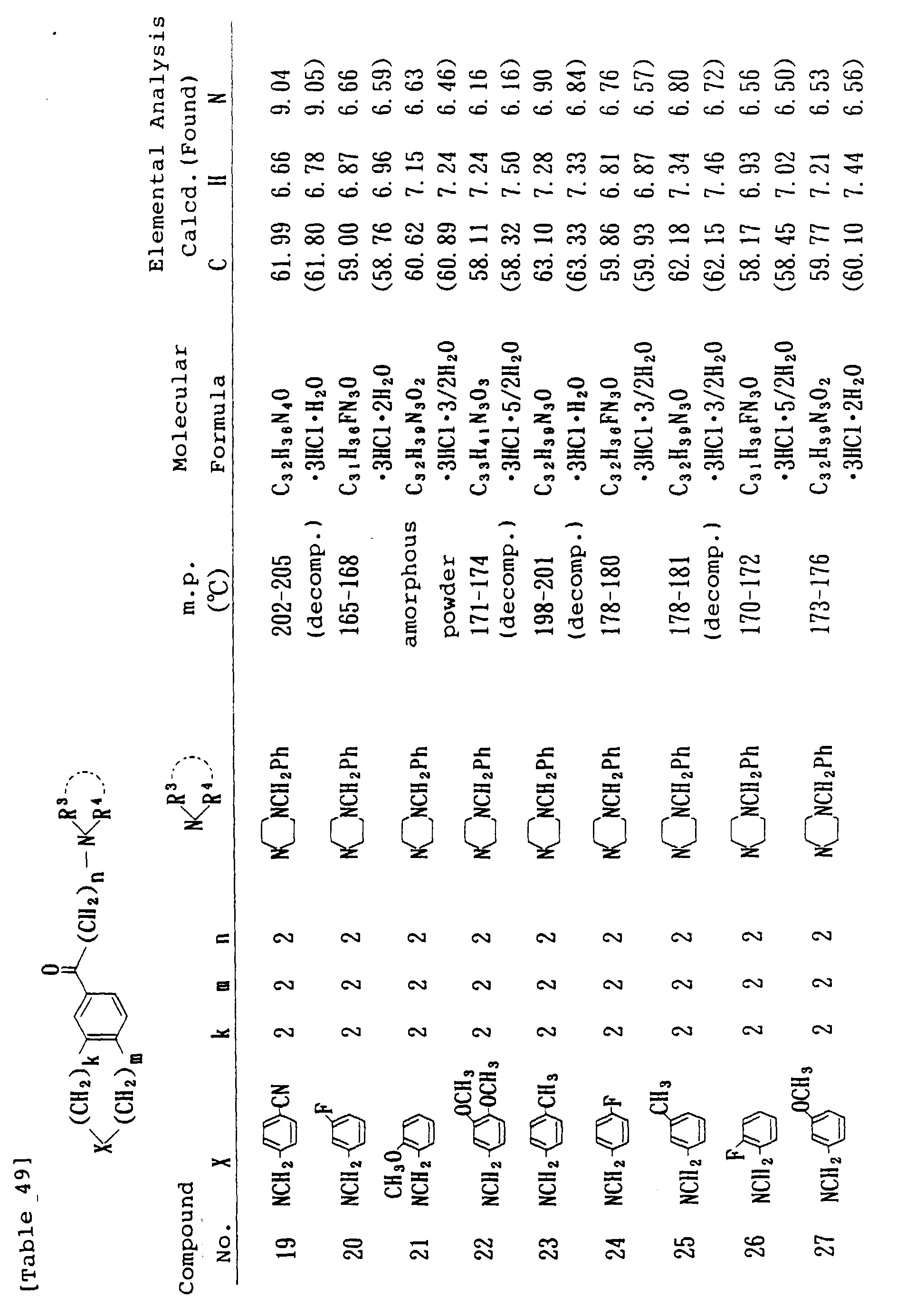

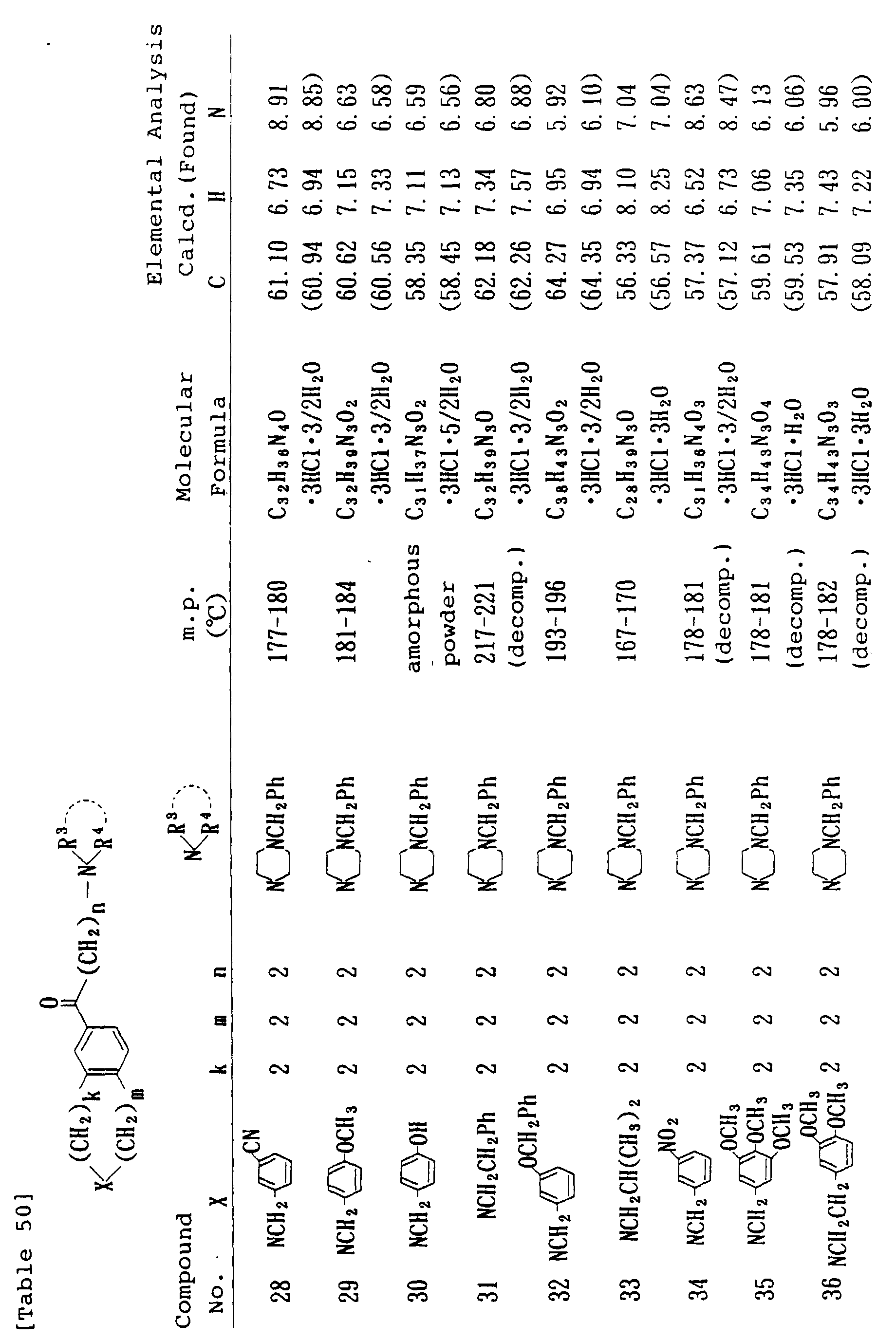

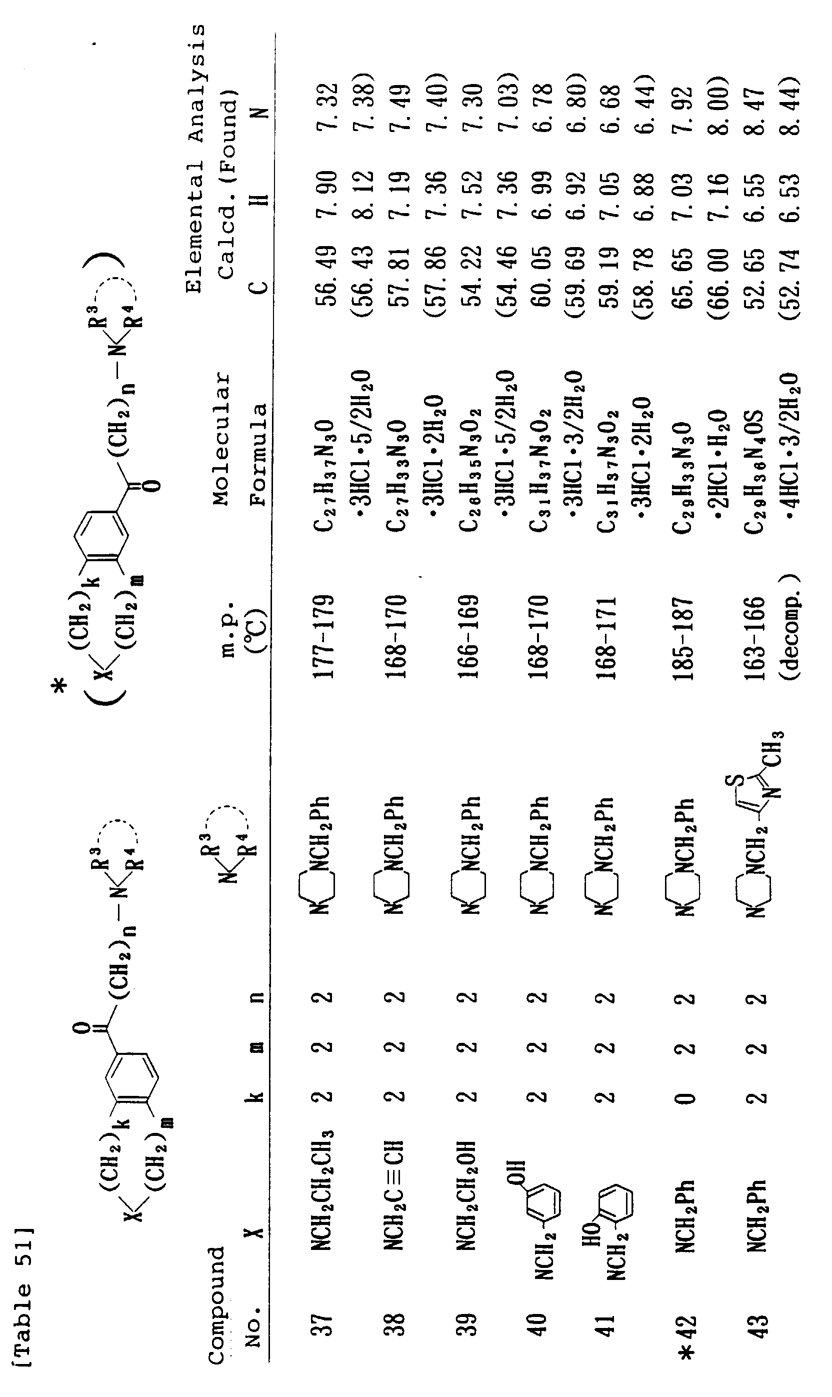

### Working Example 15

### 3-Diphenylamino-1-[3-(phenylmethyl)-2,3,4,5-tetrahydro-1H-3-benzazepin-7-yl]-1-propanone hydrochloride

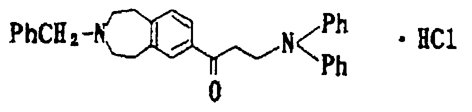

To a solution of 0.98 g of the Compound No. 5 of Reference Example 5 in 30 ml of 1,2-dichloroethane was added 0.63 ml of triethylamine. The mixture was stirred for one hour at room temperature, to which was added 0.56 g of diphenylamine, followed by heating for 72 hours under reflux. The reaction mixture was cooled to room temperature, which was then poured into 50 ml of pure water. To the mixture was added a 1N aqueous solution of sodium hydroxide. The aqueous layer was adjusted to pH not lower than 12, followed by extraction with dichloromethane. The extract solution was dried over anhydrous sodium sulfate, then the solvent was distilled off to leave an oily product. The oily product was purified by means of a silica gel column chromatography (developing solvent: dichloromethane - ethyl acetate =4:1 (v/v)) to afford 0.30 g of the free base of the title compound. The free base was added to 4N methanolic hydrochloric acid, then the solvent was distilled off. The residue was triturated from diethyl ether - hexane to afford 0.25 g of the title compound as amorphous powder.

| Elemental Analysis for C₃₂H₃₂N₂O·HCl·H₂O: | | | |
|---|---|---|---|
| Calcd.: | C, 74.62; | H, 6.85; | N, 5.44 |
| Found : | C, 74.29; | H, 6.91; | N, 5.40 |

### Working Example 16

### 1-(1-Acetyl-2,3-dihydro-1H-indol-5-yl)-3-[4-(phenylmethyl)piperazin-1-yl]-1-propanone dihydrochloride

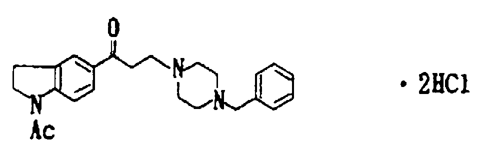

To a solution of 0.7 g of the Compound No.6 of Working Example 13 in 40 ml of dichloromethane was added dropwise 0.2 g of acetic anhydride. The mixture was stirred for 2 hours at room temperature. To the reaction mixture was added a saturated aqueous solution of sodium hydrogencarbonate, followed by extraction with dichloromethane. The extract solution was dried over anhdyrous sodium sulfate, then the solvent was distilled off. The residue was crystallized from diethyl ether to give 0.54 g of the free base of the title compound as pale yellow crystals, m.p.107-109°C.

To 0.35 g of the above-mentioned free base was added 4N methanolic hydrochloric acid (2 equivalents), followed by distilling off the solvent to afford 0.35 g of the title compound as crystalline powder, m.p.216-218°C.

| Elemental Analysis for C₂₄H₂₉N₃O₂·2HCl·2H₂O: | | | |
|---|---|---|---|
| Calcd.: | C, 57.60; | H, 7.05; | N, 8.40 |
| Found : | C, 57.47; | H, 6.86; | N, 8.26 |

### Working Example 17

Using the compound of Working Example 13, the procedure of Working Example 16 was followed to afford the compounds shown in Table 52.

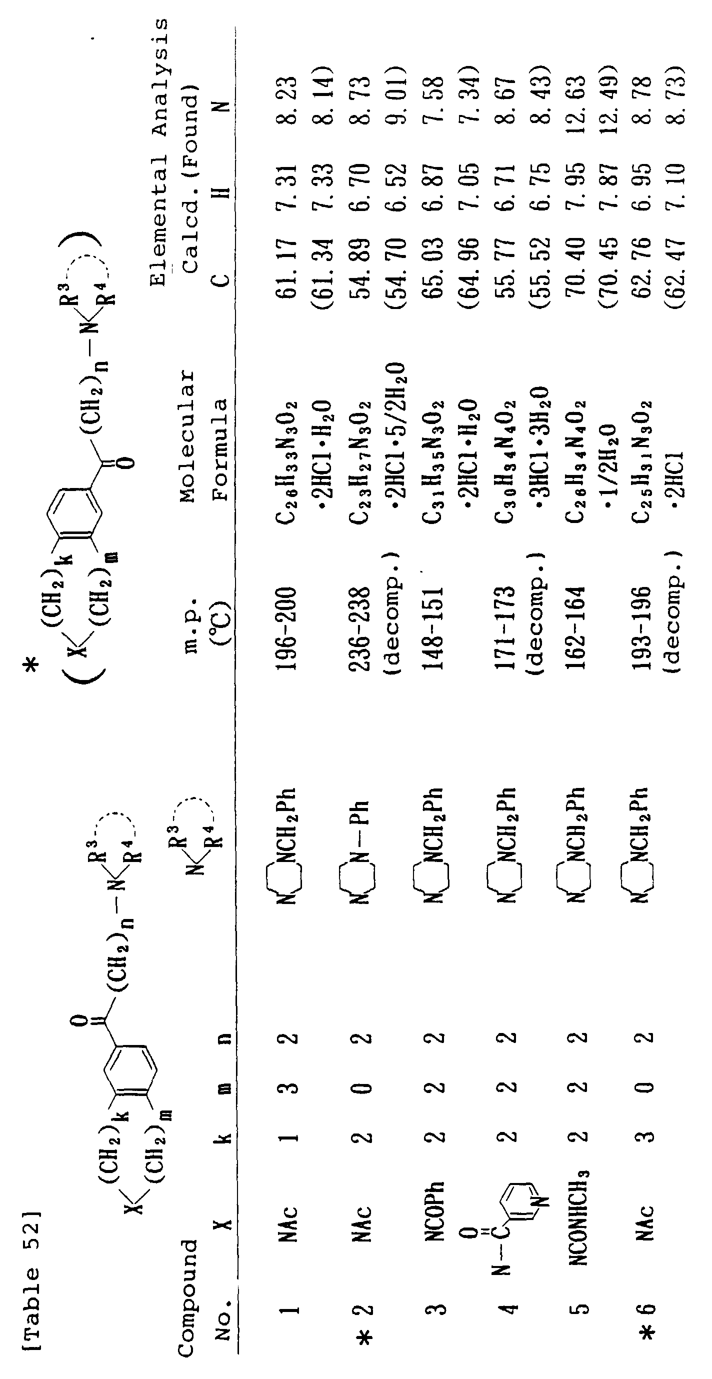

### Working Example 18

### 1-(3-Methyl-2,3,4,5-tetrahydro-1H-3-benzazepin-7-yl)-3-[4-(phenylmethyl)piperazin-1-yl]-1-propanone trihydrochloride

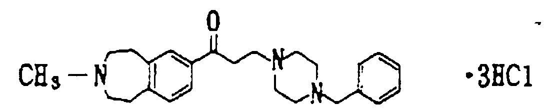

In 30 ml of ethanol was suspended 0.50 g of the compound obtained in Reference Example 6, to which were added 0.55 g of potassium carbonate and 0.42 ml of 1-benzyl piperazine at room temperature. The mixture was stirred for 3 hours, to which were added 50 ml of dichloromethane and 50 ml of pure water. The organic layer was separated and washed with 30 ml of pure water, which was then dried over anhydrous sodium sulfate. The solvent was distilled off under reduced pressure to leave 0.2 g of an oily residue. The oily residue was purified by means of an alumina chromatography (developing solvent: dichloromethane - ethyl acetate = 4:1(v/v)) to give 0.17 g an oily residue. To the oily residue was added 3 equivalents of methanolic hydrochloric acid, then methanol was distilled off to afford 0.2 g of the title compound as amorphous powder.

| Elemental Analysis for C₂₅H₃₃N₃O·2HCl·2H₂O: | | | |
|---|---|---|---|
| Calcd.: | C, 55.92; | H, 7.51; | N, 7.83 |
| Found : | C, 56.01; | H, 7.78; | N, 7.83 |

### Working Example 19

### 1-[3-(Phenylmethyl)-2,3,4,5-tetrahydro-1H-3-benzazepin-7-yl]-2-phenyl-4-(piperidin-1-yl)-1-butanone dihydrochloride

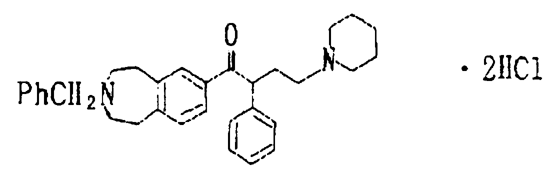

In 20 ml of dichloromethane was dissolved 0.92 g (2 mmol.) of the compound No.1 obtained in Reference Example 9. To the solution was added 0.99 ml (10 mmol.) of piperidine, and the mixture was stirred for one day at room temperature. The reaction mixture was washed with 20 ml of 1N aqueous solution of sodium hydroxide and 20 ml of pure water, followed by drying over sodium sulfate. The solvent was distilled off under reduced pressure. The residue was purified by means of a silica gel column chromatography [developing solvent: ethyl acetate - methanol (4:1)] to afford 0.53 g of the title compound in the free form as an oily substance.

To the oily substance was added 4N-methanolic hydrochloric acid (2 equivalents), then the solvent was distilled off under reduced pressure to give 0.58 g of the title compound as a hydroscopic amorphous powder.

| Elemental Analysis for C₃₂H₃₈N₂O·2HCl·H₂O: | | | |
|---|---|---|---|
| Calcd.: | C, 68.93; | H, 7.59; | N, 5.02 |
| Found : | C, 69.01; | H, 7.55; | N, 5.05 |

### Working Example 20

By substantially the same procedure as in Working Example 19, using the compounds obtained in Reference Example 8 or Reference Example 9, compounds shown in Table 53 were obtained.

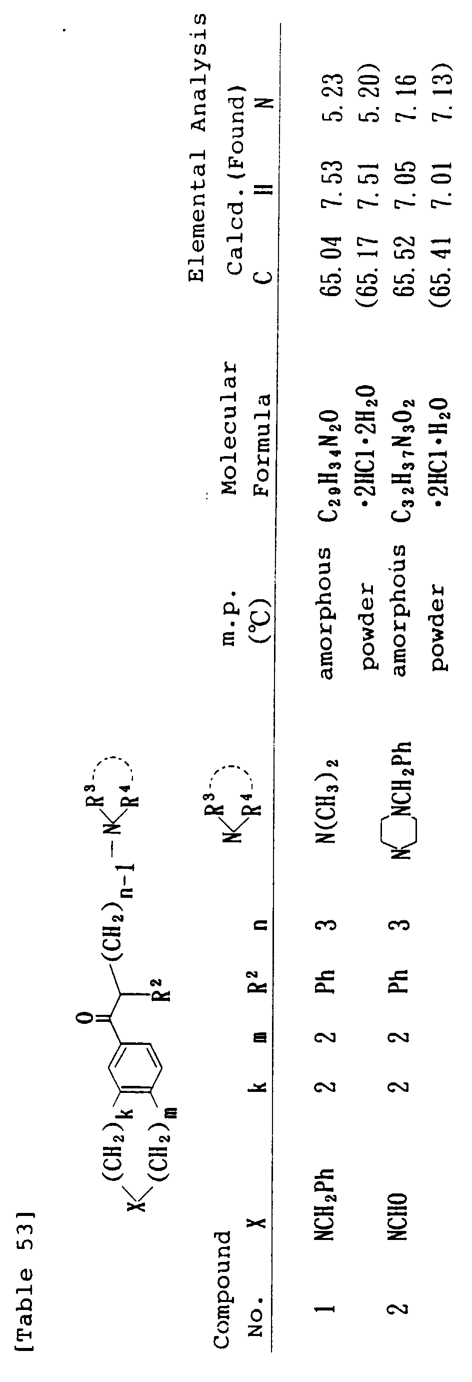

### Formulation Example 1

| | | |
|---|---|---|
| (1) | 3-[4-(Phenylmethyl)piperazin-1-yl]-1-[3-(phenylmethyl)-2,3,4,5-tetrahydro-1H-3-benzazepin-7-yl]-1-propanone trihydrochloride (Compound No.1 in [Table 17], Working Example 6) | 1 g |
| (2) | Lactose | 197 g |
| (3) | Corn starch | 50 g |
| (4) | Magnesium stearate | 2 g |

(1), (2) and 20 g of corn starch were blended and the mixture was granulated with a paste prepared from 15 g of corn starch and 25 ml of water. To this granular product were added 15 g of corn starch and (4), and the resulting composition was compression-molded to provide 2000 tablets each measuring 3 mm in diameter and containing 0.5 mg of (1).

### Formulation Example 2

| | | |
|---|---|---|
| (1) | 3-[4-(phenylmethyl)piperazin-1-yl]-1-[3-(phenylmethyl)-2,3,4,5-tetrahydro-1H-3-benzazepin-7-yl]-1-propanone trihydrochloride (Compound No.1 in [Table 17], Working Example 6) | 2 g |
| (2) | Lactose | 197 g |
| (3) | Corn starch | 50 g |
| (4) | Magnesium stearate | 2 g |

(1), (2) and 20 g of corn starch were blended and the mixture was granulated with a paste prepared from 15 g of corn starch and 25 ml of water. To this granular product were added 15 g of corn starch and (4), and the resulting composition was compression-molded to provide 2000 tablets each measuring 3 mm in diameter and containing 1.0 mg of (1).

### Experimental Example 1

The cholinesterase inhibitory activity of the compound of this invention was assayed with (acetyl-[³H])-acetylcholine. More specifically, using the S₁ fraction of a homogenate of male Wistar rat cerebral cortex as the cholinesterase source, (acetyl[³H])-acetylcholine as the substrate and the compound of this invention as the test sample were added. The mixture was incubated for 30 minutes, then the reaction was terminated, to which was added a toluene-based scintillant, then the mixture was shaken. The reaction product [³H]-acetic acid was transferred to the toluene layer, which was subjected to determination of cholinesterase activity by counting with a liquid scintillation counter.

The cholinesterase inhibitory activity of the test compound was expressed in 50% inhibitory concentration (IC₅₀). The cholinesterase activity of physostigmine was also determined by the same procedure. The results are shown in Table 54.

**Table 54**

| Compound (Working Example No.) | Acetylcholinesterase inhibitory activity I C₅₀ (µM) |
|---|---|
| 3-4 | 0.119 |
| 6-1 | 0.049 |
| 6-5 | 0.0526 |
| 10-18 | 0.0152 |
| 10-19 | 0.171 |
| 10-20 | 0.0152 |
| 10-22 | 0.0928 |
| 10-26 | 0.0526 |
| 10-27 | 0.169 |
| 10-30 | 0.0599 |
| 10-32 | 0.118 |
| 13-1 | 0.0493 |
| 13-3 | 0.0789 |
| 13-8 | 0.163 |
| 14-2 | 0.538 |
| 14-4 | 0.147 |
| 14-5 | 0.0188 |
| 14-6 | 0.113 |
| 14-10 | 0.167 |
| 14-14 | 0.0636 |
| 14-19 | 0.0339 |
| 14-20 | 0.0478 |
| 14-21 | 0.0226 |
| 14-22 | 0.0215 |
| 14-23 | 0.0661 |
| 14-25 | 0.0696 |
| 14-27 | 0.133 |
| 14-29 | 0.0257 |
| 14-31 | 0.033 |
| 14-33 | 0.0341 |
| 14-36 | 0.0168 |
| 14-40 | 0.0602 |
| 16 | 0.0251 |
| 17-3 | 0.0825 |
| 18 | 0.0338 |
| Physostigmine | 0.220 |

The results shown in Table 54 indicate that the compound of the present invention has superior cholinesterase inhibitory activity to physostigmine.

### Experimental Example 2

Effects of the compound of this invention on monoamine uptake were investigated using [³H]-norepinephirine(NE) and [³H]-serotonin (5-HT). Rats were sacrificed by decapitation. The cerebral cortex and hippocampus were removed and homogenized in 10-15 volumes (W/V) of an ice-cold medium containing 0.32 M sucrose. Crude synaptosomal preparations (P2) were isolated after differential centrifugation at 1000 x g for 10 min and 20,000 x g for 30 min at 4°C. Synaptosomal membranes were suspended in Krebs-Ringer bicarbonate (KRB) solution (116 mM NaCl, 4.8 mM KCl, 1.3 mM CaCl₂, 1.2 mM MgSO₄, 1.2 mM NaH₂PO₄, 25 mM NaHCO₃, 0.1 mM EDTA-2Na, 11.1 mM glucose, 0.11 mM ascorbic acid, 0.01 mM pargyline). Synaptosomal membrane suspension (900 µl) was preincubated with the test compound dissolved in DMSO solution at 37°C for 5 min. The reaction was initiated by addition of 100 µl of [³H]-NE(11 nM in final concentration) or [³H]-5-HT (10 nM in final concentration). Five minutes later, the reaction was stopped by the addition of 4 ml of ice-cold KRB and the reaction mixture was filtered through Whatman GF/B. Filters were washed twice with 4 ml of KRB and the radioactivity bound was counted with liquid scintillant. Imipramine and desipramine were used as positive control. All compounds were tested at 10⁻⁸, 10⁻⁷, 10⁻⁶ and 10⁻⁵M. The results are shown in Table 55.

**Table 55**

| Compound (Working Example No.) | Monoamine reuptake inhibitory activity IC₅₀(µM) | |
|---|---|---|
| | NE | 5-HT |
| 14-1 | 0. 147 | 0. 416 |
| 14-6 | 0. 725 | 0. 0345 |
| 14-7 | 0. 822 | 0. 0421 |
| 14-23 | 0. 912 | 0. 0583 |
| 14-29 | 0. 429 | 0. 0544 |
| 14-31 | 0. 441 | 0. 0305 |
| 14-33 | 0. 74 | 0. 0559 |
| 14-36 | 0. 70 | 0. 0133 |
| 14-40 | 0. 359 | 0. 0413 |
| Desipramine | 0. 15 | 0. 45 |
| Imipramine | 1. 12 | 0. 063 |

The results shown in Table 55 indicate that the compound of the present invention has superior inhibitory activity of monoamine uptake to reference compounds such as desipramine or imipramine.

### [Effects of Invention]

The compound of the present invention has excellent cholinesterase inhibitory activity and monoamine reuptake inhibitory activity and is useful as therapeutic/prophylactic medicament of senile dementia.

## Claims

1. A compound of the formula: 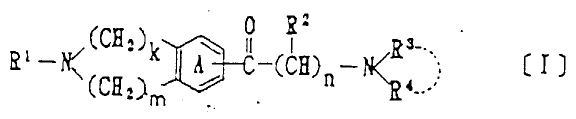 wherein R¹ is (1) a hydrogen atom, (2) a straight-chain or branched C₁₋₁₁ alkyl, C₂₋₄ alkenyl or C₂₋₄ alkynyl group which may be substituted by 1 to 5 substituents selected from the group consisting of a halogen, nitro, cyano, hydroxy, C₁₋₄, alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-membered heterocyclic group or its condensed ring group containing 1 to 3 hetero atoms selected from N, S and 0 which may be substituted with a C₁₋₄ alkyl , (3) a C₃₋₇ monocyclic cycloalkyl group which may be substituted by 1 to 5 substituents selected from the group consisting of a halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-membered heterocyclic group or its condensed ring group containing 1 to 3 hetero atoms selected from N, S and 0 which may be substituted with a C₁₋₄ alkyl, (4) a bicyclo[3.2. 1]oct-2-yl, bicyclo[3.2. 1]non-2-yl or adamantan-1-yl group which may be substituted by 1 to 5 substituents selected from the group consisting of a halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-membered heterocyclic group or its condensed ring group containing 1 to 3 hetero atoms selected from N, S and 0 which may be substituted with a C₁₋₄ alkyl, (5) a phenyl or naphthyl group which may be substituted by 1 to 5 substituents selected from the group consisting of a C₁₋₄ alkyl, halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₇₋₁₈ aralkyloxy, aminocarbonyloxy, mono-or di-C₁₋₄ alkyl-substituted aminocarbonyloxy, C₁₋₄ alkylsulfonylamino, C₁₋₄ alkoxy-carbonyl, carboxyl, C₁₋₆ alkyl-carbonyl, C₃₋₇ cycloalkyl-carbonyl, carbamoyl, mono- or di-C₁₋₄ alkyl-substituted carbamoyl, C₁₋₆ alkylsulfonyl, C₃₋₇ cycloalkylsulfonyl and a phenyl, naphthyl, mono- or di-phenyl-C₁₋₃ alkyl, phenoxy, benzoyl, phenoxycarbonyl, benzylcarbonyl, phenyl-C₁₋₄ alkyl-carbamoyl, phenylcarbamoyl, phenyl-C₁₋₄ alkylcarbonylamino, benzoylamino, phenyl-C₁₋₄, alkylsulfonyl, phenylsulfonyl, phenyl-C₁₋₄ alkylsulfinyl, phenyl-C₁₋₄ alkylsulfonylamino or phenylsulfonylamino group which may be substituted by 1 to 4 substituents selected from the group consisting of a C₁₋₄ alkyl, C₁₋₄ alkoxy, halogen, hydroxy, benzyloxy, amino, mono- or di-C₁₋₄ alkyl-substituted amino, nitro, C₁₋₆ alkyl-carbonyl and benzoyl, (6) a C₇₋₁₈ aralkyl, C₆₋₁₄ aryl-C₂₋₁₂ alkenyl, C₆₋₁₄ aryl-C₂₋₁₂ alkynyl or C₃₋₇ cycloalkyl-C₁₋₆ alkyl group which may be substituted by 1 to 5 substituents selected from the group consisting of a C₁₋₄ alkyl, halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₇₋₁₈ aralkyloxy, aminocarbonyloxy, mono-or di-C₁₋₄ alkyl-substituted aminocarbonyloxy, C₁₋₄ alkylsulfonylamino, C₁₋₄ alkoxy-carbonyl, carboxyl, C₁₋₆ alkyl-carbonyl, C₃₋₇ cycloalkyl-carbonyl, carbamoyl, mono- or di-C₁₋₄ alkyl-substituted carbamoyl, C₁₋₆ alkylsulfonyl, C₃₋₇ cycloalkylsulfonyl and a phenyl, naphthyl, mono- or di-phenyl-C₁₋₃ alkyl, phenoxy, benzoyl, phenoxycarbonyl, benzylcarbonyl, phenyl-C₁₋₄ alkyl-carbamoyl, phenylcarbamoyl, phenyl-C₁₋₄ alkyl-carbonylamino, benzoylamino, phenyl-C₁₋₄ alkylsulfonyl, phenylsulfonyl, phenyl-C₁₋₄ alkylsulfinyl, phenyl-C₁₋₄ alkylsulfonylamino or phenylsulfonylamino group which may be substituted by 1 to 4 substituents selected from the group consisting of a C₁₋₄ alkyl, C₁₋₄ alkoxy, halogen, hydroxy, benzyloxy, amino, mono- or di-C₁₋₄ alkyl-substituted amino, nitro, C₁₋₆ alkyl-carbonyl and benzoyl, (7) a C₁₋₈ alkyl-carbonyl or C₆₋₁₄ aryl-carbonyl group which may be substituted by 1 to 3 substituents selected from the group consisting of a halogen, amino, mono- or di-C₁₋₆ alkyl substituted amino and C₁₋₄ alkoxy, (8) a C₁₋₇ alkylsulfonyl or C₆₋₁₄ arylsulfonyl group which may be substituted by 1 to 3 substituents selected from the group consisting of a halogen, amino, mono- or di-C₁₋₆ alkyl substituted amino and C₁₋₄ alkoxy, (9) a C₁₋₇ alkylphosphonyl or C₆₋₁₄ arylphosphonyl group which may be substituted by 1 to 3 substituents selected from the group consisting of a halogen, amino, mono- or di-C₁₋₆ alkyl substituted amino and C₁₋₄ alkoxy, (10) a C₁₋₈ alkoxy-carbonyl or C₇₋₁₈ aralkyloxy-carbonyl group which may be substituted by 1 to 3 substituents selected from the group consisting of a halogen, amino, mono- or di-C₁₋₆ alkyl substituted amino and C₁₋₄ alkoxy, (11) heterocyclic-carbonyl group wherein the heterocyclic group is a 5- or 6-membered heterocyclic group containing 1 to 3 hetero atoms selected from N, and 0 which may be substituted by 1 to 3 substituents selected from the group consisting of a halogen, amino, mono- or di-C₁₋₆ alkyl substituted amino and C₁₋₄ alkoxy, (12) carbamoyl group which may be substituted by 1 to 3 substituents selected from the group consisting of a halogen, amino, mono- or di-C₁₋₆ alkyl substituted amino and C₁₋₄ alkoxy, (13) mono- or di-C₁₋₄ alkyl-carbamoyl group which may be substituted by 1 to 3 substituents selected from the group consisting of a halogen, amino, mono- or di-C₁₋₆ alkyl substituted amino and C₁₋₄ alkoxy, or (14) formyl; R² is (1') a hydrogen atom, (2') a straight-chain or branched C₁₋₁₁ alkyl, C₂₋₄ alkenyl or C₂₋₄ alkynyl group which may be substituted by 1 to 5 substituents selected from the group consisting of a halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-membered heterocyclic group or its condensed ring group containing 1 to 3 hetero atoms selected from N, S and 0 which may be substituted with a C₁₋₄ alkyl , (3') a C₃₋₇ monocyclic cycloalkyl group which may be substituted by 1 to 5 substituents selected from the group consisting of a halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-membered heterocyclic group or its condensed ring group containing 1 to 3 hetero atoms selected from N, S and 0 which may be substituted with a C₁₋₄ alkyl, (4') a bicyclo [3.2.1]oct-2-yl, bicyclo [3.2.1]non-2-yl or adamantan-1-yl group which may be substituted by 1 to 5 substituents selected from the group consisting of a halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-membered heterocyclic group or its condensed ring group containing 1 to 3 hetero atoms selected from N, S and 0 which may be substituted with a C₁₋₄ alkyl, (5') a phenyl or naphthyl group which may be substituted by 1 to 5 substituents selected from the group consisting of a C₁₋₄ alkyl, halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkylcarbonylamino, C₇₋₁₈ aralkyloxy, aminocarbonyloxy, mono-or di-C₁₋₄ alkyl-substituted aminocarbonyloxy, C₁₋₄ alkylsulfonylamino, C₁₋₄ alkoxy-carbonyl, carboxyl, C₁₋₆ alkyl-carbonyl, C₃₋₇ cycloalkyl-carbonyl, carbamoyl, mono- or di-C₁₋₄ alkyl-substituted carbamoyl, C₁₋₆ alkylsulfonyl, C₃₋₇ cycloalkylsulfonyl and a phenyl, naphthyl, mono- or di-phenyl-C₁₋₃ alkyl, phenoxy, benzoyl, phenoxycarbonyl, benzylcarbonyl, phenyl-C₁₋₄ alkyl-carbamoyl, phenylcarbamoyl, phenyl-C₁₋₄ alkylcarbonylamino, benzoylamino, phenyl-C₁₋₄ alkylsulfonyl, phenylsulfonyl, phenyl-C₁₋₄ alkylsulfinyl, phenyl-C₁₋₄ alkylsulfonylamino or phenylsulfonylamino group which may be substituted by 1 to 4 substituents selected from the group consisting of a C₁₋₄ alkyl, C₁₋₄ alkoxy, halogen, hydroxy, benzyloxy, amino, mono- or di-C₁₋₄ alkyl-substituted amino, nitro, C₁₋₆ alkyl-carbonyl and benzoyl, (6') a C₇₋₁₈ aralkyl, C₆₋₁₄ aryl- C₂₋₁₂ alkenyl, C₆₋ ₁₄ aryl-C₂₋₁₂ alkynyl or C₃₋₇ cycloalkyl-C₁₋₆ alkyl group which may be substituted by 1 to 5 substituents selected from the group consisting of a C₁₋₄ alkyl, halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkylcarbonylamino, C₇₋₁₈ aralkyloxy, aminocarbonyloxy, mono-or di-C₁₋₄ alkyl-substituted aminocarbonyloxy, C₁₋₄ alkylsulfonylamino, C₁₋₄ alkoxy-carbonyl, carboxyl, C₁₋₆ alkyl-carbonyl, C₃₋₇ cycloalkyl-carbonyl, carbamoyl, mono- or di-C₁₋₄ alkyl-substituted carbamoyl, C₁₋₆ alkylsulfonyl, C₃₋₇ cycloalkylsulfonyl and a phenyl, naphthyl, mono- or di-phenyl-C₁₋₃ alkyl, phenoxy, benzoyl, phenoxycarbonyl, benzylcarbonoyl, phenyl-C₁₋₄ alkyl-carbamoyl, phenylcarbamoyl, phenyl-C₁₋₄ alkyl-carbonylamino, benzoylamino, phenyl-C₁₋₄ alkylsulfonyl, phenylsulfonyl, phenyl-C₁₋₄ alkylsulfinyl, phenyl-C₁₋₄ alkylsulfonylamino or phenylsulfonylamino group which may be substituted by 1 to 4 substituents selected from the group consisting of a C₁₋₄ alkyl, C₁₋₄ alkoxy, halogen, hydroxy, benzyloxy, amino, mono- or di-C₁₋₄ alkyl-substituted amino, nitro, C₁₋₆ alkyl-carbonyl and benzoyl; one of R³ and R⁴ is H or a straight-chain or branched C₁₋₄ alkyl group and the other is a straight-chain or branched C₁₋₄ alkyl group, a phenyl-C₁₋₃ alkyl group or a naphthyl-C₁₋₃ alkyl group. R³ and R⁴ may, taken together with the adjacent nitrogen atom, form a 3- to 13-membered heterocyclic group having, other than carbon atoms and one nitrogen atom, optionally 1 to 3 nitrogen, oxygen and/or sulfur atoms as hetero atoms, which may be substituted by 1 to 5 substituents selected from the group consisting of (1") a straight-chain or branched C₁₋₁₁ alkyl, C₂₋₄ alkenyl or C₂₋₄, alkynyl group which may be substituted by 1 to 5 substituents selected from the group consisting of a halogen, nitro, cyano, hydroxy, C₁₋₄, alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-member heterocyclic group or its condensed ring group containing 1 to 3 hetero atoms selected from N, S, and 0 which may be substituted with a C₁₋₄ alkyl, (2") a C₃₋₇ monocyclic cycloalkyl group which may be substituted by 1 to 5 substituents selected from the group consisting of a halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-membered heterocyclic group or its condensed ring group containing 1 to 3 hetero atoms selected from N, S and 0 which may be substituted with a C₁₋₄ alkyl, (3") a bicyclo [3.2.1] oct-2-yl, bicyclo [3.2.1] non-2-yl or adamatan-1-yl group which may be substituted by 1 to 5 substituents selected from the group consisting of a halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₁₋₄ alkylsulfonylamino, C₁₋₄ alkoxy-carbonyl, carboxyl, C₁₋₆ alkyl-carbonyl, carbamoyl, mono- or di-C₁₋₄ alkyl-substituted carbamoyl, C₁₋₆ alkylsulfonyl, C₁₋₄ alkylenedioxy and 5- or 6-membered heterocyclic group or its condensed ring group containing 1 to 3 hetero atoms selected from N, S and 0 which may be substituted with a C₁₋₄ alkyl (4") a phenyl or naphthyl group which may be substituted by 1 to 5 substituents selected from the group consisting of a C₁₋₄ alkyl, halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₇₋₁₈ aralkyloxy, aminocarbonyloxy, mono-or di-C₁₋₄ alkyl-substituted aminocarbonyloxy, C₁₋₄ alkylsulfonylamino, C₁₋₄ alkoxy-carbonyl, carboxyl, C₁₋₆ alkyl-carbonyl, C₃₋₇ cycloalkyl-carbonyl, carbamoyl, mono- or di-C₁₋₄ alkyl-substituted carbamoyl, C₁₋₆ alkylsulfonyl, C₃₋₇ cycloalkylsulfonyl, C₁₋₄ alkylenedioxy and a phenyl, naphthyl, mono- or di-phenyl-C₁₋₃ alkyl, phenoxy, benzoyl, phenoxycarbonyl, benzylcarbonyl, phenyl-C₁₋₄ alkyl-carbamoyl, phenylcarbamoyl, phenyl-C₁₋₄ alkyl-carbonylamino, benzoylamino, phenyl-C₁₋₄ alkolsulfonyl, phenylsulfonyl, phenyl-C₁₋₄ alkylsulfinyl, phenyl-C₁₋₄ alkylsulfonylamino or phenylsulfonylamino group which may be substituted by 1 to 4 substituents selected from the group consisting of a C₁₋₄ alkyl, C₁₋₄ alkoxy, halogen, hydroxy, benzyloxy, amino, mono- or di-C₁₋₄ alkyl-substituted amino, nitro, C₁₋₆ alkyl-carbonyl and benzoyl, (5") a C₇₋₁₈ aralkyl, C₆₋₁₄ aryl-C₂₋₁₂ alkenyl, C₆₋₁₄ aryl-C₂₋₁₂ alkynyl or C₃₋₇ cycloalkyl-C₁₋₆ alkyl group which may be substituted by 1 to 5 substituents selected from the group consisting of a C₁₋₄ alkyl, halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₇₋₁₈ aralkyloxy, aminocarbonyloxy, mono-or di-C₁₋₄ alkyl-substituted aminocarbonyloxy, C₁₋₄ alkylsulfonylamino, C₁₋₄ alkoxy-carbonyl, carboxyl, C₁₋₆ alkyl-carbonyl, C₃₋₇ cycloalkyl-carbonyl, carbamoyl, mono- or di-C₁₋₄ alkyl-substituted carbamoyl, C₁₋₆ alkylsulfonyl, C₃₋₇ cycloalkylsulfonyl, C₁₋₄ alkylenedioxy and a phenyl, naphthyl, mono- or diphenyl-C₁₋₃ alkyl, phenoxy, benzoyl, phenoxycarbonyl, benzylcarbonyl, phenyl-C₁₋₄ alkyl-carbamoyl, phenylcarbamoyl, phenyl-C₁₋₄ alkyl-carbonylamino, benzoylamino, phenyl-C₁₋₄ alkylsulfonyl, phenylsulfonyl, phenyl-C₁₋₄ alkylsulfinyl, phenyl-C₁₋₄ alkylsulfonylamino or phenylsulfonylamino group which may be substituted by 1 to 4 substituents selected from the group consisting of a C₁₋₄ alkyl, C₁₋₄ alkoxy, halogen, hydroxy, benzyloxy, amino, mono- or di-C₁₋₄ alkyl-substituted amino, nitro, C₁₋₆ alkyl-carbonyl and benzoyl, (6") halogen atom, (7'') nitro group, (8") cyano group, (9") hydroxyl group, (10") C₁₋₄ alkoxy group, (11") C₁₋₄ alkylthio group, (12'') amino group, (13") mono or di C₁₋₄ alkylamino group, (14") C₁₋₄ alkyl-carbonylamino group, (15'') C₁₋₄ alkyl-sulfonylamino group, (16") C₁₋₄ alkoxy-carbonyl group, (17") carboxyl group, (18") formyl group, (19") C₁₋₆ alkyl-carbonyl group, (20") C₁₋₄ alkyl-carbonyloxy group, (21") ω-oxo-ω-(tetrahydrobenzazepinyl) C₁₋₆ alkyl group, (22") benzoyl group which may be substituted by 1 to 3 substituents selected from the group consisting of C₁₋₄ alkyl, halogen, C₁₋₄ alkoxy, mono- or di-C₁₋₄ alkylamino, 5- to 7-membered cyclic amino group, nitro and hydroxy, (23") carbamoyl group, (24") mono or di C₁₋₄ alkyl-carbamoyl group, (25") C₁₋₆ alkylsulfonyl group, (26") oxo group and (27") heterocyclic group selected from pyridinyl, pyrazinyl, pyrimidinyl, quinolinyl, isoquinolinyl, naphthyridinyl, benzothiazolyl, benzoxazolyl, furanyl and thienyl; ring A is a benzene ring which may be further substituted by 1 to 3 substituents selected from the group consisting of a C₁₋₄ alkyl, halogen, nitro, cyano, hydroxy, C₁₋₄ alkoxy, C₁₋₄ alkylthio, amino, mono- or di-C₁₋₄ alkyl-substituted amino, 5 to 7-membered cyclic amino, C₁₋₄ alkyl-carbonylamino, C₇₋₁₈ aralkyloxy, aminocarbonyloxy, mono-or di-C₁₋₄ alkyl-substituted aminocarbonyloxy, C₁₋₄ alkylsulfonylamino, C₁₋₄ alkoxy-carbonyl, carboxyl, C₁₋₆ alkyl-carbonyl, C₃₋₇ cycloalkyl-carbonyl, carbamoyl, mono- or di-C₁₋₄ alkyl-substituted carbamoyl, C₁₋₆ alkylsulfonyl, C₃₋₇ cycloalkylsulfonyl and a phenyl, naphthyl, mono- or di-phenyl-C₁₋₃ alkyl, phenoxy, benzoyl, phenoxycarbonyl, benzylcarbonyl, phenyl-C₁₋₄ alkyl-carbamoyl, phenylcarbamoyl, phenyl-C₁₋₄ alkyl-carbonylamino, benzoylamino, phenyl-C₁₋₄ alkylsulfonyl, phenylsulfonyl, phenyl-C₁₋₄ alkylsulfinyl, phenyl-C₁₋₄ alkylsulfonylamino or phenylsulfonylamino which may be substituted by 1 to 4 substituents selected from the group consisting of a C₁₋₄ alkyl, C₁₋₄ alkoxy, halogen, hydroxy, benzyloxy, amino, mono- or di-C₁₋₄ alkyl-substituted amino, nitro, C₁₋₆ alkyl-carbonyl and benzoyl; R²'s may be different from one another in the repetition of n; k is a whole number of 0 to 3; and m is a whole-number of 1 to 8; n is a whole number of 1 to 10; provided that when k=0 and m=2, n is a whole number of not less than 2, or a pharmaceutically acceptable salt thereof.

2. A compound as claimed in claim 1, wherein R¹ is
(i) hydrogen atom,
(ii) a C₁₋₄ alkyl group which may be substituted with a hydroxy group,
(iii) a C₂₋₄ alkynyl group,
(iv) a phenyl-C₁₋₃ alkyl group which may be substituted with one to three substituents selected from the group consisting of a halogen, nitro, cyano, C₁₋₄ alkyl, C₁₋₄ alkoxy, hydroxy and phenylmethoxy group,
(v) formyl group,
(vi) a C₁₋₄ alkyl-carbonyl group,
(vii) benzoyl group,
(viii) a C₁₋₄ alkoxy-carbonyl group,
(ix) pyridylcarbonyl group, or
(x) a mono- or di-C₁₋₄ alkylcarbamoyl group.

3. A compound as claimed in claim 1, wherein R¹ is (i) H, (ii) a straight-chain or branched C₁₋₄ alkyl group, (iii) a phenyl-C₁₋₃ alkyl group which may be substituted by 1 to 3 substituents selected from the group consisting of C₁₋₄ alkyl, halogen, nitro, cyano, hydroxy, C₁₋₄ alkoxy and C₇₋₁₈ aralkyloxy, (iv) a naphthyl-C₁₋₃ alkyl group, (v) a C₁₋₃ alkyl-carbonyl, (vi) a phenyloxycarbonyl or (vii) a C₁₋₄ alkoxy-carbonyl.

4. A compound as claimed in claim 1, wherein R¹ is (i) H, (ii) a straight-chain or branched C₁₋₄ alkyl group, (iii) a C₁₋₄ alkyl-carbonyl group or (iv) a phenyl-C₁₋₃ alkyl group which may be substituted by a C₁₋₄ alkoxy.

5. A compound as claimed in claim 1, wherein k + m is a whole number of 2 to 6.

6. A compound as claimed in claim 1, wherein k is a whole number of 0 to 2 and m is a whole number of 2 to 5.

7. A compound as claimed in claim 1, wherein the moiety in the formula [I] of 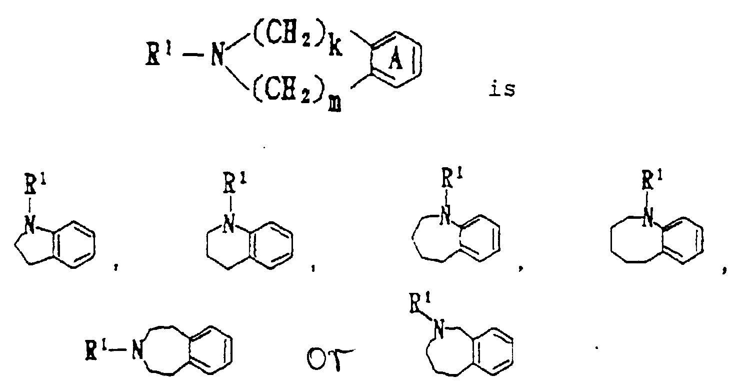 and R¹ is (i) H, (ii) a straight-chain or branched C₁₋₄ alkyl group, (iii) a C₁₋₃ alkyl-carbonyl group or (iv) a phenyl-C₁₋₃ alkyl group which may be substituted by a C₁₋₄ alkoxy.

8. A compound as claimed in claim 1, wherein the ring A is a benzene ring.

9. A compound as claimed in claim 1, wherein n is a whole number of 1 to 6.

10. A compound as claimed in claim 1, wherein R² is hydrogen atom or phenyl group.

11. A compound as claimed in claim 1, wherein R² is H.

12. A compound as claimed in claim 1, wherein R³ and R⁴ are independently
(i) a C₁₋₄ alkyl group which may be substituted with a hydroxy group,
(ii) phenyl group or
(iii) a phenyl-C₁₋₃ alkyl group which may be substituted with a halogen atom, or R³ and R⁴, taken together with the adjacent nitrogen atom, form
(i') a piperidinyl group which may be substituted with a phenyl-C₁₋₃ alkyl group, a hydroxy group or oxo,
(ii') 4-oxo-1-phenyl-1,3,8-triazaspiro[4,5]decan-8-yl,
(iii') 1,2,3,4-tetrahydroisoquinolin-2-yl,
(iv') pyrrolidinyl,
(v') morpholinyl,
(vi') a homopiperazinyl group which may be substituted with a phenyl-C₁₋₃ alkyl group, or
(vii') a piperazinyl group which may be substituted with (1) a phenyl-C₁₋₃ alkyl group which may be substituted with a halogen atom, a C₁₋₄ alkoxy group or a C₁₋₄ alkylenedioxy group, (2) pyridyl group, (3) a benzoyl group which may be substituted with a halogen atom, (4) a C₁₋₄ alkyl group which may be substituted with hydroxy group, 3-oxo-3[3-(phenylmethyl)-2,3,4,5-tetrahydro-1H-3-benzazepin-7-yl]propyl, pyridyl, furyl or 2-methyl-thiazol-4-yl group, (5) formyl group, (6) a C₁₋₆ alkyl-carbonyl group, (7) a phenyl group which may be substituted with a halogen atom, (8) hydroxyl group or (9) a diphenyl-C₁₋₃ alkyl group.

13. A compound as claimed in claim 1, wherein one of R³ and R⁴ is H or a straight-chain or branched C₁₋₄ alkyl group and the other is a straight-chain or branched C₁₋₄ alkyl group, a phenyl-C₁₋₃ alkyl group or a naphthyl-C₁₋₃ alkyl group.

14. A compound as claimed in claim 1, wherein R³ and R⁴ taken together with the adjacent nitrogen atom, may form a pyrrolidinyl, piperidinyl, piperazinyl, morpholinyl or 1,2,3,4-tetrahydroquinolinyl group which may be substituted by 1 or 2 substituents selected from the group consisting of (1) formyl, (2) C₁₋₄ alkyl-carbonyl, (3) hydroxy, (4) oxo, (5) pyridyl, (6) benzoyl which may be substituted by 1 to 3 halogen atoms, (7) straight-chain or branched C₁₋₇ alkyl which may be substituted by 1 to 3 substituents selected from the group consisting of hydroxy, pyridyl, furyl, thiazol-4-yl and 2-methyl-thiazol-4-yl, (8) phenyl which may be substituted by 1 to 3 substituents selected from the group consisting of halogen, hydroxy and C₁₋₄ alkylenedioxy, (9) C₇₋₁₈ aralkyl which may be substituted by 1 to 3 substituents selected from the group consisting of halogen, hydroxy and C₁₋₄ alkylenedioxy and (10) ω-oxo-ω-(tetrahydrobenzazepinyl) C₁₋₆ alkyl.

15. A compound as claimed in claim 1, wherein R³ and R⁴ taken together with the adjacent nitrogen atom, may form a 4-(phenylmethyl)-piperazin-1-yl or 4-[(2-methylthiazol-4-yl)methyl]-piperazin-1-yl.

16. A compound as claimed in claim 1, wherein n is a whole number of 3 to 8 when the group of the formula: 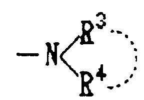 does not form a heterocyclic group and n is a whole number of 2 to 5 when the group forms a heterocyclic group.

17. A compound as claimed in claim 1, which is a compound of the formula: 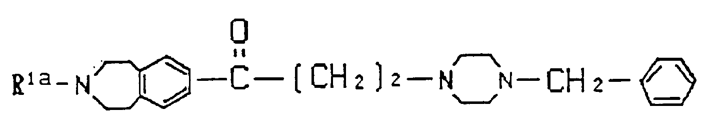 wherein R^{1a} is
(i) a C₁₋₄ alkyl group or
(ii) a phenyl-C₁₋₃ alkyl group which may be substituted with a C₁₋₄ alkoxy group.
or a pharmaceutically acceptable salt.

18. A compound as claimed in claim 1, which is 1-[3-[(4-methoxyphenyl)methyl]-2,3,4,5-tetrahydro-1H-3-benzazepin-7-yl]-3-[4-(phenylmethyl)piperazin-1-yl]-1-propanone or a pharmaceutically acceptable salt thereof.

19. A compound as claimed in claim 1, which is 1-[3-(2-phenylethyl)-2,3,4,5-tetrahydro-1H-3-benzazepin-7-yl]-3-[4-(phenylmethyl)piperazin-1-yl]-1-propanone or a pharmaceutically acceptable salt thereof.

20. A compound as claimed in claim 1, which is 1-[3-(2-methylpropyl)-2,3,4,5-tetrahydro-1H-3-benzazepin-7-yl]-3-[4-(phenylmethyl)piperazin-1-yl]-1-propanone or a pharmaceutically acceptable salt thereof.

21. A compound as claimed in claim 1, which is 1-[3-(phenylmethyl)-2,3,4,5-tetrahydro-1H-3-benzazepin-7-yl]-3-[4-(phenylmethyl)piperazin-1-yl]-1-propanone or a pharmaceutically acceptable salt thereof.

22. A compound as claimed in claim 1, which is 1-[1-(phenylmethyl)-2,3-dihydro-1H-indol-5-yl]-3-[4-(phenylmethyl)piperazin-1-yl]-1-gropanone or a pharmaceutically acceptable salt thereof.

23. A compound as claimed in claim 1, which is 1-[2-(phenylmethyl)-2,3,4,5-tetrahydro-1H-2-benzazepin-8-yl]-3-[4-(phenylmethyl)piperazin-1-yl]-1-propanone or a pharmaceutically acceptable salt thereof.

24. A compound as claimed in claim 1, which is 3-[4-[(2-methylthiazol-4-yl)methyl]piperazin-1-yl]-1-[3-(phenylmethyl)-2,3,4,5-tetrahydro-1H-3-benzazepin-7-yl]-1-propanone or a pharmaceutically acceptable salt thereof.

25. A compound of the formula: 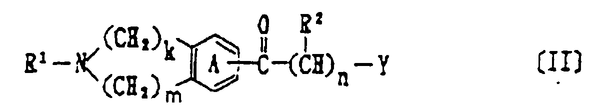 wherein Y is halogen, C₁₋₆ alkylsulfonyloxy or C₆₋₁₀ arylsulfonyloxy; R¹,ring A, R², n, k and m are of the same meaning as defined in Claim 1 or a salt thereof; provided that when k = 0 and m = 2 or 3, n denotes an integer of not less than 2.

26. A method for producing a compound of the formula: 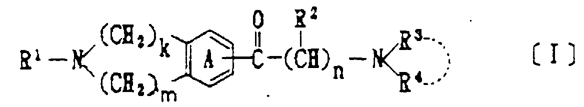 wherein R¹, ring A, R², R³, R⁴, k, m and n are as defined in claim 1 or a pharmaceutically acceptable salt thereof, which comprises reacting a compound of the formula: 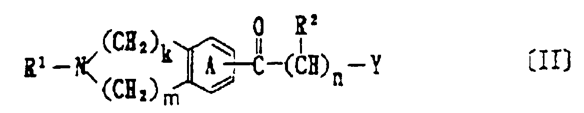 wherein Y is a leaving group; R¹, ring A, R², n, k and m are as defined in claim 1, or a salt thereof, with a compound of the formula: 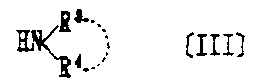 wherein R³ and R⁴ are as defined above, or a salt thereof.

27. A method for producing a compound of the formula: 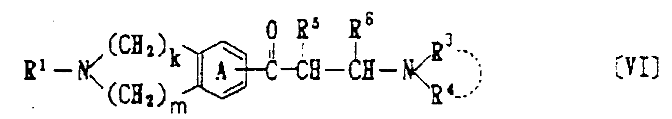 wherein R¹, R³, R⁴, ring A, k, and m are as defined in claim 1 and R⁵ and R⁶ are defined as R² in claim 1 or a pharmaceutically acceptable salt thereof, which comprises reacting a compound of the formula: 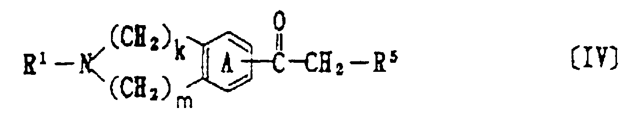 wherein R¹, R⁵, ring A, k and m are as defined above, or a salt thereof and a compound of the formula:
R⁶-CHO [V]
wherein R⁶ is as defined above, with a compound of the formula: 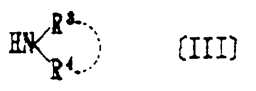 wherein R³ and R⁴ are as defined above, or a salt thereof.

28. A cholinesterase inhibitor, which contains a compound of the formula: 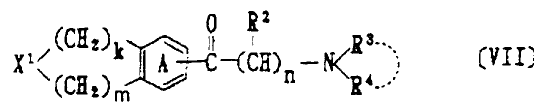 wherein R¹, R², R³, R⁴, ring A, k, m and n are as defined in claim 1, and wherein X¹ is R¹-N, O or S; or a pharmaceutically acceptable salt thereof.

29. A pharmaceutical composition for a disease caused by acetylcholinesterase activity which contains an effective cholinesterase inhibiting amount of a compound of the formula [I] as claimed in claim 1 or a pharmaceutically acceptable salt thereof and a pharmacologically acceptable carrier.

30. A pharmaceutical composition for a disease caused by acetylcholinesterase activity which contains an effective cholinesterase inhibiting amount of a compound of the formula [VII] as claimed in claim 28 or a pharmaceutically acceptable salt thereof and a pharmacologically acceptable carrier.

31. A pharmaceutical composition as claimed in claimed 29 , in which the disease is senile dementia and/or Alzheimer's disease.

32. A pharmaceutical composition as claimed in claim 30 , in which the disease is senile dementia and/or Alzheimer's disease.

33. Use of a compound of the formula: 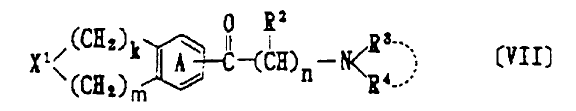 wherein R¹, R², R³, R⁴, ring A, k, m and n are as defined in claim 1, and wherein X¹ is R¹-N, O or S;
or a pharmaceutically acceptable salt thereof as a component in the preparation of a cholinesterase inhibitor.

## Patentansprüche

1. Verbindung der Formel 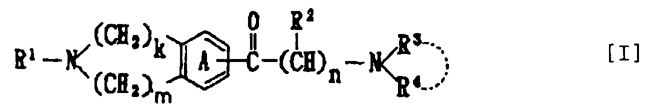 worin R¹ (1) ein Wasserstoffatom, (2) eine geradkettige oder verzweigte C₁₋₁₁-Alkyl, C₂₋₄-Alkenyl- oder C₂₋₄-Alkinylgruppe, die durch 1 bis 5 Substituenten substituiert sein kann, die aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋4-Alkylcarbonylamino, C₁₋₄-Alkylsulfonylamino, C₁₋₄-Alkoxycarbonyl, Carboxyl, C₁₋₆-Alkylcarbonyl, Carbamoyl, mono- oder di-C₁₋₄-alkylsubstituiertem Carbamoyl, C₁₋₆-Alkylsulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alkyl substituiert sein kann, (3) eine monocyclische C₃₋₇-Cycloalkylgruppe, die durch 1 bis 5 Substituenten substituiert sein kann, welche aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5-bis 7-gliedrigem cyclischem Amino, C₁₋₄-Alkylcarbonylamino, C₁₋₄-Alkylsulfonylamino, C₁₋₄-Alkoxycarbonyl, Carboxyl, C₁₋₆-Alkycarbonyl, Carbamoyl, mono- oder di-C₁₋₄-alkylsubstituiertem Carbamoyl, C₁₋₆-Alkylsulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alkyl substituiert sein kann, (4) eine Bicyclo[3.2.1]oct-2-yl-, Bicyclo[3.2.1]non-2-yl- oder Adamant-1-yl-gruppe, die mit 1 bis 5 Substituenten substituiert sein kann, welche aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₁₋₄-Alkylsulfonylamino, C₁₋₄-Alkoxycarbonyl, Carboxyl, C₁₋₆-Alkylcarbonyl, Carbamoyl, mono- oder di-C₁₋₄-alkylsubstituiertem Carbamoyl, C₁₋₆-Alkysulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alky substituiert sein kann, (5) eine Phenyl- oder Naphthylgruppe, die durch 1 bis 5 Substituenten substituiert sein kann, welche aus der Gruppe ausgewählt sind, die aus C₁₋₄-Alkyl, Halogen, Nitro, Cyano, Hydroxy, C₁₋₄-Alkoxy, C₁₋₄-Alkylthio, Amino, mono- oder di-C₁₋₄-alkyl-substituiertem Amino, 5- bis 7-gliedrigem cyclischem Amino, C₁₋₄-Alkylcarbonylamino, C₇₋₁₈-Aralkyloxy, Aminocarbonyloxy, mono- oder di-C₁₋₄-alkylsubstituiertem Aminocarbonyloxy, C₁₋₄-Alkylsulfonylamino, C₁₋₄-Alkoxycarbonyl, Carboxyl, C₁₋₆-Alkylcarbonyl, C₃₋₇-Cycloalkylcarbonyl, Carbamoyl, mono- oder di-C₁₋₄-alkylsubstituiertem Carbamoyl, C₁₋₆-Alkylsulfonyl, C₃₋₇-Cycloalkylsulfonyl und einer Phenyl-, Naphthyl-, Mono- oder Diphenyl -C₁₋₃-alkyl-, Phenoxy-, Benzoyl-, Phenoxycarbonyl-, Benzylcarbonyl-, Phenyl-C₁₋₄-alkylcarbamoyl-, Phenylcarbamoyl-, Phenyl-C₁₋₄-alkylcarbonylamino-, Benzoylamino-, Phenyl-C₁₋₄-alkylsulfonyl-, Phenylsulfonyl-, Phenyl-C₁₋₄-alkylsulfinyl-, Phenyl-C₁₋₄-alkylsulfonylamino- oder Phenylsulfonylaminogruppe besteht, die durch 1 bis 4 Substituenten substituiert sein kann, welche aus der Gruppe ausgewählt sind, die aus C₁₋₄-Alkyl, C₁₋₄-Alkoxy, Halogen, Hydroxy, Benzyloxy, Amino, mono- oder di-C₁₋₄-alkylsubstituiertem Amino, Nitro, C₁₋₆-Alkylcarbonyl und Benzoyl besteht, (6) eine C₇₋₁₈-Aralkyl-, C₆₋₁₄-Aryl-C₂₋₁₂-alkenyl-, C₆₋₁₄-Aryl-C₂₋₁₂-Alkinyl- oder C₃₋₇-Cycloalkyl-C₁₋₆-alkylgruppe, die durch 1 bis 5 Substituenten substituiert sein kann, welche aus der Gruppe ausgewählt sind, die aus C₁₋₄-Alkyl, Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₇₋₁₈-Aralkyloxy, Aminocarbonyloxy, mono- oder di-C₁₋₄-alkylsubstituiertem Aminocarbonyloxy, C₁₋₄-Alkylsulfonylamino, C₁₋₄-Alkoxycarbonyl, Carboxyl, C₁₋₆-Alkylcarbonyl, C₃₋₇-Cycloalkylcarbonyl, Carbamoyl, mono- oder di-C₁₋₄-alkylsubstituiertem Carbamoyl, C₁₋₆-Alkylsulfonyl, C₃₋₇-Cycloalkylsulfonyl und einer Phenyl-, Naphthyl-, Mono- oder Diphenyl-C₁₋₃-alkyl-, Phenoxy-, Benzoyl-, Phenoxycarbonyl-, Benzylcarbonyl-, Phenyl-C₁₋₄-alkylcarbamoyl-, Phenylcarbamoyl-, Phenyl-C₁₋₄-alkylcarbonylamino-, Benzoylamino-, Phenyl-C₁₋₄-alkylsulfonyl-, Phenylsulfonyl-, Phenyl-C₁₋₄-alkylsulfinyl-, Phenyl-C₁₋₄-alkylsulfonylamino- oder Phenylsulfonylaminogruppe besteht, die durch 1 bis 4 Substituenten substituiert sein kann, welche aus der Gruppe ausgewählt sind, die aus C₁₋₄-Alkyl, C₁₋₄-Alkoxy, Halogen, Hydroxy, Benzyloxy, Amino, mono- oder di-C₁₋₄-alkylsubstituiertem Amino, Nitro, C₁₋₆-Alkylcarbonyl und Benzoyl besteht, (7) eine C₁₋₈-Alkylcarbonyl- oder C₆₋₁₄-Arylcarbonylgruppe, die durch 1 bis 3 Substituenten substituiert sein kann, welche aus der Gruppe ausgewählt sind, die aus Halogen, Amino, mono- oder di-C₁₋₆-alkylsubstituiertem Amino und C₁₋₄-Alkoxy besteht, (8) eine C₁₋₇-Alkylsufonyl- oder C₆₋₁₄-Arylsulfonylgruppe, die durch 1 bis 3 Substituenten substituiert sein kann, welche aus der Gruppe ausgewählt sind, die aus Halogen, Amino, mono- oder di-C₁₋₆-alkyl-substituiertem Amino und C₁₋₄-Alkoxy besteht, (9) eine C₁₋₇-Alkylphosphonyl- oder C₆₋₁₄-Arylphosphonylgruppe, die durch 1 bis 3 Substituenten substituiert sein kann, welche aus der Gruppe ausgewählt sind, die aus Halogen, Amino, mono- oder di-C₁₋₆-alkylsubstituiertem Amino und C₁₋₄-Alkoxy besteht, (10) eine C₁₋₈-Alkoxycarbonyl- oder C₇₋ ₁₈-Aralkyloxycarbonylgruppe, die durch 1 bis 3 Substituenten substituiert sein kann, welche aus der Gruppe ausgewählt sind, die aus Halogen, Amino, mono- oder di-C₁₋₆-alkylsubstituiertem Amino und C₁₋ ₄-Alkoxy besteht, (11) Heterocyclocarbonylgruppe, worin die heterocyclische Gruppe eine 5- oder 6-gliedrige heterocyclische Gruppe ist, die 1 bis 3 aus N, S und O ausgewählte Heteroatome enthält und durch 1 bis 3 Substituenten substituiert sein kann, welche aus der Gruppe ausgewählt sind, die aus Halogen, Amino, mono- oder di-C₁₋₆-alkylsubstituiertem Amino und C₁₋₄-Alkoxy besteht, (12) Carbamoylgruppe, die durch 1 bis 3 Substituenten substituiert sein kann, welche aus der Gruppe ausgewählt sind, die aus Halogen, Amino, mono- oder di-C₁₋₆-alkylsubstituiertem Amino und C₁₋₄-Alkoxy besteht, (13) Mono- oder Di-C₁₋₄-alkylcarbamoylgruppe, die durch 1 bis 3 Substituenten substituiert sein kann, welche aus der Gruppe ausgewählt sind, die aus Halogen, Amino, mono- oder di-C₁₋₆-alkylsubstituiertem Amino und C₁₋₄-Alkoxy besteht, oder (14) Formyl ist;
R² (1') ein Wasserstoffatom, (2') eine geradkettige oder verzweigte C₁₋₁₁-Alkyl-, C₂₋₄-Alkenyl- oder C₂₋₄-Alkinylgruppe, die durch 1 bis 5 Substituenten substituiert sein kann, die aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5-bis 7-gliedrigem cyclischem Amino, C₁₋₄-Alkylcarbonylamino, C₁₋₄-Alkylsulfonylamino, C₁₋₄-Alkoxycarbonyl, Carboxyl, C₁₋₆-Alkycarbonyl, Carbamoyl, mono- oder di-C₁₋₄-alkylsubstituiertem Carbamoyl, C₁₋₆-Alkylsulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alkyl substituiert sein kann, (3' ) eine monocyclische C₃₋₇-Cycloalkylgruppe, die durch 1 bis 5 Substituenten substituiert sein kann, welche aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₁₋₄-Alkylsulfonylamino, C₁₋₄-Alkoxycarbonyl, Carboxyl, C₁₋₆-Alkylcarbonyl, Carbamoyl, mono-oder di-C₁₋₄-alkylsubstituiertem Carbamoyl, C₁₋₆-Alky-sulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alkyl substituiert sein kann, (4') eine Bicyclo[3.2.1]oct-2-yl-, Bicyclo[3.2.1]non-2-yl- oder Adamant-1-ylgruppe, die mit 1 bis 5 Substituenten substituiert sein kann, welche aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5-bis 7-gliedrigem cyclischem Amino, C₁₋₄-Alkylcarbonylamino, C₁₋₄-Alkylsulfonylamino, C₁₋₄-Alkoxycarbonyl, Carboxyl, C₁₋₆-Alkylcarbonyl, Carbamoyl, mono- oder di-C₁₋₄-alkylsubstituiertem Carbamoyl, C₁₋₆-Alkylsulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alkyl substituiert sein kann, (5') eine Phenyl- oder Naphthylgruppe, die durch 1 bis 5 Substituenten substituiert sein kann, welche aus der Gruppe ausgewählt sind, die aus C₁₋₄-Alkyl, Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₇₋₁₈-Aralkyloxy, Aminocarbonyloxy, mono- oder di-C₁₋₄-alkylsubstituiertem Aminocarbonyloxy, C₁₋₄-Alkylsulfonylamino, C₁₋₄-Alkoxycarbonyl, Carboxyl, C₁₋₆-Alkylcarbonyl, C₃₋₇-Cycloalkylcarbonyl, Carbamoyl, mono- oder di-C₁₋₄-alkylsubstituiertem Carbamoyl, C₁₋₆-Alkylsulfonyl, C₃₋₇-Cycloalkylsulfonyl und einer Phenyl-, Naphthyl-, Mono- oder Diphenyl-C₁₋₃-alkyl-, Phenoxy-, Benzoyl-, Phenoxycarbonyl-, Benzylcarbonyl-, Phenyl-C₁₋₄-alkylcarbamoyl-, Phenylcarbamoyl-, Phenyl-C₁₋₄-alkylcarbonylamino-, Benzoylamino-, Phenyl-C₁₋₄-alkylsulfonyl-, Phenylsulfonyl-, Phenyl-C₁₋₄-alkylsulfinyl-, Phenyl-C₁₋₄-alkylsulfonylamino- oder Phenylsulfonylaminogruppe besteht, die durch 1 bis 4 Substituenten substituiert sein kann, welche aus der Gruppe ausgewählt sind, die aus C₁₋₄-Alkyl, C₁₋₄-Alkoxy, Halogen, Hydroxy, Benzyloxy, Amino, mono- oder di-C₁₋₄-alkylsubstituiertem Amino, Nitro, C₁₋₆-Alkylcarbonyl und Benzoyl besteht, (6') eine C₇₋₁₈-Aralkyl-, C₆₋₁₄-Aryl-C₂₋₁₂-alkenyl-, C₆₋₁₄-Aryl-C₂₋₁₂-alkinyl- oder C₃₋₇-Cycloalkyl-C₁₋₆-alkylgruppe, die durch 1 bis 5 Substituenten substituiert sein kann, die aus der Gruppe ausgewählt sind, die aus C₁₋₄-Alkyl, Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₇₋₁₈-Aralkyloxy, Aminocarbonyloxy, mono- oder di-C₁₋₄-alkylsubstituiertem Aminocarbonyloxy, C₁₋₄-Alkylsulfonylamino, C₁₋₄-Alkoxycarbonyl, Carboxyl, C₁₋₆-Alkylcarbonyl, C₃₋₇-Cycloalkylcarbonyl, Carbamoyl, mono- oder di-C₁₋₄-alkylsubstituiertem Carbamoyl, C₁₋₆-Alkylsulfonyl, C₃₋₇-Cycloalkylsulfonyl und einer Phenyl-, Naphthyl-, Mono- oder Diphenyl-C₁₋₃-alkyl-, Phenoxy-, Benzoyl-, Phenoxycarbonyl-, Benzylcarbonyl-, Phenyl-C₁₋₄-alkylcarbamoyl-, Phenylcarbamoyl-, Phenyl-C₁₋₄-alkylcarbonylamino-, Benzoylamino-, Phenyl-C₁₋₄-alkylsulfonyl-, Phenylsulfonyl-, Phenyl-C₁₋₄-alkylsulfinyl-, Phenyl-C₁₋₄-alkylsulfonylamino- oder Phenylsulfonylaminogruppe besteht, die durch 1 bis 4 Substituenten substituiert sein kann, welche aus der Gruppe ausgewählt sind, die aus C₁₋₄-Alkyl, C₁₋₄-Alkoxy, Halogen, Hydroxy, Benzyloxy, Amino, mono- oder di-C₁₋₄-alkylsubstituiertem Amino, Nitro, C₁₋₆-Alkylcarbonyl und Benzoyl besteht; eines von R³ und R⁴ H oder eine geradkettige oder verzweigte C₁₋₄-Alkylgruppe ist und das andere eine geradkettige oder verzweigte C₁₋₄-Alkylgruppe, eine Phenyl-C₁₋₃-alkylgruppe oder eine Naphthyl-C₁₋₃-alkylgruppe ist; R³ und R⁴ mit dem benachbarten Stickstoff zusammengenommen eine 3- bis 13-gliedrige heterocyclische Gruppe mit außer Kohlenstoffatomen und einem Stickstoffatom gegebenenfalls 1 bis 3 Stickstoff-, Sauerstoff- und/oder Schwefelatomen als Heteroatomen bilden, welche durch 1 bis 5 Substituenten substituiert sein kann, die aus der Gruppe ausgewählt sind, die aus (1'') einer geradkettigen oder verzweigten C₁₋₁₁-Alkyl-, C₂₋₄-Alkenyl- oder C₂₋₄-Alkinylgruppe, die durch 1 bis 5 Substituenten substituiert sein kann, die aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₁₋₄-Alkylsulfonylamino, C₁₋₄-Alkoxycarbonyl, Carboxyl, C₁₋₆-Alkylcarbonyl, Carbamoyl, mono- oder di-C₁₋₄-alkylsubstituiertem Carbamoyl, C₁₋₆-Alkylsulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alkysubstituiert sein kann, (2'') einer monocyclischen C₃₋₇-Cycloalkylgruppe, die durch 1 bis 5 Substituenten substituiert sein kann, welche aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₁₋₄-Alkylsulfonylamino, C₁₋₄-Alkoxycarbonyl, Carboxyl, C₁₋₆-Alkylcarbonyl, Carbamoyl, mono- oder di-C₁₋₄-alkylsubstituiertem Carbamoyl, C₁₋₆-Alkylsulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alkyl substituiert sein kann, (3") einer Bicyclo[3.2.1]oct-2-yl-, Bicyclo[3.2.1]non-2-yl- oder Adamant-1-ylgruppe, die mit 1 bis 5 Substituenten substituiert sein kann, welche aus der Gruppe ausgewählt sind, die aus Halogen, Nitro, Cyano, Hydroxy, C₁₋₄-Alkoxy, C₁₋₄-Alkylthio, Amino, mono- oder di-C₁₋₄-alkylsubstituiertem Amino, 5-bis 7-gliedrigem cyclischem Amino, C₁₋₄-Alkylcarbonylamino, C₁₋₄-Alkylsulfonylamino, C₁₋₄-Alkoxycarbonyl, Carboxyl, C₁₋₆-Alkylcarbonyl, Carbamoyl, mono- oder di-C₁₋₄-alkylsubstituiertem Carbamoyl, C₁₋₆-Alkylsulfonyl, C₁₋₄-Alkylendioxy und einer 5- oder 6-gliedrigen heterocyclischen Gruppe oder dieser Gruppe mit einem kondensierten Ring besteht, die 1 bis 3 aus N, S und O ausgewählte Heteroatome enthält und mit C₁₋₄-Alkyl substituiert sein kann, (4") einer Phenyl- oder Naphthylgruppe, die durch 1 bis 5 Substituenten substituiert sein kann, welche aus der Gruppe ausgewählt sind, die aus C₁₋₄-Alkyl, Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₇₋₁₈-Aralkyloxy, Aminocarbonyloxy, mono- oder di-C₁₋₄-alkylsubstituiertem Aminocarbonyloxy, C₁₋₄-Alkylsulfonylamino, C₁₋₄-Alkoxycarbonyl, Carboxyl, C₁₋₆-Alkylcarbonyl, C₃₋₇-Cycloalkylcarbonyl, Carbamoyl, mono- oder di-C₁₋₄-alkylsubstituiertem Carbamoyl, C₁₋₆-Alkylsulfonyl, C₃₋₇-Cycloalkylsulfonyl, C₁₋₄-Alkylendioxy und einer Phenyl-, Naphthyl-, Mono- oder Diphenyl-C₁₋₃-alkyl-, Phenoxy-, Benzoyl-, Phenoxycarbonyl-, Benzylcarbonyl-, Phenyl-C₁₋₄-alkylcarbamoyl-, Phenylcarbamoyl-, Phenyl-C₁₋₄-alkylcarbonylamino-, Benzoylamino-, Phenyl-C₁₋₄-alkylsulfonyl-, Phenylsulfonyl-, Phenyl-C₁₋₄-alkylsulfinyl-, Phenyl-C₁₋₄-alkylsulfonylamino- oder Phenylsulfonylaminogruppe besteht, die durch 1 bis 4 Substituenten substituiert sein kann, welche aus der Gruppe ausgewählt sind, die aus C₁₋₄-Alkyl, C₁₋₄-Alkoxy, Halogen, Hydroxy, Benzyloxy, Amino, mono- oder di-C₁₋₄-alkylsubstituiertem Amino, Nitro, C₁₋₆-Alkylcarbonyl und Benzoyl besteht, (5") einer C₇₋₁₈-Aralkyl-, C₆₋₁₄-Aryl-C₂₋₁₂-alkenyl-, C₆₋₁₄-Aryl-C₂₋₁₂-alkinyl- oder C₃₋₇-Cycloalkyl-C₁₋₆-alkylgruppe, die durch 1 bis 5 Substituenten substituiert sein kann, welche aus der Gruppe ausgewählt sind, die aus C₁₋₄-Alkyl, Halogen, Nitro, Cyano, Hydroxy, C₁₋₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₇₋₁₈-Aralkyloxy, Aminocarbonyloxy, mono- oder di-C₁₋₄-alkylsubstituiertem Aminocarbonyloxy, C₁₋₄-Alkylsulfonylamino, C₁₋₄-Alkoxycarbonyl, Carboxyl, C₁₋₆-Alkylcarbonyl, C₃₋₇-Cycloalkylcarbonyl, Carbamoyl, mono- oder di-C₁₋₄-alkylsubstituiertem Carbamoyl, C₁₋₆-Alkylsulfonyl, C₃₋₇-Cycloalkylsulfonyl, C₁₋₄-Alkylendioxy und einer Phenyl-, Naphthyl-, Mono- oder Diphenyl-C₁₋₃-alkyl-, Phenoxy-, Benzoyl-, Phenoxycarbonyl-, Benzylcarbonyl-, Phenyl-C₁₋₄-alkylcarbamoyl-, Phenylcarbamoyl-, Phenyl-C₁₋₄-alkylcarbonylamino-, Benzoylamino-, Phenyl-C₁₋₄-alkylsulfonyl-, Phenylsulfonyl-, Phenyl-C₁₋₄-alkylsulfinyl-, Phenyl-C₁₋₄-alkylsulfonylamino- oder Phenylsulfonylaminogruppe besteht, die durch 1 bis 4 Substituenten substituiert sein kann, welche aus der Gruppe ausgewählt sind, die aus C₁₋₄-Alkyl, C₁₋₄-Alkoxy, Halogen, Hydroxy, Benzyloxy, Amino, mono- oder di-C₁₋₄-alkylsubstituiertem Amino, Nitro, C₁₋₆-Alkylcarbonyl und Benzoyl besteht, (6'') einem Halogenatom, (7" ) Nitrogruppe, (8" ) Cyanogruppe, (9" ) Hydroxylgruppe, (10" ) C₁₋₄-Alkoxygruppe, (11" ) C₁₋₄-Alkylthiogruppe, (12") Aminogruppe, (13") Mono- oder Di-C₁₋₄-alkylaminogruppe, (14") C₁₋₄-Alkylcarbonylaminogruppe, (15" ) C₁₋₄-Alkylsulfonylaminogruppe, (16") C₁₋₄-Alkoxycarbonylgruppe, (17") Carboxylgruppe, (18") Formylgruppe, (19") C₁₋₆-Alkylcarbonylgruppe, (20") C₁₋₄-Alkylcalbonyloxygruppe, (21") ω-Oxo-ω-(tetrahydrobenzazepinyl) -C₁₋₆-alkylgruppe, (22") Benzoylgruppe, die durch 1 bis 3 Substituenten substituiert sein kann, die aus der Gruppe ausgewählt sind, welche aus C₁₋₄-Alkyl, Halogen, C₁₋₄-Alkoxy, Mono- oder Di-C₁₋₄-alkylamino, einer 5- bis 7-gliedrigen cyclischen Aminogruppe, Nitro und Hydroxy ausgewählt ist, (23") Carbamoylgruppe, (24") Mono- oder Di-C₁₋₄-alkylcarbamoylgruppe, (25") C₁₋₆-Alkylsulfonylgruppe, (26") Oxogruppe und (27") heterocyclischen Gruppe ausgewählt sind, die aus Pyridyl, Pyrazinyl, Pyrimidinyl, Chinolinyl, Isochinolinyl, Naphthyridinyl, Benzothiazolyl, Benzoxazolyl, Furanyl und Thiophenyl ausgewählt ist; Ring A ein Benzolring ist, der weiter durch 1 bis 3 Substituenten substituiert sein kann, die aus der Gruppe ausgewählt sind, welche aus C₁₋₄-Alkyl, Halogen, Nitro, Cyano, Hydroxy, C₁₄-Alkoxy, C₁₋₄-Alkylthio, Amino, mono- oder di-C₁₋₄-alkylsubstituiertem Amino, 5- bis 7-gliedrigem cyclischem Amino, C₁₋₄-Alkylcarbonylamino, C₇₋₁₈-Aralkyloxy, Aminocarbonyloxy, mono- oder di-C₁₋₄-alkylsubstituiertem Aminocarbonyloxy, C₁₋₄ -Alkylsulfonylamino, C₁₋₄-Alkoxycarbonyl, Carboxyl, C₁₋₆-Alkylcarbonyl, C₃₋₇-Cycloalkylcarbonyl, Carbamoyl, mono- oder di-C₁₋₄-alkylsubstituiertem Carbamoyl, C₁₋ ₆-Alkylsulfonyl, C₃₋₇-Cycloalkylsulfonyl und Phenyl, Naphthyl, Mono- oder Diphenyl-C₁₋₃-alkyl, Phenoxy, Benzoyl, Phenoxycarbonyl, Benzylcarbonyl, Phenyl-C₁₋₄-alkylcarbamoyl, Phenylcarbamoyl, Phenyl-C₁₋₄-alkylcarbonylamino, Benzoylamino, Phenyl-C₁₋₄-alkylsulfonyl, Phenylsulfonyl, Phenyl-C₁₋₄-alkylsulfinyl, Phenyl-C₁₋₄-alkylsulfonylamino oder Phenylsulfonylamino besteht, die durch 1 bis 4 Substituenten substituiert sein kann, welche aus der Gruppe ausgewählt sind, die aus C₁₋₄-Alkyl, C₁₋₄-Alkoxy, Halogen, Hydroxy, Benzyloxy, Amino, mono- oder di-C₁₋₄-alkylsubstituiertem Amino, Nitro, C₁₋₆-Alkylcarbonyl und Benzoyl besteht; sich die R² voneinander in der Wiederholung n unterscheiden können; k eine ganze Zahl 0 bis 3 ist, m eine ganze Zahl 1 bis 8 ist und n eine ganze Zahl 1 bis 10 ist, vorausgesetzt, daß wenn k = 0 und m = 2, n eine nicht kleinere ganze Zahl als 2 ist, oder ein pharmazeutisch annehmbares Salz derselben.

2. Verbindung wie in Anspruch 1 beansprucht, wobei R¹
(i) ein Wasserstoffatom,
(ii) eine C₁₋₄-Alkylgruppe, die mit einer Hydroxygruppe substituiert sein kann,
(iii) eine C₂₋₄-Alkinylgruppe,
(iv) eine Phenyl-C₁₋₃-alkylgruppe, die mit einem bis drei Substituenten substituiert sein kann, die aus der Gruppe ausgewählt sind, welche aus Halogen, einer Nitro-, Cyano-, C₁₋₄-Alkyl, C₁₋₄-Alkoxy-, Hydroxy- und Phenylmethoxygruppe besteht,
(v) Formylgruppe,
(vi) eine C₁₋₄-Alkylcarbonylgruppe,
(vii) Benzoylgruppe,
(viii) eine C₁₋₄-Alkoxycarbonylgruppe,
(ix) Pyridylcarbonylgruppe oder
(x) eine Mono- oder Di-C₁₋₄-alkylcarbamoylgruppe ist.

3. Verbindung wie in Anspruch 1 beansprucht, wobei R¹ (i) H, (ii) eine geradkettige oder verzweigte C₁₋₄-Alkylgruppe, (iii) eine Phenyl-C₁₋₃-alkylgruppe, die durch 1 bis 3 Substituenten substituiert sein kann, die aus der Gruppe ausgewählt sind, welche aus C₁₋₄-Alkyl, Halogen, Nitro, Cyano, Hydroxy, C₁₋₄-Alkoxy und C₇₋₈-Aralkyloxy besteht, (iv) einer Naphthyl-C₁₋₃-alkylgruppe, (v) C₁₋₃-Alkylcarbonyl, (vi) Phenyloxycarbonyl oder (vii) C₁₋₄-Alkoxycarbonyl ist.

4. Verbindung wie in Anspruch 1 beansprucht, wobei R¹ (i) H, (ii) eine geradkettige oder verzweigte C₁₋₄-Alkylgruppe, (iii) eine C₁₋₄-Alkylcarbonylgruppe oder (iv) eine Phenyl-C₁₋₃-alkylgruppe ist, die durch C₁₋₄-Alkoxy substituiert sein kann.

5. Verbindung wie in Anspruch 1 beansprucht, wobei k + m eine ganze Zahl 2 bis 6 ist.

6. Verbindung wie in Anspruch 1 beansprucht, wobei k eine ganze Zahl 0 bis 2 ist und m eine ganze Zahl 2 bis 5 ist.

7. Verbindung wie in Anspruch 1 beansprucht, wobei die Struktureinheit von Formel [I] 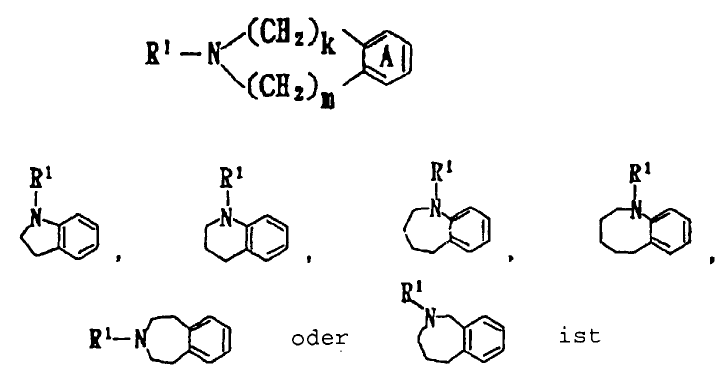 und R¹ (i) H, (ii) eine geradkettige oder verzweigte C₁₋₄-Alkylgruppe, (iii) eine C₁₋₄-Alkylcarbonylgruppe oder (iv) eine Phenyl-C₁₋₃-alkylgruppe ist, die durch C₁₋₄-Alkoxy substituiert sein kann.

8. Verbindung wie in Anspruch 1 beansprucht, wobei der Ring A ein Benzolring ist.

9. Verbindung wie in Anspruch 1 beansprucht, wobei n eine ganze Zahl 1 bis 6 ist.

10. Verbindung wie in Anspruch 1 beansprucht, wobei R² ein Wasserstoffatom oder eine Phenylgruppe ist.

11. Verbindung wie in Anspruch 1 beansprucht, wobei R² H ist.

12. Verbindung wie in Anspruch 1 beansprucht, wobei R³ und R⁴ unabhängig
(i) eine C₁₋₄-Alkylgruppe, die mit einer Hydroxygruppe substituiert sein kann,
(ii) Phenylgruppe oder
(iii) eine Phenyl-C₁₋₃-alkylgruppe ist, die mit einem Halogenatom substituiert sein kann,
oder R³ und R⁴ mit dem benachbarten Stickstoffatom zusammengenommen
(i') eine Piperidinylgruppe, die mit einer Phenyl-C₁₋₃-alkylgruppe, einer Hydroxygruppe oder Oxo substituiert sein kann,
(ii') 4-Oxo-1-phenyl-1,3,8-triazaspiro[4,5]decan-8-yl,
(iii') 1,2,3,4-Tetrahydroisochinolin-2-yl,
(iv' ) Pyrrolidinyl,
(v') Morpholinyl,
(vi') eine Homopiperazinylgruppe, die mit einer Phenyl-C₁₋₃-alkylgruppe substituiert sein kann, oder
(vii') eine Piperazinylgruppe bilden kann, die mit (1) einer Phenyl-C₁₋₃-alkylgruppe, welche mit einem Halogenatom, einer C₁₋₄-Alkoxygruppe oder einer C₁₋₄-Alkylendioxygruppe substituiert sein kann, (2) Pyridylgruppe, (3) einer Benzoylgruppe, die mit einem Halogenatom substituiert sein kann, (4) einer C₁₋₄-Alkylgruppe, die mit einer Hydroxygruppe, 3-Oxo-3-[3-(phenylmethyl)-2,3,4,5-tetrahydro-1H-3-benzazepin-7-yl]propyl-, Pyridyl-, Furyl- oder 2-Methylthiazol-4-ylgruppe substituiert sein kann, (5) Formylgruppe, (6) einer C₁₋₆-Alkylcarbonylgruppe, (7) einer Phenylgruppe, die mit einem Halogenatom substituiert sein kann, (8) Hydroxylgruppe oder (9) einer Diphenyl-C₁₋₃-alkylgruppe substituiert sein kann.

13. Verbindung wie in Anspruch 1 beansprucht, wobei eines von R³ und R⁴ H oder eine geradkettige oder verzweigte C₁₋₄-Alkylgruppe ist und das andere eine geradkettige oder verzweigte C₁₋₄-Alkylgruppe, eine Phenyl-C₁₋₃-alkylgruppe oder eine Naphthyl-C₁₋₃-alkylgruppe ist.

14. Verbindung wie in Anspruch 1 beansprucht, wobei R³ und R⁴ mit dem benachbarten Stickstoffatom zusammengenommen eine Pyrrolidinyl-, Piperidinyl-, Piperazinyl-, Morpholinyl- oder 1,2,3,4-Tetrahydrochinolylgruppe bilden können, die durch 1 oder 2 Substituenten substituiert sein kann, welche aus der Gruppe ausgewählt sind, die aus (1) Formyl, (2) C₁₋₄-Alkylcarbonyl, (3) Hydroxy, (4) Oxo, (5) Pyridyl, (6) Benzoyl, das durch 1 bis 3 Halogenatome substituiert sein kann, (7) geradkettigem oder verzweigtem C₁₋₇-Alkyl, das durch 1 bis 3 Substituenten substituiert sein kann, welche aus der Gruppe ausgewählt sind, die aus Hydroxy, Pyridyl, Furyl, Thiazol-4-yl und 2-Methylthiazol-4-yl besteht, (8) Phenyl, das durch 1 bis 3 Substituenten substituiert sein kann, welche aus der Gruppe ausgewählt sind, die aus Halogen, Hydroxy und C₁₋₄-Alkylendioxy besteht, (9) C₇₋₁₈-Aralkyl, das durch 1 bis 3 Substituenten substituiert sein kann, welche aus der Gruppe ausgewählt sind, die aus Halogen, Hydroxy und C₁₋₄-Alkylendioxy besteht, und (10) ω-Oxo-ω- (tetrahydrobenzazepinyl) -C₁₋₆-alkyl besteht.

15. Verbindung wie in Anspruch 1 beansprucht, wobei R³ und R⁴ mit dem benachbarten Stickstoffatom zusammengenommen 4-(Phenylmethyl)-piperazin-1-yloder4-[(2-Methylthiazol-4-yl)methyl]-piperazin-1-yl bilden können.

16. Verbindung wie in Anspruch 1 beansprucht, wobei n eine ganze Zahl 3 bis 8 ist, wenn die Gruppe der Formel 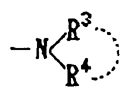 keine heterocyclische Gruppe bildet und n eine ganze Zahl 2 bis 5 ist, wenn die Gruppe eine heterocyclische Gruppe bildet.

17. Verbindung wie in Anspruch 1 beansprucht, die eine Verbindung der Formel 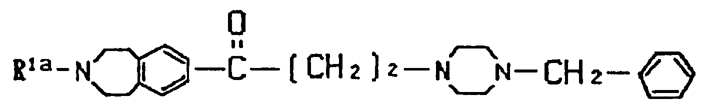 ist, worin R^{1a}
(i) eine C₁₋₄-Alkylgruppe oder
(ii) eine Phenyl-C₁₋₃-alkylgruppe ist, die mit einer C₁₋₄-Alkoxygruppe substituiert sein kann,
oder ein pharmazeutisch annehmbares Salz.

18. Verbindung wie in Anspruch 1 beansprucht, die 1-[3-[(4-Methoxyphenyl)methyl]-2,3,4,5-tetrahydro-1H-3-benzazepin-7-yl]-3-[4-(phenylmethyl)piperazin-1-yl]-1-propanon oder ein pharmazeutisch annehmbares Salz desselben ist.

19. Verbindung wie in Anspruch 1 beansprucht, die 1-[3-(2-Phenylethyl)-2,3,4,5-tetrahydro-1H-3-benzazepin-7-yl]-3-[4-(phenylmethyl)piperazin-1-yl]-1-propanon oder ein pharmazeutisch annehmbares Salz desselben ist.

20. Verbindung wie in Anspruch 1 beansprucht, die 1-[3-(2-Methylpropyl)-2,3,4,5-tetrahydro-1H-3-benzazepin-7-yl]-3-[4-(phenylmethyl)piperazin-1-yl]-1-propanon oder ein pharmazeutisch annehmbares Salz desselben ist.

21. Verbindung wie in Anspruch 1 beansprucht, die 1-[3-(Phenylmethyl)-2,3,4,5-tetrahydro-1H-3-benzazepin-7-yl]-3-[4-(phenylmethyl)piperazin-1-yl]-1-propanon oder ein pharmazeutisch annehmbares Salz desselben ist.

22. Verbindung wie in Anspruch 1 beansprucht, die 1-[1-(Phenylmethyl)-2,3-dihydro-1H-indol-5-yl]-3-[4-(phenylmethyl)piperazin-1-yl]-1-propanon oder ein pharmazeutisch annehmbares Salz desselben ist.

23. Verbindung wie in Anspruch 1 beansprucht, die 1-[2-(Phenylmethyl)-2,3,4,5-tetrahydro-1H-2-benzazepin-8-yl]-3-[4-(phenylmethyl)piperazin-1-yl]-1-propanon oder ein pharmazeutisch annehmbares Salz desselben ist.

24. Verbindung wie in Anspruch 1 beansprucht, die 3-[4-[(2-Methylthiazol-4-yl)methyl]piperazin-1-yl]-1-[3-(phenylmethyl)-2,3,4,5-tetrahydro-1H-3-benzazepin-7-yl]-1-propanon oder ein pharmazeutisch annehmbares Salz desselben ist.

25. Verbindung der Formel 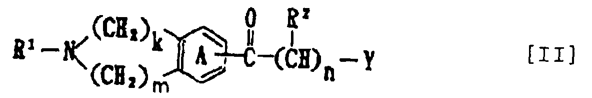 worin Y Halogen, C₁₋₆-Alkylsulfonyloxy oder C₆₋₁₀-Arylsulfonyloxy ist, R¹, Ring A, R², n, k und m von derselben Bedeutung sind wie in Anspruch 1 definiert sind, oder ein Salz derselben, vorausgesetzt, daß wenn k = 0 und m = 2 oder 3, n eine ganze Zahl nicht kleiner als 2 bezeichnet.

26. Verfahren zum Herstellen einer Verbindung der Formel 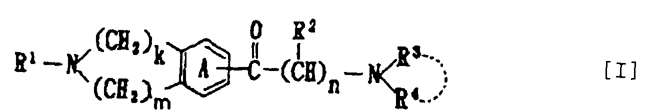 worin R¹, Ring A, R², R³, R⁴, k, m und n wie in Anspruch 1 definiert sind, oder eines pharmazeutisch annehmbaren Salzes derselben, welches das Umsetzen einer Verbindung der Formel 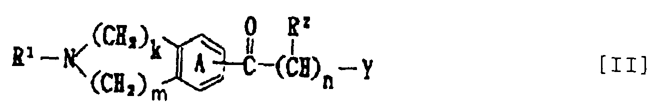 worin Y eine Abgangsgruppe ist, R¹, Ring A, R², n, k und m wie in Anspruch 1 definiert sind, oder eines Salzes derselben mit einer Verbindung der Formel 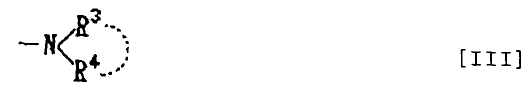 worin R³ und R⁴ wie vorstehend definiert sind, oder einem Salz derselben umfaßt.

27. Verfahren zum Herstellen einer Verbindung der Formel 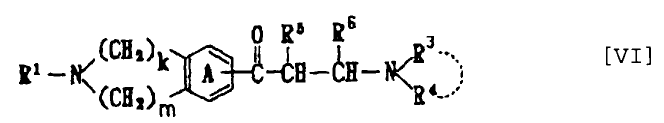 worin R¹, R³, R⁴, Ring A, k und m wie in Anspruch 1 definiert sind und R⁵ und R⁶ wie R² in Anspruch 1 definiert sind, oder eines pharmazeutisch annehmbaren Salzes derselben, welches das Umsetzen einer Verbindung der Formel 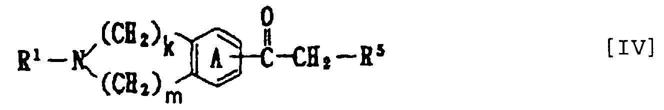 worin R¹, R⁵, Ring A, k und m wie vorstehend definiert sind, oder eines Salzes derselben und einer Verbindung der Formel
R⁶-CHO [V]
worin R⁶ wie vorstehend definiert ist, mit einer Verbindung der Formel 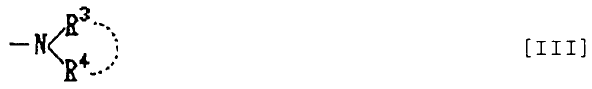 worin R³ und R⁴ wie vorstehend definiert sind, oder einem Salz derselben umfaßt.

28. Cholinesterasehemmer, der eine Verbindung der Formel 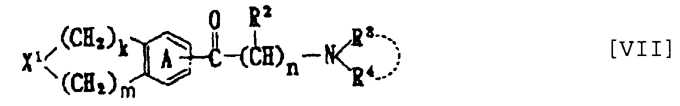 worin R¹, R², R³, R⁴, Ring A, k, m und n wie in Anspruch 1 definiert sind und worin X¹ R¹-N, O oder S ist, oder ein pharmazeutisch annehmbares Salz derselben enthält.

29. Pharmazeutische Zusammensetzung für eine durch Acetylcholinesteraseaktivität verursachte Erkrankung, welche eine cholinesterasehemmende wirksame Menge einer in Anspruch 1 beanspruchten Verbindung der Formel [I] oder eines pharmazeutisch annehmbaren Salzes derselben und einen pharmakologisch annehmbaren Träger enthält.

30. Pharmazeutische Zusammensetzung für eine durch Acetylcholinesteraseaktivität verursachte Erkrankung, welche eine cholinesterasehemmende wirksame Menge einer in Anspruch 28 beanspruchten Verbindung der Formel [VII] oder eines pharmazeutisch annehmbaren Salzes derselben und einen pharmakologisch annehmbaren Träger enthält.

31. Pharmazeutische Zusammensetzung wie in Anspruch 29 beansprucht, bei welcher die Erkrankung senile Demenz und/oder Alzheimersche Krankheit ist.

32. Pharmazeutische Zusammensetzung wie in Anspruch 30 beansprucht, bei welcher die Erkrankung senile Demenz und/oder Alzheimersche Krankheit ist.

33. Verwendung einer Verbindung der Formel 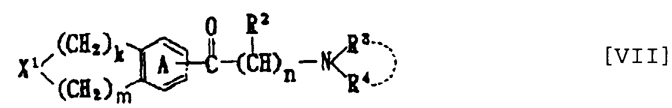 worin R¹, R², R³, R⁴, Ring A, k, m und n wie in Anspruch 1 definiert sind und worin X¹ R¹-N, O oder S ist, oder eines pharmazeutisch annehmbaren Salzes derselben als Bestandteil bei der Herstellung eines Cholinesterasehemmers.

## Revendications

1. Composé de formule 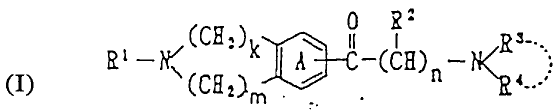 dans laquelle R¹ représente
(1) un atome d'hydrogène
(2) un groupe alkyle en C₁₋₁₁ à chaîne linéaire ou ramifiée, un groupe alcényle en C₂₋₄ ou alcynyle en C₂₋₄ pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, (alkyle en C₁₋₄)-sulfonylamino, (alcoxy en C₁₋₄)-carbonyle, carboxyle, (alkyle en C₁₋₆)-carbonyle, carbamoyle, mono- ou di-(alkyle en C₁₋₄)-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄,
(3) un groupe cycloalkyle en C₃₋₇ monocyclique pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, (alkyle en C₁₋₄)-sulfonylamino, (alcoxy en C₁₋₄)-carbonyle, carboxyle, (alkyle en C₁₋₆)-carbonyle, carbamoyle, mono- ou di-(alkyle en C₁₋₄)-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄,
(4) un groupe bicyclo[3.2.1]oct-2-yle, bicyclo[3.2.1]non-2-yle ou adamantan-lyle pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-Carbonylamino, (alkyle en C₁₋₄)-sulfonylamino, (alcoxy en C₁₋₄)-carbonyle, carboxyle, (alkyle en C₁₋₆)-carbonyle, carbamoyle, mono- ou di-(alkyle en C₁₋₄)-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄,
(5) un groupe phényle ou naphtyle pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, aralkyloxy en C₇₋₁₈, aminocarbonyloxy, mono- ou di-(alkyle en C₁₋₄)-aminocarbonyloxy, (alkyle en C₁₋₄)-sulfonylamino, (alcoxy en C₁₋₄-carbonyle, carboxyle, (alkyle en C₁₋₆)-carbonyle, (cycloalkyle en C₃₋₇)-carbonyle, carbamoyle, mono- ou di-(alkyle en C₁₋₄)-carbamoyle, (alkyle en C₁₋₆)-sulfonyle, (cycloalkyle en C₃₋₇)-sulfonyle, et des résidus phényle, naphtyle, mono- ou diphényl-(alkyle en C₁₋₃), phénoxy, benzoyle, phénoxycarbonyle, benzylcarbonyle, phényl-(alkyle en C₁₋₄)-carbamoyle, phénylcarbamoyle, phényl-(alkyle en C₁₋₄)-carbonylamino, benzoylamino, phényl-(alkyle en C₁₋₄)-sulfonyle, phénylsulfonyle, phényl-(alkyle en C₁₋₄)-sulfinyle, phényl-(alkyle en C₁₋₄)-suflonylamino ou phénylsulfonylamino pouvant porter, à leur tour, de 1 à 4 substituants choisis dans le groupe formé par les résidus alkyle en C₁₋₄, alcoxy en C₁₋₄, halogéno, hydroxy, benzyloxy, amino, mono- ou di-(alkyle en C₁₋₄)amino, nitro, (alkyle en C₁₋₆)-carbonyle et benzoyle, et
(6) un résidu aralkyle en C₇₋₁₈, (aryle en C₆₋₁₄)-(alcényle en C₂₋₁₂), (aryle en C₆₋₁₄)-(alcynyle en C₂₋₁₂) ou (cycloalkyle en C₃₋₇)-(alkyle en C₁₋₆) pouvant porter, à leur tour, de 1 à 5 substituants choisis dans le groupe formé par les résidus alkyle en C₁₋₄, halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄-carbonylamino, aralkyloxy en C₇₋₁₈, aminocarbonyloxy, mono- ou di-(alkyle en C₁₋₄)-aminocarbonyloxy, (alkyle en C₁₋₄)-sulfonylamino, (alcoxy en C₁₋₄)-carbonyle, carboxyle, (alkyle en C₁₋₆)-carbonyle, (cycloalkyle en C₃₋₇)-carbonyle, carbamoyle, mono- ou di-(alkyle en C₁₋₄-carbamoyle, (alkyle en C₁₋₆)-sulfonyle, (cycloalkyle en C₃₋₇)-sulfonyle, et des résidus phényle, naphtyle, mono- ou diphényl-(alkyle en C₁₋₃), phénoxy, benzoyle, phénoxycarbonyle, benzylcarbonyle, phényl-(alkyle en C₁₋₄)-carbamoyle, phénylcarbamoyle, phényl-(alkyle en C₁₋₄)-carbonylamino, benzoylamino, phényl-(alkyle en C₁₋₄)-sulfonyle, phénylsulfonyle, phényl-(alkyle en C₁₋₄)-sulfinyle, phényl-(alkyle en C₁₋₄)-sulfonylamino ou phénylsulfonylamino pouvant porter, à leur tour, de 1 à 4 substituants choisis dans le groupe formé par les résidus alkyle en C₁₋₄, alcoxy en C₁₋₄, halogéno, hydroxy, benzoyloxy, amine, mono ou di-(alkyle en C₁₋₄)-amino, nitro, (alkyle en C₁₋₆)-carbonyle et benzoyle,
(7) un résidu (alkyle en C₁₋₈)-carbonyle ou (aryle en C₆₋₁₄)-carbonyle pouvant porter de 1 à 3 substituants choisis dans le groupe formé par les résidus halogéno, amino, mono- ou di-(alkyle en C₁₋₆)-amino et alcoxy en C₁₋₆,
(8) un résidu (alkyle en C₁₋₇)-sulfonyle ou (aryle en C₆₋₁₄)-sulfonyle pouvant porter de 1 à 3 substituants choisis dans le groupe formé par les résidus halogéno, amino, mono- ou di-(alkyle en C₁₋₆)-amino et alcoxy en C₁₋₆,
(9) un résidu (alkyle en C₁₋₇)-posphonyle ou (aryle en C₆₋₁₄)-phosphonyle pouvant porter de 1 à 3 substituants choisis dans le groupe formé par les résidus halogéno, amino, mono- ou di-(alkyle en C₁₋₆)-amino et alcoxy en C₁₋₆,
(10) un résidu (alcoxy en C₁₋₈)-carbonyle ou (aralkyle en C₇₋₁₈)-carbonyle pouvant porter de 1 à 3 substituants choisis dans le groupe formé par les résidus halogéno, amino, mono- ou di-(alkyle en C₁₋₆)-amino et alcoxy en C₁₋₆,
(11) un groupe hétérocyclo-carbonyle dans lequel le groupe hétérocyclique est un hétérocycle à 5 ou 6 chaînons contenant de 1 à 3 hétéroatomes choisis parmi N, S et O, qui peut porter de 1 à 3 substituants choisis dans le groupe formé par les résidus halogéno, amino, mono- ou di-(alkyle en C₁₋₆)-amine et alcoxy en C₁₋₆,
(12) un groupe carbamoyle pouvant porter de 1 à 3 substituants choisis dans le groupe formé par les résidus halogéno, amino, mono- ou di-(alkyle en C₁₋₆)-amine et alcoxy en C₁₋₆,
(13) un résidu mono- ou di-(alkyle en C₁₋₄)-carbamoyle pouvant porter de 1 à 3 substituants choisis dans le groupe formé par les résidus halogéno, amino, mono- ou di-(alkyle en C₁₋₆)-amino et alcoxy en C₁₋₆, ou
(14) un groupe formyle;
R² représente
(1') un atome d'hydrogène,
(2') un groupe alkyle en C₁₋₁₁ à chaîne linéaire ou ramifiée, un groupe alcényle en C₂₋₄ ou alcynyle en C₂₋₄ pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄-amino, amino cyclique à 5 - 7 chaînons, (alkyle en C₁₋₄)-carbonylamino, (alkyle en C₁₋₄)-sulfonylamino, (alcoxy en C₁₋₄)-carbonyle, carboxyle, (alkyle en C₁₋₆)-carbonyle, carbamoyle, mono- ou di-(alkyle en C₁₋₄-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄,
(3') un groupe cycloalkyle en C₃₋₇ monocyclique pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄-carbonylamino, (alkyle en C₁₋₄)-sulfonylamino, (alcoxy en C₁₋₄)-carbonyle, carboxyle, (alkyle en C₁₋₆)-carbonyle, carbamoyle, mono- ou di-(alkyle en C₁₋₄)-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄,
(4') un groupe bicyclo[3.2.1]oct-2-yle, bicyclo[3.2.1]non-2-yle ou adamantan-lyle pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 à 7 chaînons, (alkyle en C₁₋₄)-carbonylamino, (alkyle en C₁₋₄)-sulfonylamino, (alcoxy en C₁₋₄)-carbonyle, carboxyle, (alkyle en C₁₋₄)-carbonyle, carbamoyle, mono- ou di-(alkyle en C₁₋₄)-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄,
(5') un groupe phényle ou naphtyle pouvant porter de 1 à 5 substituants choisis dans le groupe formé par les résidus alkyle en C₁₋₄, halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, aralkyloxy en C₇₋₁₈, aminocarbonyloxy, mono- ou di-(alkyle en C₁₋₄)-aminocarbonyloxy, (alkyle en C₁₋₄)-sulfonylamino, (alcoxy en C₁₋₄)-carbonyle, carboxyle, (alkyle en C₁₋₆)-carbonyle, carbamoyle, mono- ou di-(alkyle en C₁₋₄)-carbamoyle, (alkyle en C₁₋₆)-sulfonyle, (cycloalkyle en C₃₋₇)-carbonyle et des résidus phényle, naphtyle, mono- ou diphényl-(alkyle en C₁₋₃), phénoxy, benzoyle, phénoxycarbonyle, benzylcarbonyle, phényl-(alkyle en C₁₋₄)-carbamoyle, phénylcarbamoyle, phényl-(alkyle en C₁₋₄)-carbonylamino, benzoylamino, phényl-(alkyle en C₁₋₄)-sulfonyle, phénylsulfonyle, phényl-(alkyle en C₁₋₄)-sulfinyle, phényl-(alkyle en C₁₋₄)-sulfonylamino ou phénylsulfonylamino pouvant porter à leur tour de 1 à 4 substituants choisis dans le groupe formé par les résidus alkyle en C₁₋₄, alcoxy en C₁₋₄, halogéno, hydroxy, benzyloxy, amino, mono- ou di-(alkyle en C₁₋₄)-amino, nitro, (alkyle en C₁₋₆)-carbonyle et benzoyle,
(6') un résidu aralkyle en C₇₋₁₈, (aryle en C₆₋₁₄)-(alcényle en C₂₋₁₂) (aryle en C₆₋₁₄)-(alcynyle en C₂₋₁₂) ou (cycloalkyle en C₃₋₇)-(alkyle en C₁₋₆) pouvant porter, à leur tour, de 1 à 5 substituants choisis dans le groupe formé par les résidus alkyle en C₁₋₄, halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, aralkyloxy en C₇₋₁₈, aminocarbonyloxy, mono- ou di-(alkyle en C₁₋₄)-aminocarbonyloxy, (alkyle en C₁₋₄)-sulfonylamino, (alcoxy en C₁₋₄)-carbonyle, carboxyle, (alkyle en C₁₋₆)-carbonyle, (cycloalkyle en C₃₋₇)-carbonyle, carbamoyle, mono- ou di-(alkyle en C₁₋₄)-carbamoyle, (alkyle en C₁₋₆)-sulfonyle, (cycloalkyle en C₃₋₇)-sulfonyle, et des résidus phényle, naphtyle, mono- ou diphényl-(alkyle en C₁₋₃), phénoxy, benzoyle, phénoxycarbonyle, benzylcarbonyle, phényl-(alkyle en C₁₋₄)-carbamoyle, phénylcarbamoyle, phényl-(alkyle en C₁₋₄)-carbonylamino, benzoylamino, phényl-(alkyle en C₁₋₄)-sulfonyle, phénylsulfonyle, phényl-(alkyle en C₁₋₄)-sulfinyle, phényl-(alkyle en C₁₋₄)-sulfonylamino ou phénylsulfonylamino pouvant porter, à leur tour, de 1 à 4 substituants choisis dans le groupe formé par les résidus alkyle en C₁₋₄, alcoxy en C₁₋₄, halogéno, hydroxy, benzoyloxy, amino, mono ou di-(alkyle en C₁₋₄)-amino, nitro, (alkyle en C₁₋₆)-carbonyle et benzoyle;
un des résidus R³ et R⁴ représente un atome d'hydrogène ou un résidu alkyle en C₁₋₄ à chaîne linéaire ou ramifiée et l'autre représente un résidu alkyle en C₁₋₄ à chaîne linéaire ou ramifiée, un groupe phényl-(alkyle en C₁₋₃) ou naphtyl-(alkyle en C₁₋₃), R³ et R⁴ peuvent former avec l'atome d'azote auquel ils sont liés un groupe hétérocyclique à 3 - 13 chaînons comportant, autre que les atomes de carbone et un atome d'azote, éventuellement de 1 à 3 atomes d'azote, d'oxygène et/ou de soufre, qui peut porter de 1 à 5 substituants choisis dans le groupe formé par
(1") un groupe alkyle en C₁₋₁₁ à chaîne linéaire ou ramifiée, un groupe alcényle en C₂₋₄ ou alcynyle en C₂₋₄ pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, (alkyle en C₁₋₄)-sulfonylamino, (alcoxy en C₁₋₄)-carbonyee, carboxyle, (alkyle en C₁₋₆)-carbonyle, carbamoyle, mono- ou di-(alkyle en C₁₋₄)-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄,
(2") un groupe cycloalkyle en C₃₋₇ monocyclique pouvant porter de 1 à 5 substituants choisis dans le groupe formé par les résidus halogéno, nitro, cyano, hydroxy, alcoxy en C₁₋₄, alkylthio en C_{1 -4}, amino, mono- ou di-(alkyle en C₁₋₄)-amino, amino cyclique à 5 - 7 chaînons, (alkyle en C₁₋₄-carbonylamino, (alkyle en C₁₋₄)-sulfonylamino, (alcoxy en C₁₋₄)-carbonyle, carboxyle, (alkyle en C₁₋₆)-carbonyle, carbamoyle, mono- ou di-(alkyle en C₁₋₄)-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄, et
(3") un groupe bicyclo[3.2.1]oct-2-yle, bicyclo[3.2.1]non-2-yle ou adamantan-lyle pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, (alkyle en C₁₋₄)-sulfonylamino, (alcoxy en C₁₋₄)-carbonyle, carboxyle, (alkyle en C₁₋₆)-carbonyle, carbamoyle, mono- ou di-(alkyle en C₁₋₄)-carbamoyle, (alkyle en C₁₋₆)-sulfonyle, (alkylène en C₁₋₄)-dioxy, et un groupe hétérocyclique à 5 ou 6 chaînons ou un tel groupe à cycles condensés comportant de 1 à 3 hétéroatomes choisis parmi N, S et O qui, à son tour, peut porter un substituant alkyle en C₁₋₄,
(4") un groupe phényle ou naphtyle pouvant porter de 1 à 5 substituants choisis dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, aralkyloxy en C₇₋₁₈, aminocarbonyloxy, mono- ou di-(alkyle en C₁₋₄)-aminocarbonyloxy, (alkyle en C₁₋₄)-sulfonylamino, (alcoxy en C₁₋₄)-carbonyle, carboxyle, (alkyle en C₁₋₆)-carbonyle, (cycloalkyle en C₃₋₇)-carbonyle, carbamoyle, mono- ou di-(alkyle en C₁₋₄)-carbamoyle, (alkyle en C₁₋₆)-sulfonyle, (cycloalkyle en C₃₋₇)-sulfonyle, et des résidus phényle, naphtyle, mono- ou diphényl-(alkyle en C₁₋₃), phénoxy, benzoyle, phénoxycarbonyle, benzylcarbonyle, phényl-(alkyle en C₁₋₄)-carbamoyle, phénylcarbamoyle, phényl-(alkyle en C₁₄)-carbonylamino, benzoylamino, phényl-(alkyle en C₁₋₄)-sulfonyle, phénylsulfonyle, phényl-(alkyle en C₁₋₄)-sulfinyle, phényl-(alkyle en C₁₋₄)-suflonylamino ou phénylsulfonylamino pouvant porter, à leur tour, de 1 à 4 substituants choisis dans le groupe formé par les résidus alkyle en C₁₋₄, alcoxy en C₁₋₄, halogéno, hydroxy, benzyloxy, amino, mono- ou di-(alkyle en C₁₋₄)amino, nitro, (alkyle en C₁₋₆)-carbonyle et benzoyle, et
(5") un résidu aralkyle en C₇₋₁₈, (aryle en C₆₋₁₄)-(alcényle en C₂₋₁₂), (aryle en C₆₋₁₄)-(alcynyle en C₂₋₁₂) ou (cycloalkyle en C₃₋₇)-(alkyle en C₁₋₆) pouvant porter, à leur tour, de 1 à 5 substituants choisis dans le groupe formé par les résidus alkyle en C₁₋₄, halogéno, nitro, cyano, hydroxy, alcoxy en C₁₋₄, alkylthio en C₁₋₄, amino, mono- ou di-(alkyle en C₁₋₄)-mmino, amino cyclique à 5 - 7 chaînons, (alkyle en C₁₋₄)-carbonylamino, aralkyloxy en C₇₋₁₈, aminocarbonyloxy, mono- ou di-(alkyle en C₁₋₄)-aminocarbonyloxy, (alkyle en C_{1 -4})-sulfonylamino, (alcoxy en C₁₋₄)-carbonyle, carboxyle, (alkyle en C₁₋₆)-carbonyle, (cycloalkyle en C₃₋₇)-carbonyle, carbamoyle, mono- ou di-(alkyle en C₁₋₄)-carbamoyle, (alkyle en C₁₋₆)-sulfonyle, (cycloalkyle en C₃₋₇)-sulfonyle, et des résidus phényle, naphtyle, mono- ou diphényl-(alkyle en C₁₋₃), phénoxy, benzoyle, phénoxycarbonyle, benzylcarbonyle, phényl-(alkyle en C₁₋₄)-carbamoyle, phénylcarbamoyle, phényl-(alkyle en C₁₋₄)-carbonylamino, benzoylamino, phényl-(alkyle en C₁₋₄)-sulfonyle, phénylsulfonyle, phényl-(alkyle en C₁₋₄)-sulfinyle, phényl-(alkyle en C₁₋₄)-sulfonylamino ou phénylsulfonylamino pouvant porter, à leur tour, de 1 à 4 substituants choisis dans le groupe formé par les résidus alkyle en C₁₋₄, alcoxy en C₁₋₄, halogéno, hydroxy, benzoyloxy, amino, mono ou di-(alkyle en C₁₋₄)-amino, nitro, (alkyle en C₁₋₆)-carbonyle et benzoyle;
(6") un atome d'halogène,
(7") un groupe nitro,
(8") un groupe cyano,
(9") un groupe hydroxyle,
(10") un groupe alcoxy en C₁₋₄,
(11") un groupe alkylthio en C₁₋₄,
(12") un groupe amino,
(13") un groupe mono- ou di-(alkyle en C₁₋₄),
(14") un groupe (alkyle en C₁₋₄)-carbonylamino,
(15") un groupe (alkyle en C₁₋₄)-sulfonylamino,
(16") un groupe (alcoxy en C₁₋₄)-carbonyle,
(17") un groupe carboxyle
(18") un groupe formyle,
(19") un groupe (alkyle en C₁₋₆)-carbonyle,
(20") un groupe (alkyle en C₁₋₄)-carbonyloxy,
(21") un groupe ω-oxo-ω-(tétrahydrobenzazépinyl-(alkyle en C₁₋₆),
(22") un groupe benzoyle pouvant porter de 1 à 3 substituants choisis dans le groupe formé par les résidus alkyle en C₁₋₄, halogéno, alcoxy en C₁₋₄, mono- ou di-(alkyle en C₁₋₄)-amino, un hétérocyle aminé à 5 - 7 chaînons, un résidu nitro et hydroxy,
(23") un groupe carbamoyle,
(24") un groupe mono- ou di-(alkyle en C₁₋₄)-carbamoyle,
(25") un groupe (alkyle en C₁₋₆)-sulfonyle,
(26") un groupe oxo,
(27") un groupe hétérocyclique choisi parmi les résidus pyridyle, pyrazinyle, pyrimidinyle, quinolinyle, isoquinolinyle, naphtyridinyle, benzothiazolyle, benzoxazolyle, furanyle et thiényle;
le cycle A est un noyau benzène pouvant porter de 1 à 3 substituants choisi dans le groupe formé par les résidus halogéno, nitro, cyano, hydroxy, alcoxy en C₁₋₄, alkylthio en C₁₋₄, amino, mono- ou di-(alkyle en C₁₋₄)-amino, amino cyclique à 5 - 7 chaînons, (alkyle en C₁₋₄)-carbonylamino, aralkyloxy en C₇₋₁₈, aminocarbonyloxy, mono- ou di-(alkyle en C₁₋₄-aminocarbonyloxy, (alkyle en C₁₋₄)-sulfonylamino, (alcoxy en C₁₋₄)-carbonyle, carboxyle, (alkyle en C₁₋₆)-carbonyle, (cycloalkyle en C₃₋₇)-carbonyle, carbamoyle, mono- ou di-(alkyle en C₁₋₄)-carbamoyle, (alkyle en C₁₋₆)-sulfonyle, (cycloalkyle en C₃₋₇)-sulfonyle, et des résidus phényle, naphtyle, mono- ou diphényl-(alkyle en C₁₋₃), phénoxy, benzoyle, phénoxycarbonyle, benzylcarbonyle, phényl-(alkyle en C₁₋₄)-carbamoyle, phénylcarbamoyle, phényl-(alkyle en C₁₋₄)-carbonylamino, benzoylamino, phényl-(alkyle en C₁₋₄)-sulfonyle, phénylsulfonyle, phényl-(alkyle en C₁₋₄)-sulfinyle, phényl-(alkyle en C₁₋₄)-sulfonylamino ou phénylsulfonylamino pouvant porter, à leur tour, de 1 à 4 substituants choisis dans le groupe formé par les résidus alkyle en C₁₋₄, alcoxy en C₁₋₄, halogéno, hydroxy, benzyloxy, amino, mono- ou di-(alkyle en C₁₋₄)amino, nitro, (alkyle en C₁₋₆)-carbonyle et benzoyle; les résidus R^{2'} au nombre de n peuvent être différents les uns des autres;
k est un nombre entier compris entre 0 et 3;
m est un nombre entier compris entre 1 et 8;
n est un nombre entier compris entre 1 et 10;
à condition que, lorsque k = O et m = 2, n soit un nombre entier au moins égal à 2, ou un sel pharmaceutiquement acceptable d'un tel composé.

2. Composé conforme à la revendication 1 dans lequel R¹ représente
(i) un atome d'hydrogène,
(ii) un groupe alkyle en C₁₋₄ pouvant être substitué par un groupe hydroxy,
(iii) un groupe alcynyle en C₂₋₄,
(iv) un groupe phényl-(alkyle en C₁₋₃) pouvant porter de 1 à 3 substituants choisis parmi halogéno, nitro, cyano, alkyle en C₁₋₄, alcoxy en C₁₋₄, hydroxy et phénylméthoxy,
(v) un groupe formyle,
(vi) un groupe (alkyle en C₁₋₄)-carbonyle,
(vii) un groupe benzoyle,
(viii) un groupe (alcoxy en C₁₋₄)-carbonyle,
(ix) un groupe pyridylcarbonyle, ou
(x) un groupe mono- ou di-(alkyle en C₁₋₄)-carbamoyle.

3. Composé conforme à la revendication 1 dans lequel R¹ représente
(i) un atome d'hydrogène,
(ii) un groupe alkyle en C₁₋₄ à chaîne linéaire ou ramifiée,
(iii) un groupe phényl-(alkyle en C₁₋₃) pouvant porter de 1 à 3 substituants choisis parmi les résidus alkyle en C₁₋₄, halogéno, nitro, cyano, hydroxy, alcoxy en C₁₋₄, et aralkyloxy en C₇₋₁₈,
(iv) un groupe naphtyl-(alkyle en C₁₋₃),
(v) un groupe (alkyle en C₁₋₃)-carbonyle,
(vi) un groupe phényloxycarbonyle ou
(vii) un groupe (alcoxy en C₁₋₄)-carbonyle.

4. Composé conforme à la revendication 1 dans lequel R¹ représente
(i) un atome d'hydrogène,
(ii) un groupe alkyle en C₁₋₄ à chaîne linéaire ou ramifiée,
(iii) un groupe (alkyle en C₁₋₄)-carbonyle, ou
(iv) un groupe phényl-(alkyle en C₁₋₃) pouvant être substitué par un groupe alcoxy en C₁₋₄.

5. Composé conforme à la revendication 1 dans lequel la somme de k + m est un nombre entier compris entre 2 et 6.

6. Composé conforme à la revendication 1 dans lequel k est un nombre entier compris entre 0 et 2 et m est un nombre entier compris entre 2 et 5.

7. Composé conforme la revendication 1 dans lequel le fragment 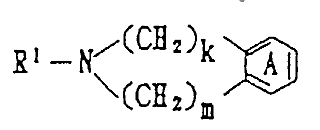 dans la formule (I) est un groupe 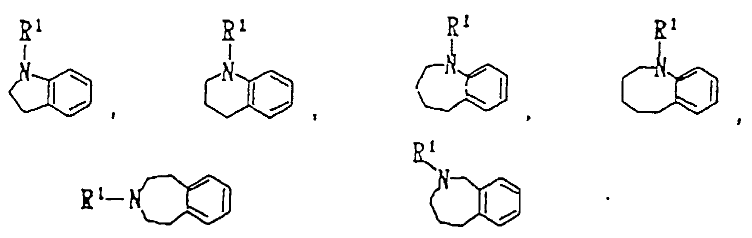 et R¹ représente
(i) un atome d'hydrogène,
(ii) un groupe alkyle en C₁₋₄ à chaîne linéaire ou ramifiée,
(iii) un groupe (alkyle en C₁₋₃)-carbonyle, ou
(iv) un groupe phényl-(alkyle en C₁₋₃) pouvant être substitué par un groupe alcoxy en C₁₋₄.

8. Composé conforme à la revendication 1 dans lequel le cycle A est un noyau benzène.

9. Composé conforme à la revendication 1 dans lequel n est un nombre entier compris entre 1 et 6.

10. Composé conforme à la revendication 1 dans lequel R² représente un atome d'hydrogène ou un groupe phényle.

11. Composé conforme à la revendication 1 dans lequel R² est un atome d'hydrogène.

12. Composé conforme à la revendication 1 dans lequel R³ et R⁴ représentent indépendamment
(i) un groupe alkyle en C₁₋₄ pouvant être hydroxylé,
(ii) un groupe phényle ou
(iii) un groupe phényl-(alkyle en C₁₋₃) pouvant être substitué par un atome d'halogène, ou
R³ et R⁴ forment avec l'atome d'azote auquel ils sont liés
(i') un groupe pipéridinyle pouvant être substitué par un groupe phényl-(alkyle en C₁₋₃), un groupe hydroxy ou oxo,
(ii') un groupe 4-oxo-1-phényl-1,3,8-triazaspiro[4,5]décan-8-yle,
(iii') un groupe 1,2,3,4-tétrahydroisoqunolin-2-yle,
(iv') un groupe pyrrolidinyle,
(v') un groupe morpholinyle,
(vi') un groupe homopipérazinyle pouvant être substitué par un groupe phényl-(alkyle en C₁₋₃), ou
(vii') un groupe pipérazinyle pouvant être substitué par
(1) un groupe phényl-(alkyle en C₁₋₃) éventuellement halogéné, un groupe alcoxy en C₁₋₄ ou alkylènedioxy en C₁₋₄,
(2) un groupe pyridyle,
(3) un groupe benzoyle éventuellement halogéné,
(4) un groupe alkyle en C₁₋₄ éventuellement hydroxylé, 3-oxo-3[3-(phénylméthyl)-2,3,4,5-tétrahydro-1H-3-benzazépin-7-yl]propyle, pyridyle, furyle ou 2-méthylthiazol-4-yle,
(5) un groupe formyle,
(6) un groupe (alkyle en C₁₋₆)-carbonyle,
(7) un groupe phényle éventuellement halogéné,
(8) un groupe hydroxyle ou (9) un groupe diphényl-(alkyle en C₁₋₃).

13. Composé conforme à la revendication 1 dans lequel un des résidus R³ et R⁴ représente un atome d'hydrogène ou un groupe alkyle en C₁₋₄ à chaîne linéaire ou ramifiée et l'autre représente un groupe alkyle en C₁₋₄ à chaîne linéaire ou ramifiée, un groupe phényl-(alkyle en C₁₋₃) ou un groupe naphtyl-(alkyle en C₁₋₃).

14. Composé conforme à la revendication 1 où R³ et R⁴ forment avec l'atome d'azote auquel ils sont liés un groupe pyrrolidinyle, pipéridinyle, pipérazinyle, morpholinyle ou 1,2,3,4-tétrahydroquinolinyle qui peuvent porter 1 ou 2 substituants choisis dans le groupe formé par les résidus
(1) formyle,
(2) (alkyle en C₁₋₄)-carbonyle,
(3) hydroxy,
(4) oxo,
(5) pyridyle,
(6) benzoyle pouvant porter de 1 à 3 atomes d'halogène,
(7) alkyle en C₁₋₇ à chaîne linéaire ou ramifiée pouvant porter de 1 à 3 substituants choisis dans le groupe formé par les résidus hydroxy, pyridyle, furyle, thiazol-4-yle et 2-méthyl-thiazol-4-yle,
(8) un groupe phényle pouvant porter de 1 à 3 substituants choisis dans le groupe formé par les résidus halogéno, hydroxy et alkylènedioxy en C₁₋₄,
(9) aralkyle en C₇₋₁₈ pouvant porter de 1 à 3 substituants choisis dans le groupe formé par les résidus halogéno, hydroxy et alkylènedioxy en C₁₋₄ et
(10) ω-owo-ω-(tétrahydrobenzazépinyl)-alkyle en C₁₋₆.

15. Composé conforme à la revendication 1 où R³ et R⁴ forment avec l'atome d'azote auquel ils sont liés un groupe 4-(phénylméthyl)-pipérazin-1-yle ou 4-[(2-méthylthiazol-4-yl)méthyl]-pipérazin-1-yle.

16. Composé conforme à la revendication 1 où n est un nombre entier compris entre 3 et 8 lorsque le groupe de formule 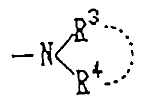 ne forme pas un hétérocycle, et n est un nombre entier compris entre 2 et 5 lorsque ce groupe forme un hétérocycle.

17. Composé conforme à la revendication 1 qui est un composé de formule 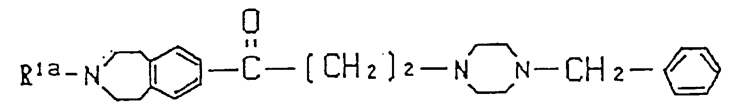 où R^{1a} est un résidu
(i) alkYle en C₁₋₄ ou
(ii) phényl-(alkyle en C₁₋₃) pouvant être substitué par un groupe alcoxyen C₁₋₄,
ou un sel pharmaceutiquement acceptable d'un tel composé.

18. Composé conforme à la revendication 1 qui est la 1-[3-[(4-méthoxyphényl)méthyl]-2,3,4,5-tétrahydro-1H-3-benzazépin-7-yl]-3-[4-(phénylméthyl)pipérazin-1-yl]-1-propanone ou un sel pharmaceutiquement acceptable d'un tel composé.

19. Composé conforme à la revendication 1 qui est la 1-[3-(2-phényléthyl)-2,3,4,5-tétrahydro-1H-3-benzazépin-7-yl]-3-[4-(phénylméthyl)pipérazin-1-yl]-1-propanone ou un sel pharmaceutiquement acceptable d'un tel composé.

20. Composé conforme à la revendication 1, qui est la 1-[3-(2-méthylpropyl)-2,3,4,5-tétrahydro-1H-3-benzazépin-7-yl]-3-[4-(phénylméthyl)pipérazin-1-yl]-1-propanone ou un sel pharmaceutiquement acceptable d'un tel composé.

21. Composé conforme à la revendication 1 qui est la 1-[3-(phénylméthyl)-2,3,4,5-tétrahydro-1H-3-benzazépin-7-yl]-3-[4-(phénylméthyl)pipérazin-1-yl]-1-propanone ou un sel pharmaceutiquement acceptable d'un tel composé.

22. Composé conforme à la revendication 1 qui est la 1-[1-(phénylméthyl)-2,3-dihydro-1H-indol-5-yl]-3[4-(phénylméthyl)pipérazin1-yl]-1-propanone ou un sel pharmaceutiquement acceptable d'un tel composé.

23. Composé conforme à la revendication 1 qui est la 1-[2-(phénylméthyl)-2,3,4,5-tétrahydro-1H-2-benzazépin-8-yl]-3-[4-(phénylméthyl)pipérazin-1-yl]-1-propanone ou un sel pharmaceutiquement acceptable d'un tel composé.

24. Composé conforme à la revendication 1, qui est la 3-[4-[(2-méthylthiazol-4-yl)méthyl]pipérazin-1-yl]-1-[3-(phénylméthyl)-2,3,4,5-tétrahydro-1H-3-benzazépin-7-yl]-1-propanone ou un sel pharmaceutiquement acceptable d'un tel composé.

25. Composé de formule 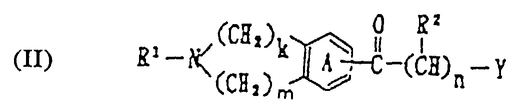 où Y représente un atome d'hydrogène, un résidu (alkyle en C₁₋₆)-sulfonyloxy ou (aryle en C₆₋₁₀)-sulfonyloxy, R¹, le cycle A, R², n, k et m ont la même signification que celle indiquée dans la revendication 1, ou un sel d'un tel composé, à condition que lorsque k = 0 et m = 2 ou 3, n soit égal à un nombre entier valant au moins 2.

26. Procédé de préparation d'un composé de formule 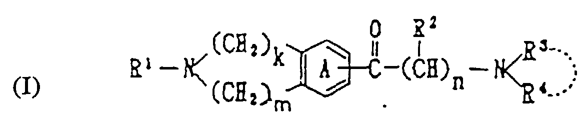 dans laquelle R¹, le cycle A, R², R³, R⁴, n, k et m ont la même signification que celle indiquée dans la revendication 1, ou d'un sel pharmaceutiquement acceptable d'un tel composé, lequel procédé comprend la réaction d'un composé de formule 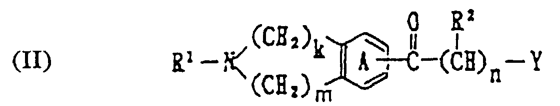 où Y représente un groupe partant, R¹, le cycle A, R², n, k et m sont définis comme dans la revendication 1, ou un sel d'un tel composé, avec un composé de formule 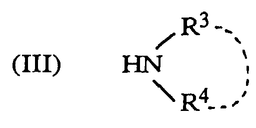 dans laquelle R³ et R⁴ sont définis comme ci-dessus, ou avec un sel d'un tel composé.

27. Procédé de préparation d'un composé de formule 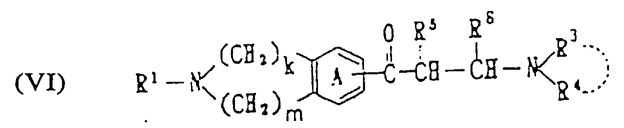 dans laquelle R¹, R³, R⁴, le cycle A, n, k et m sont définis comme dans la revendication 1, et R⁵ et R⁶ sont définis comme R² dans la revendication 1, ou d'un sel pharmaceutiquemen acceptable d'un tel composé, lequel procédé comprend la réaction d'un composé de formule 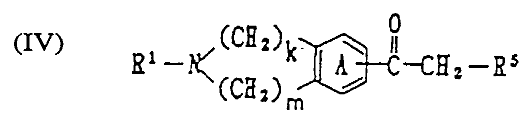 dans laquelle R¹, R⁵, le cycle A, k et m sont définis comme ci-dessus, ou d'un sel d'un tel composé, et d'un composé de formule
(V) R⁶-CHO
dans laquelle R⁶ est défini comme ci-dessus, avec un composé de formule 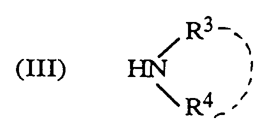 dans laquelle R³ et R⁴ sont définis comme ci-dessus, ou avec un sel d'un tel composé.

28. Inhibiteur de la cholinestérase contenant un composé de formule 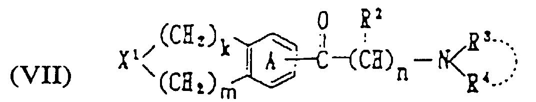 où R¹, R², R³, R⁴, le cycle A, R², n, k et m sont définis comme dans la revendication 1 et où X¹ représente un résidu R¹-N, O ou S, ou un sel pharmaceutiquement acceptable d'un tel composé.

29. Composition pharmaceutique pour traiter une maladie due à l'activité de l'acétylcholinestérase, contenant une quantité inhibitrice de la cholinestérase d'un composé de formule (I) conforme à la revendication 1 ou d'un sel pharmaceutiquement acceptable d'un tel composé, et un véhicule pharmacologiquement acceptable.

30. Composition pharmaceutique pour traiter une maladie due à l'activité de l'acétylcholinestérase, contenant une quantité inhibitrice de la cholinestérase, d'un composé de formule (VII) conforme à la revendication 28 ou d'un sel pharmaceutiquement acceptable d'un tel composé, et un véhicule pharmacologiquement acceptable.

31. Composition pharmaceutique conforme à la revendication 29 dans laquelle la maladie est la démence sénile et/ou la maladie d'Alzheimer.

32. Composition pharmaceutique conforme à la revendication 30 dans laquelle la maladie est la démence sénile et/ou la maladie d'Alzheimer.

33. Utilisation d'un composé de formule 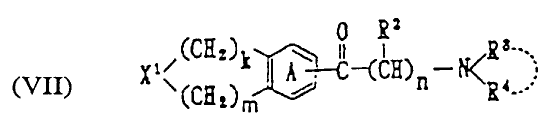 où R¹, R², R³, R⁴, le cycle A, n, k et m sont définis comme dans la revendication 1 et où X¹ représente un résidu R¹-N, O ou S, ou d'un sel pharmaceutiquement acceptable d'un tel composé en tant que composant pour la préparation d'un inhibiteur de la cholinestérase.
